# EUROPÄISCHE PATENTSCHRIFT

(11) **EP 1 891 004 B1**
(45) Veröffentlichungstag und Bekanntmachung des Hinweises auf die Patenterteilung: **02.12.2009**
(21) Anmeldenummer: 06753678.9
(22) Anmeldetag: 17.05.2006
(51) Int. Cl.: C07C 381/00, A61K 31/17

(54) **PENTAFLUORSULFANYL-SUBSTITUIERTE VERBINDUNG UND DEREN VERWENDUNG ZUR HERSTELLUNG VON ARZNEIMITTELN**
PENTAFLUOROSULPHANYL-SUBSTITUTED COMPOUND AND ITS USE FOR PRODUCING MEDICAMENTS
COMPOSES A SUBSTITUTION PENTAFLUOROSULFANYLE, ET LEUR UTILISATION POUR PRODUIRE DES MEDICAMENTS

(30) Priorität: 20.05.2005 DE 102005023943
(43) Veröffentlichungstag der Anmeldung: 27.02.2008
(73) Patentinhaber: Grünenthal GmbH, 52078 Aachen (DE)
(72) Erfinder: FRANK, Robert, 52070 Aachen (DE); SUNDERMANN, Bernd, 52074 Aachen (DE); SCHICK, Hans, 13086 Berlin (DE)
(74) Vertreter: Brosch, Oliver
(86) Internationale Anmeldenummer: PCT/EP2006/004658
(87) Internationale Veröffentlichungsnummer: WO 2006/122773

(56) Entgegenhaltungen:
- US-A- 3 073 861
- US-A- 5 741 935
- BOWDEN R D ET AL: "A New Method for the Synthesis of Aromatic Sulfurpentafluorides and Studies of the Stability of the Sulfurpentafluoride Group in Common Synthetic Transformations" Mai 2000 (2000-05), TETRAHEDRON, ELSEVIER SCIENCE PUBLISHERS, AMSTERDAM, NL, PAGE(S) 3399-3408 , XP004204450 ISSN: 0040-4020 Seite 3399, linke Spalte, Zeile 1 - Zeile 6 Seite 3399 - Seite 3400
- A.M.SIPYAGIN ET AL: "New 4-pentafluorosulfanyl and 4-perfluoroalkylthio derivatives of 1-chloro-2-nitro- and 1-chloro-2,6,-dinitrobenzenes" JOURNAL OF FLUORINE CHEMISTRY, Bd. 125, 2004, Seiten 1305-1316, XP009070674
- SERGEEVA T A ET AL: "A new synthesis of pentafluorosulfanylbenzene" 8. Juli 2004 (2004-07-08), ORGANIC LETTERS, ACS, WASHINGTON, DC, US, PAGE(S) 2417-2419 , XP002320625 ISSN: 1523-7060 das ganze Dokument

## Beschreibung

Die vorliegende Erfindung betrifft pentafluorsulfanyl-substituierte Verbindungen, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufrieden stellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz, insbesondere von neuropathischem Schmerz, stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Haminkontinenz.

Eine Aufgabe der vorliegenden Erfindung bestand daher darin, neue Verbindungen zur Verfügung zu stellen, die sich bevorzugt als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

US 3,073,861 beschreibt Ureido-substituierte Arylschwefelpentafluoride.

Aus der Veröffentlichung von Bowden et al. Tetrahedron 56 (2000), Seiten 3399-3408 sind Verfahren zur Herstellung von aromatischen Schwefelpentafluoriden bekannt.

US 5,741,935 betrifft die Herstellung organischer Schwefelpentafluoride.

Überraschenderweise wurde nun gefunden, dass sich pentafluorsulfanyl-substituierte Verbindungen der allgemeinen Formel I zur Bekämpfung von Schmerzen eignen und eine, ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden.

Ein Gegenstand der vorliegenden Erfindung sind daher pentafluorsulfanyl-substituierte Verbindungen der allgemeinen Formel I, 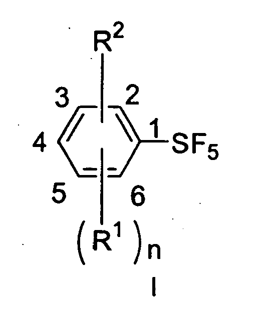 worin
- n: gleich 0, 1, 2, 3 oder 4 ist;
- R¹: für einen oder mehrere Reste unabhängig voneinander ausgewählt aus der Gruppe bestehend aus H, F, Cl, Br, I, -CN, -NC, -NO₂, -SO₃H, -S(=O₂)NH₂, -NH₂, -OH, -SH, Methyl, Ethyl, n-Propyl, Isopropyl, n-Butyl, sec-Butyl, Isobutyl, tert-Butyl, n-Pentyl, sec-Pentyl, n-Hexyl, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -N(CH₃)₂, -N(C₂H₅)_{2,} -NH-CH₃, -NH-C₂H₅, -NH-C(=O)-CH₃, -NH-C(=O)-C₂H₅, -C(=O)-NH₂, -C(=O)-CH₃, -C(=O)-C₂H₅, -C(=O)-OH, -C(=O)-OCH₃ und -C(=O)-OC₂H₅ steht;
- R²: für eine Gruppe -A-B-D, -E-F oder -N(H)-G steht, die jeweils an die 2-, 3-, 4-, 5- oder 6-Position des Phenyl-Rests der allgemeinen Formel I gebunden sein kann;
oder für eine Gruppe -Q-B-D steht; wobei Q für einen Rest ausgewählt aus der Gruppe bestehend aus Oxazolyl, Thiazolyl, Isoxazolyl, Isothiazolyl und Imidazolyl steht, der mit dem Phenyl-Rest der allgemeinen Formel I kondensiert ist und somit einen ggf. substituierten Rest ausgewählt aus der Gruppe bestehend aus Benzoxazolyl, Benzothiazolyl, Benzisothiazolyl und Benzimidazolyl bildet;
- A: für einen Rest ausgewählt aus der Gruppe bestehend aus -N(R³)-C(=O)-N(R⁴)-, -N(R³)-C(=S)-N(R⁴)-, -N(R⁵)-C(=O)-, -N(R⁵)-C(=S)-, 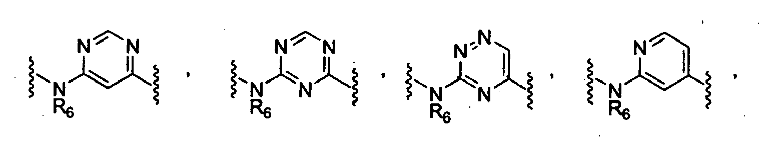 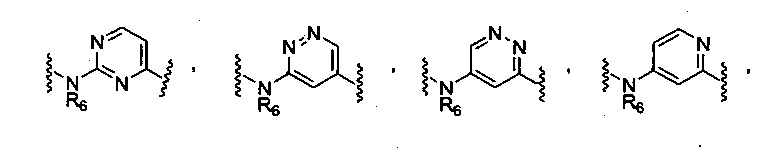 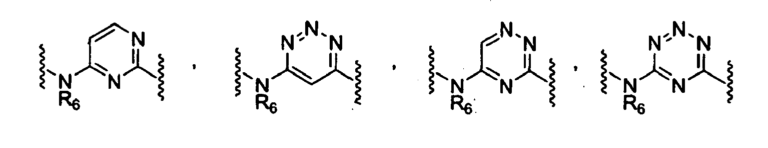 und 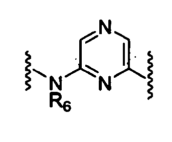 steht,
- B: für einen Rest ausgewählt aus der Gruppe bestehend aus 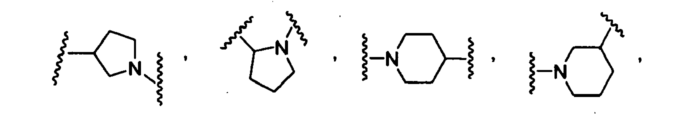 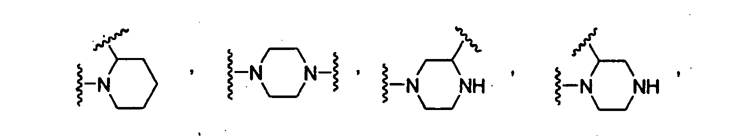 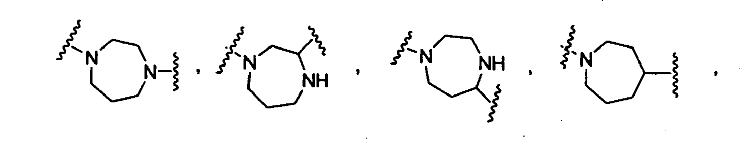 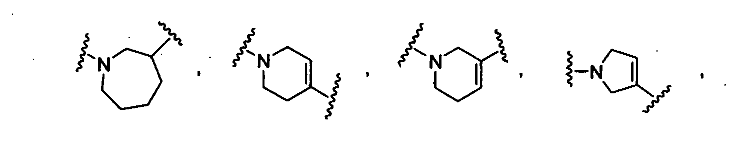 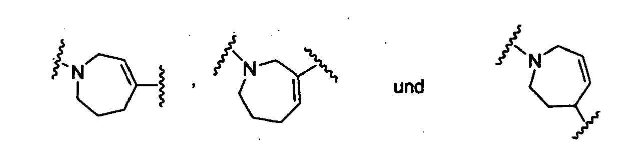 steht, der ggf. mit 1, 2, 3 oder 4 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl und n-Hexyl substituiert sein kann;
oder für einen Phenylen-Rest steht, der mit 1, 2, 3 oder 4 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl und tert-Butyl substituiert sein kann;
- D: für einen Rest ausgewählt aus der Gruppe bestehend aus Phenyl, [1,3,5]-Triazinyl, Pyridinyl, Pyridazinyl, Pyrimidinyl, Chinolinyl, Isochinolinyl und Pyrazinyl steht, der ggf. mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert sein kann;
- E: für einen Rest ausgewählt aus der Gruppe bestehend aus -(CH₂)-(CH₂₎-N(R⁷)-C(=O)-N(R⁸)-, -(CH₂)-(CH₂)-N(R⁷)-C(=S)-N(R⁸)-. -(CH₂)-N(R⁹)-C(=O)-N(R¹⁰)-, -(CH₂)-N(R⁹)-C(=S)-N(R¹⁰)-, -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R¹⁸)-C(=O)-CH(CH₃)-, -(CH₂)-N(R¹⁸)-C(=S)-CH(CH₃)-, -C(=O)-N(R¹⁹)-N(CH₂)-, -C(=S)-N(R¹⁹)-(CH₂)-, -N(R²⁰)-(CH₂)-C(=O)-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)-, -(CH₂)-N(R²⁴)-C(=O)-N(R²⁵)-CH(CH₃)- und -(CH₂)-N(R²⁴)-C(=S)-N(R²⁵)-CH(CH₃)- steht;
- F: für einen Rest ausgewählt aus der Gruppe bestehend aus Chinolinyl, Isochinolinyl, Chinoxalinyl, Chinazolinyl, (1,4)-Benzodioxanyl, (1,3)-Benzdioxolyl, (1,2,3,4)-Tetrahydronaphthyl, (1,2,3,4)-Tetrahydrochinolinyl, (1,2,3,4)-Tetrahydroisochinolinyl, Benzoxazolyl, Benzothiazolyl, Benzisoxazolyl, Benzisothiazolyl, Benzimidazolyl, 2-Oxo-2,3-dihydro-1H-benzimidazolyl, 2-Benzoxazolinonyl und 2-Benzothiazolinonyl steht, der ggf. mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert sein kann;
für einen Naphthyl-Rest steht, der mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert ist;
oder für einen Phenyl-Rest steht, der mit 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, -Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert ist;
- G: für einen Rest ausgewählt aus der Gruppe bestehend aus 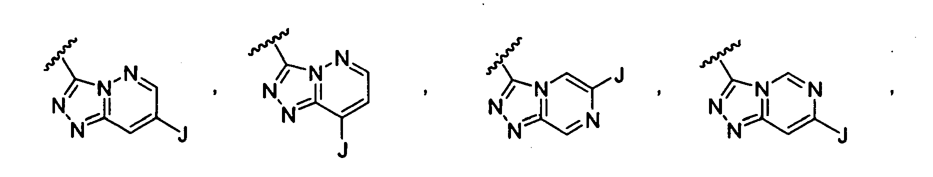 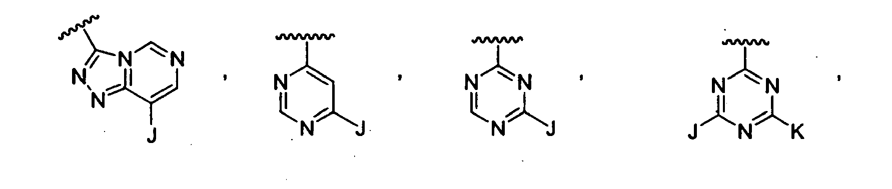 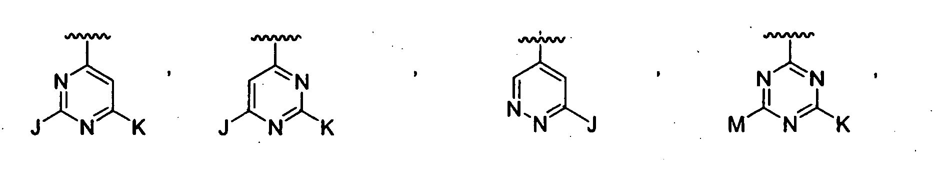 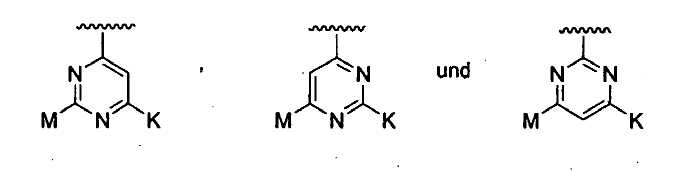 steht;
- J: für einen Rest ausgewählt aus der Gruppe bestehend aus Phenyl, [1,3,5]-Triazinyl, Pyridinyl, Pyridazinyl, Pyrimidinyl, Chinolinyl, Isochinolinyl und Pyrazinyl steht, der ggf. mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, term-butyl, n-Pentyl, sec-Pentyl, n-Hexyl, -NH-S(=O)₂-CH₃ und -NH-S(=O)₂-C₂H₅ substituiert sein kann;
- K: für einen Rest ausgewählt aus der Gruppe bestehend aus Piperidinyl, Piperazinyl, Pyrrolidinyl, Morpholinyl, Thiomorpholinyl, (1,2,3,6)-Tetrahydropyridinyl, Cyclohexyl, Cyclopentyl, Cycloheptyl, Azepanyl und Diazepanyl steht, der mit 1, 2, 3 oder 4 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, -NH-CH₃, -NH-C₂H₅, -N(CH₃)₂ und -N(C₂H₅) substituiert sein kann;
oder für eine -N ²⁶R²⁷-Gruppe steht;
- M: für einen Phenyl-Rest steht, der mit 1, 2, 3, 4 oder 5 Substituenten unabhängig voneinander ausgewählt aus der Gruppe bestehend aus -SF₅, F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl und tert-Butyl substituiert und/oder über eine -O-CH₂₋, -S-CH₂-, -CH₂-CH₂-, -CH=CH- oder -CH₂-Gruppe gebunden sein kann;
- R³, R⁴, R⁵, R⁶, R⁷,:
- R⁸, R⁹, R¹⁰, R¹¹, R¹²,:
- R¹³, R¹⁴, R¹⁵, R¹⁶, R¹⁷,:
- R¹⁸, R¹⁹, R²⁰, R²¹, R²²,:
- R²³, R²⁴ und R²⁵,: unabhängig voneinander, jeweils
für einen Wasserstoff-Rest
oder für einen Rest ausgewählt aus der Gruppe bestehend aus Wasserstoff, Methyl, Ethyl, n-Propyl, Isopropyl, n-Butyl, sec-Butyl, Isobutyl, tert-Butyl, n-Pentyl, sec-Pentyl und n-Hexyl stehen;
und
- R²⁶ und R²⁷: unabhängig voneinander, jeweils
für einen Rest ausgewählt aus der Gruppe bestehend aus Wasserstoff, Methyl, Ethyl, n-Propyl, Isopropyl, n-Butyl, sec-Butyl, Isobutyl, tert-Butyl, n-Pentyl, sec-Pentyl und n-Hexyl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind pentafluorsulfanyl-substituierte Verbindungen der vorstehend angegebenen allgemeinen Formel I, worin
- n: gleich 0 ist,
- R²: für eine Gruppe -A-B-D, -E-F oder -N(H)-G steht, die jeweils an die 4-Position des Phenyl-Rests der allgemeinen Formel I gebunden ist,
oder für eine Gruppe -Q-B-D steht; wobei Q für einen Rest ausgewählt aus der Gruppe bestehend aus Oxazolyl, Thiazolyl, Isoxazolyl, Isothiazolyl und Imidazolyl steht, der mit dem Phenyl-Rest der allgemeinen Formel I in der 4-und 5-Position kondensiert ist und somit einen ggf. substituierten Rest ausgewählt aus der Gruppe bestehend aus Benzoxazolyl, Benzothiazolyl, Benzisothiazolyl und Benzimidazolyl bildet,
- A: für einen Rest ausgewählt aus der Gruppe bestehend aus -N(R³)-C(=O)-N(R⁴)-, -N(R³)-C(-S)-N(R⁴)-, -N(R⁵)-C(-O)-, -N(R⁵)-C(=S)-, 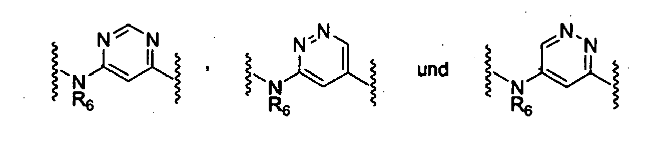 steht;
- B: für einen Rest ausgewählt aus der Gruppe bestehend aus 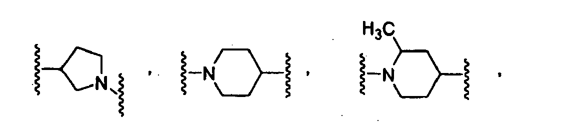 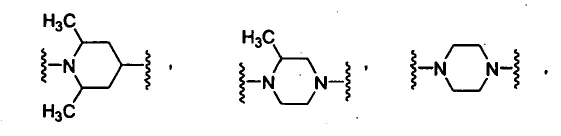 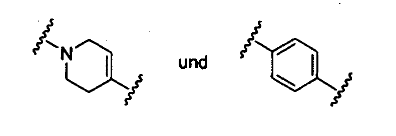 steht;
- D: für einen Rest ausgewählt aus der Gruppe bestehend aus 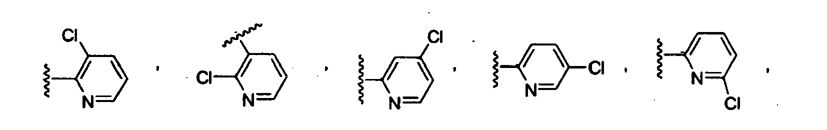 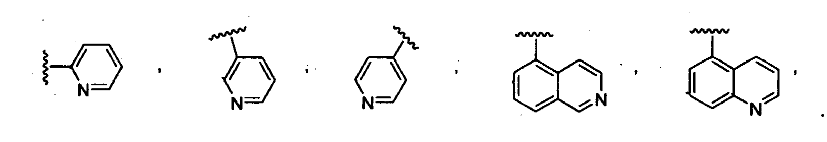 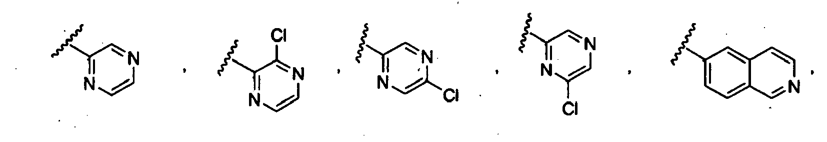 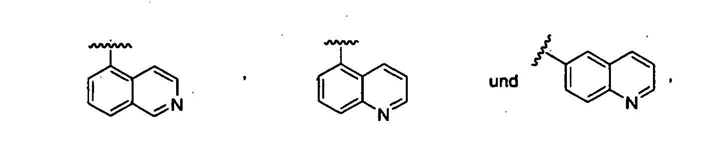 steht;
- E: für einen Rest ausgewählt aus der Gruppe bestehend aus -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R¹⁸)-C(=O)-CH(CH₃)-, -(CH₂)-N(R¹⁸)-C(=S)-CH(CH₃)-, -C(=O)-N(R¹⁹)-(CH₂)-, -C(=S)-N(R¹⁹)-(CH₂)-, -N(R²⁰)-(CH₂)-C(=O)-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)-, -(CH₂)-N(R²⁴)-C(=O)-N(R²⁵)-CH(CH₃)- und -(CH₂)-N(R²⁴)-C(=S)-N(R²⁴)-CH(CH₃)- steht;
- F: für einen Rest ausgewählt aus der Gruppe bestehend aus 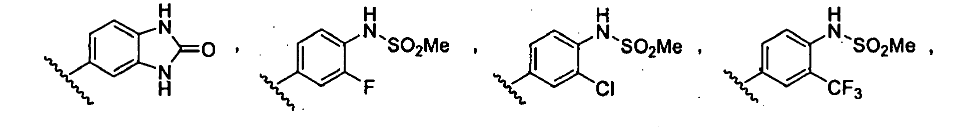 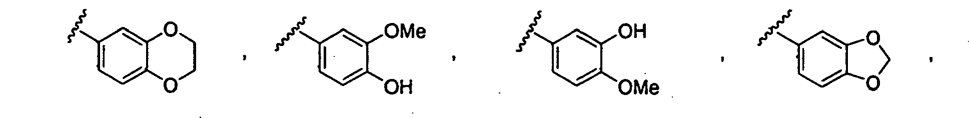 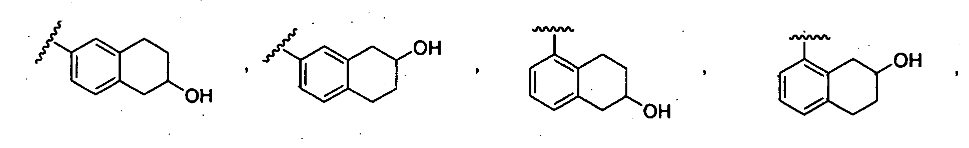 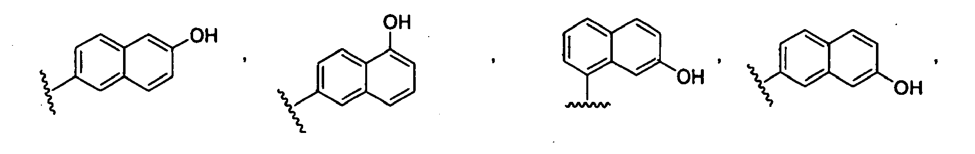 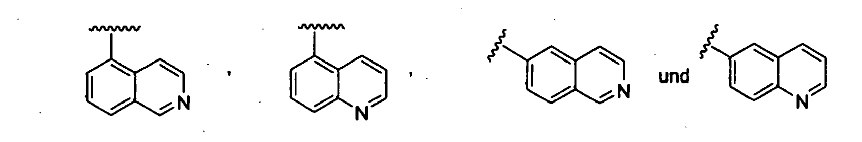 steht;
- G: für einen Rest ausgewählt aus der Gruppe bestehend aus 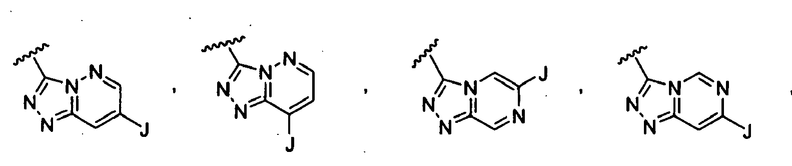 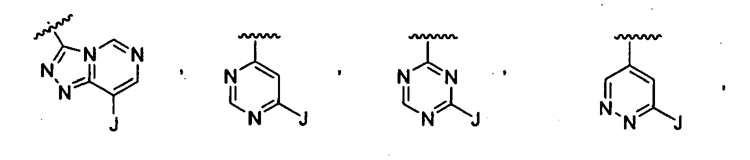 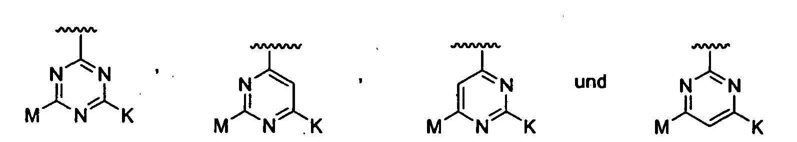 steht;
- J: für einen Rest ausgewählt aus der Gruppe bestehend aus 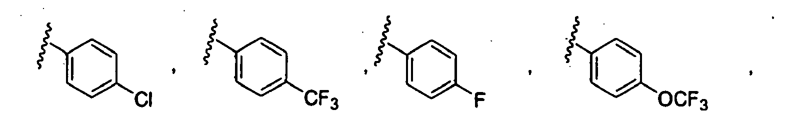 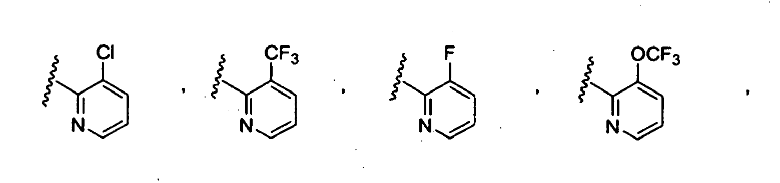 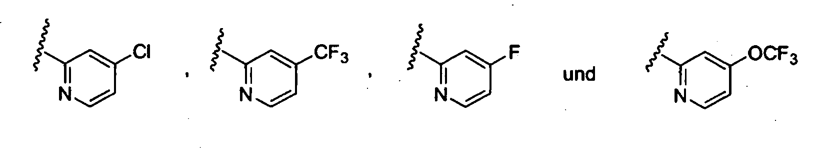 steht;
- K: für einen Rest ausgewählt aus der Gruppe bestehend aus 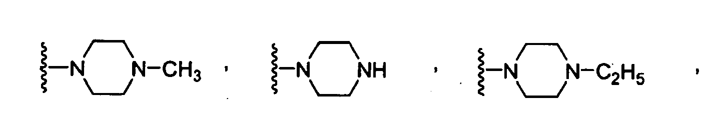 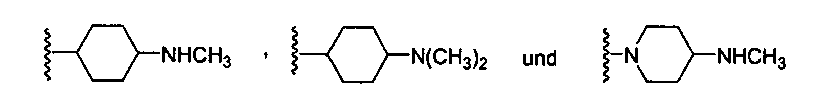 steht;
- M: für einen Rest ausgewählt aus der Gruppe bestehen aus 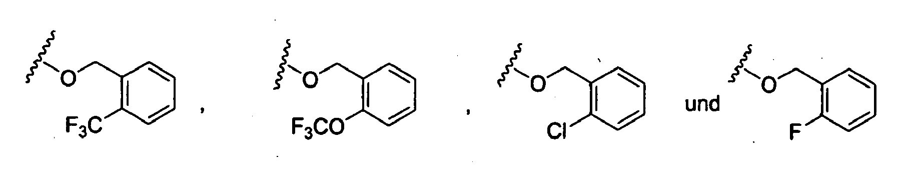 steht;
und
R³, R⁴ R⁵, R⁶, R⁷ R⁸, R⁹, R¹⁰, R¹¹, R¹², R¹³, R¹⁴, R¹⁵, R¹⁶, R¹⁷, R¹⁸, R¹⁹, R²⁰ R²¹, R²², R²³, R²⁴ und R²⁵ jeweils für einen Wasserstoff-Rest stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind pentafluorsulfanyl-substituierte Verbindungen der allgemeinen Formel I ausgewählt aus der Gruppe bestehend aus
[1] N-{4-[3-(4-Pentafluorsulfanyl-benzyl)-thioureidomethyl]-2-fluor-phenyl}-methansulfonamid
[2] 4-(3-Chlor-pyridin-2-yl)-piperazin-1-carbonsäure-(4-pentafluorsulfanyl-phenyl)-amid
[3] 4-(3-Chlor-pyridin-2-yl)-piperazin-1-thiocarbonsäure-(4-pentafluorsulfanyl-phenyl)-amid
[4] 3-(4-Pentafluorsulfanyl-phenyl)-N-(2,3-dihydro-benzo[1,4]dioxin-6-yl)-acrylamid
[5] N-(4-Pentafluorsulfanyl-benzyl)-2-(3-fluor-4-methansulfonylamino-phenyl)-propionamid
[6] (2R)-N-(4-Pentafluorsulfanyl-benzyl)₋2-(3-fluor-4-methansulfonylamino-phenyl)-propionamid
[7] (2S)-N-(4-Pentafluorsulfanyl-benzyl)-2-(3-fluor-4-methansulfonylamino-phenyl)-propionamid
[8] N-(4-Pentafluorsulfanyl-phenyl)-4-pyridin-2-yl-benzamid
[9] 1-(4-Pentafluorsulfanyl-benzyl)-3-(7-hydroxy-naphthalen-1-yl)-harnstoff
[10] 1-(4-(3-Chlor-pyrazin-2-yl)-3,6-dihydro-2H-pyridin-1-carbonsäure-(4-pentafluorsulfanyl-phenyl)-amid
[11] (4-Pentafluorsulfanyl-phenyl)-[4-(4-methyl-piperazin-1-yl)-6-(2-trifluormethyl-benzyloxy)-[1,3,5]triazin-2-yl]-amin
[12] (4-Pentafluorsulfanyll-phenyl)-[6-(4-chlor-henyl)-pyrimidin-4-yl]-amin
[13] 4-Pentafluorsulfanyl-N-(2-oxo-2,3-dihydro-1H-benzoimidazol-5-ylmethyl)-benzamid
[14] (4-Pentafluorsulfanyl-phenyl)-{6-[4-(3-chlor pyridin-2-yl)-2-(R)-methylpiperazin-1-yl]-pyrimidin-4-yl}-amin
[15] 3'-Chlor-3,6-dihydro-2H-[1,2']bipyridinyl-4-carbonsäure-(4-pentafluorsulfanyl-phenyl)-amid
[16] 2-(4-Pentafluorsulfanyl-phenylamino)-N-(7-hydroxy-naphthalen-1-yl)-acetamid
[17] (4-Pentafluorsulfanyl-phenyl)-[7-(3-trifluormethyl-pyridin-2-yl)-[1,2,4]triazolo[4,3-b]pyridazin-3-yl]-amin
[18] 1-(4-Pentafluorsulfanyl-benzyl)-3-isochinolin-5-yl-harnstoff
[19] 1-[2-(4-Pentafluorsulfanyl-phenyl)-ethyl]-3-isochinolin-5-yl-harnstoff
[20] 1-(4-Pentafluorsulfanyl-phenyl)-3-(7-hydroxy-5,6,7,8-tetrahydro-naphthalen-1-yl)-harnstoff
[21] 1-[2-(4-Pentafluorsulfanyl-phenyl)-ethyl]-3-chinolin-5-yl-harnstoff
[22] 1-(4-Pentafluorsulfänyl-phenyl)-3-(1-isochinolin-5-yl-pyrrolidin-3-yl)-harnstoff
[23] 5-Pentafluorsulfanyl-2-[4-(3-chlor-pyridin-2-yl)-piperazin-1-yl]-benzooxazol und
[24] N-{4-[3-(4-Pentafluorsulfanyl-benzyl)-ureidomethyl]-2-fluor-phenyl}-methansulfonamid.

Des weiteren können erfindungsgemäße Verbindungen bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺ -Ionen-Einstroms mit Capsaicin in einer Konzentration von 10 µM aufweisen.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Die erfindungsgemäßen Verbindungen zeichnen sich durch eine gute metabolische Stabilität aus, welche auf die Pentafluorsulfanyl-Gruppe zurückzuführen ist. Diese Gruppe wird überraschenderweise im Vergleich zu sterisch vergleichbaren Gruppen wie beispielsweise einer tert-Butyl-Gruppe langsamer abgebaut.

Die entsprechenden Strukturformeln der Verbindungen [1]-[24] sind im nachfolgenden Schema 1 angegeben.

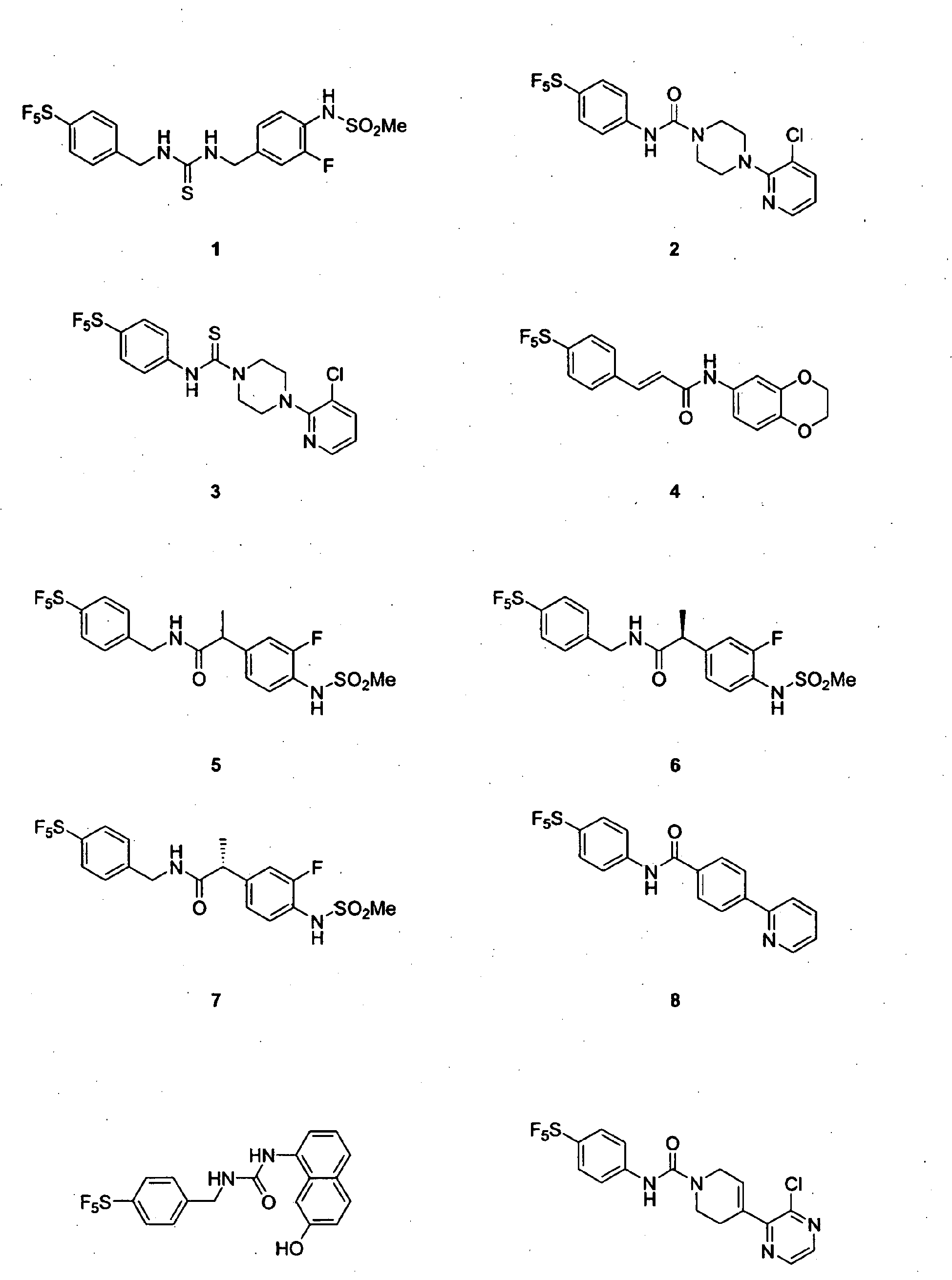

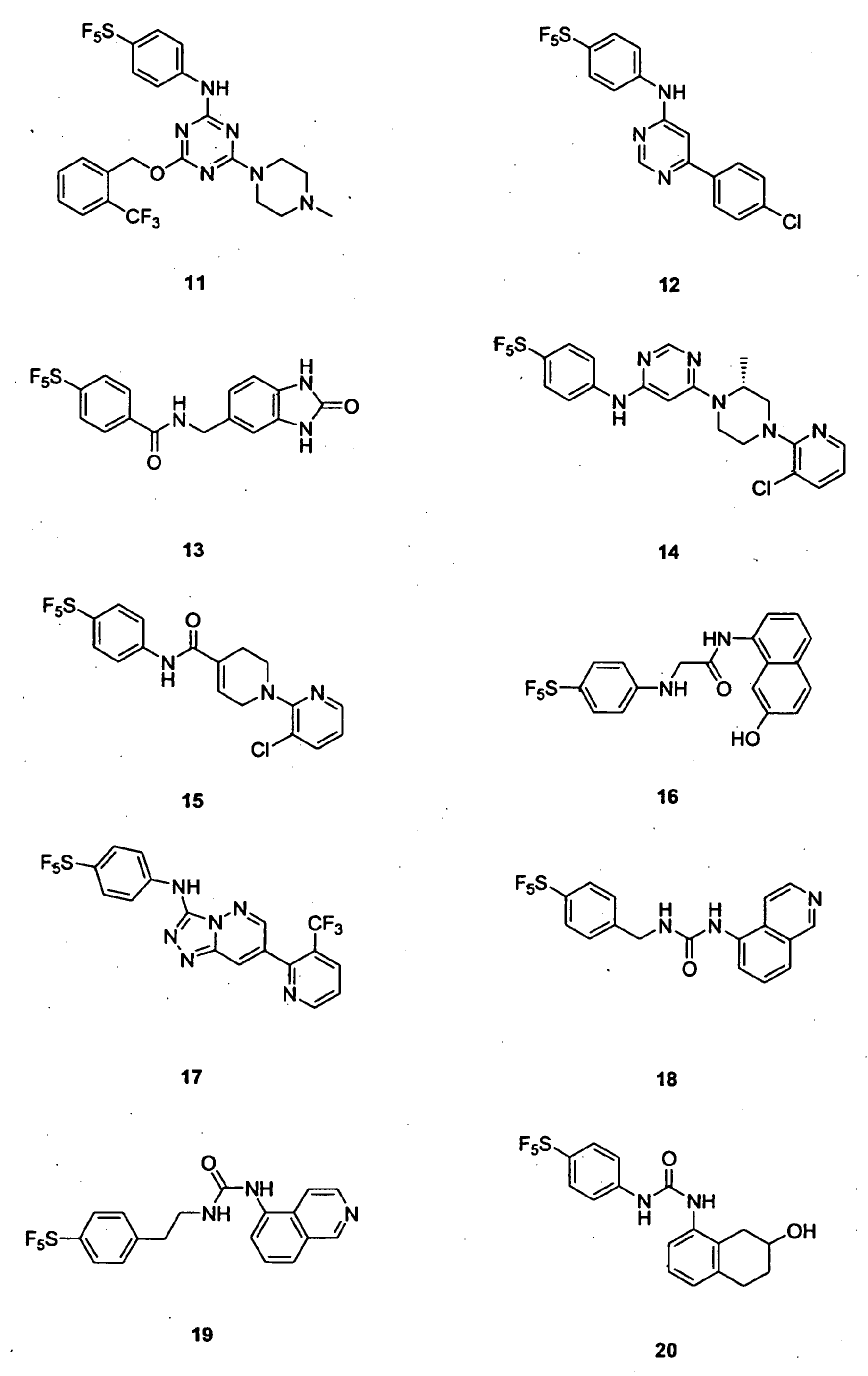

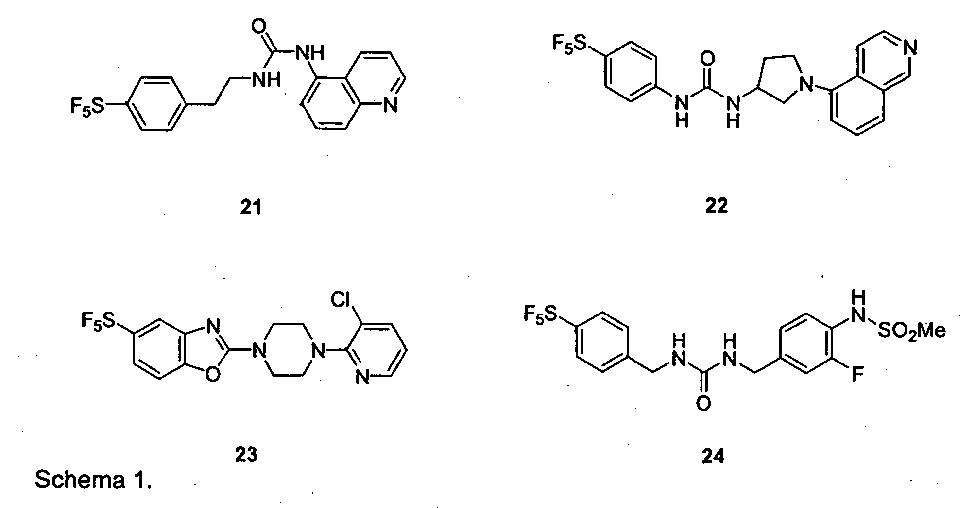

Ein weiterer Gegenstand der vorliegenden Erfindung ist ein Verfahren zur Herstellung von Verbindungen der allgemeinen Formel I gemäß dem wenigstens eine Verbindung der allgemeinen Formel II, 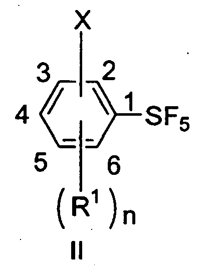 worin R¹ und n die vorstehend genannte Bedeutung haben und X für -N=C=O, - N=C=S, -(CH₂)-N=C=O, -(CH₂)-N=C=S, -(CH₂)-N=C=S, -(CH₂)-(CH₂)-N=C=O, - (CH₂)-(CH₂)-N=C=S, -CH(CH₃)-N=C=O oder -CH(CH₃)-N=C=S steht, in einem Reaktionsmedium, vorzugsweise in einem Reaktionsmedium ausgewählt aus der Gruppe bestehend aus Dimethylformamid, Toluol, Acetonitril, Dichlormethan, Diethylether, Tetrahydrofuran und Dimethylsulfoxid, in Gegenwart wenigstens einer Base, bevorzugt in Gegenwart wenigstens einer organischen Base ausgewählt aus der Gruppe bestehend aus Triethylamin, Diisopropylethylamin, N-Methylmorpholin, Pyridin und Dimethylaminopyridin, mit wenigstens einer Verbindung der allgemeinen Formel H-B-D; wobei die Gruppe H-B wenigstens eine -N(H)-Gruppe aufweist, H₂N-B-D, H₂N-F, H₂N-(CH₂)-F, H₂N-(CH₂)-(CH₂)-F oder H₂N-CH(CH₃)-F; wobei B, D und F die vorstehend genannte Bedeutung haben;
oder wenigstens eine Verbindung der allgemeinen Formel II, worin R¹ und n die vorstehend genannte Bedeutung haben und X für-NH_{2,} -(CH₂)-NH₂, -CH(CH₃)-NH₂ oder-(CH₂)-(CH₂)-NH₂ steht, in einem Reaktionsmedium, vorzugsweise in einem Reaktionsmedium ausgewählt aus der Gruppe bestehend aus Dimethylformamid, Toluol, Acetonitril, Dichlormethan, Diethylether, Tetrahydrofuran und Dimethylsulfoxid, in Gegenwart wenigstens einer Base, bevorzugt in Gegenwart wenigstens einer organischen Base ausgewählt aus der Gruppe bestehend aus Triethylamin, Diisopropylethylamin, N-Methylmorpholin, Pyridin und Dimethylaminopyridin, mit wenigstens einer Verbindung der allgemeinen Formel F-N=C=O, F-N=C=S, F-(CH₂)-N=C=O, F-(CH₂)-N=C=S, F-(CH)₂-(CH₂)-N=C=O, F-(CH₂)-(CH₂)-N=C=S, F-CH(CH₃)-N=C=O oder F-CH(CH₃)-N=C=S; wobei F die vorstehend genannte Bedeutung hat;
zu wenigstens einer Verbindung der allgemeinen Formel I; worin R¹ und n die vorstehend genannte Bedeutung haben und R² für -A-B-D oder -E-F steht, wobei A für -N(R³)-C(=O)-N(R⁴)- oder -N(R³)-C(=S)-N(R⁴)- steht; E für einen Rest ausgewählt aus der Gruppe bestehend aus -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S )-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, N(R¹⁶)-C(=O)-N(R¹⁷)-, -N(R¹⁶)-C(=S)-N(R¹⁷)-, -(CH₂)-N(R²⁴)-C(=O)-N(R²⁵)-CH(CH₃)- und -(CH₂)-N(R²⁴)-C(=S)-N(R²⁵)-CH(CH₃)- steht; wobei R³, R⁴, R⁷, R⁸, R⁹, R¹⁰, R¹², R¹³, R¹⁴, R¹⁵, R¹⁶, R¹⁷, R²⁴ und R²⁵ jeweils für Wasserstoff stehen; und B, D und F die vorstehend genannte Bedeutung haben; umgesetzt wird und diese ggf. gereinigt und/oder isoliert wird,
und ggf. wenigstens einer Verbindung der allgemeinen Formel I; worin R¹ und n die vorstehend genannte Bedeutung haben und R² für -A-B-D oder -E-F steht, wobei A für -N(R³)-C(=O)-N(R⁴)- oder -N(R³)-C(=S)-N(R⁴)- steht; E für einen Rest ausgewählt aus der Gruppe bestehend aus -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R²⁴)-C(=O)-N(R²⁵)-CH(CH₃)- und -(CH₂)-N(R²⁴)-C(=S)-N(R²⁵)-CH(CH₃)- steht; wobei R³, R⁴, R⁷, R⁸, R⁹, R¹⁰, R¹², R¹³, R¹⁴, R¹⁵, R¹⁶, R¹⁷, R²⁴ und R²⁵ jeweils für Wasserstoff stehen; und B, D und F die vorstehend genannte Bedeutung haben; in einem Reaktionsmedium, vorzugsweise in einem Reaktionsmedium ausgewählt aus der Gruppe bestehend aus Dimethylformamid, Toluol, Acetonitril, Dichlormethan, Diethylether, Tetrahydrofuran und Dimethylsulfoxid, in Gegenwart einer Base, bevorzugt in Gegenwart eines Metallhydridsalzes, besonders bevorzugt in Gegenwart von Kalium- und/oder Natriumhydrid, mit wenigstens einer Verbindung der allgemeinen Formel LG-R³, LG-R⁴, LG-R⁷, LG-R⁸, LG-R⁹, LG-R¹⁰, LG-R¹², LG-R¹³, LG-R¹⁴, LG-R¹⁵, LG-R¹⁶, LG-R¹⁷, LG-R²⁴ und LG-R²⁵; wobei R³, R⁴, R⁷, R⁸, R⁹, R¹⁰, R¹², R¹³, R¹⁴, R¹⁵, R¹⁶, R¹⁷, R²⁴ und R²⁵ die vorstehend genannte Bedeutung mit Ausnahme von Wasserstoff haben und LG für eine Abgangsgruppe, vorzugsweise für ein Halogenatom, besonders bevorzugt für ein Chloratom steht, zu wenigstens einer Verbindung der allgemeinen Formel I, worin R¹ und n die vorstehend genannte Bedeutung haben und R² für-AB-D oder -E-F steht, wobei A für -N(R³)-C(=O)-N(R⁴)- oder -N(R³)-C(=S)-N(R⁴)-steht; E für einen Rest ausgewählt aus der Gruppe bestehend aus -(CH₂)-(CH₂)-N(R7)-C(=O)-N(R⁸)-, -(CH₂)-(CH₂)-N(R⁷)-C(=S)-N(R⁸)-, -(CH₂)-N(R⁹)-C(=O)-N(R¹⁰)-, - (CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R²⁴)-C(=O)-N(R²⁵)-CH(CH₃)- und -(CH₂)-N(R²⁴)-C(=S)-N(R²⁵)-CH(CH₃)- steht; wobei R³, R⁴, R⁷, R⁸, R⁹, R¹⁰, R¹², R¹³_{,} R¹⁴, R¹⁵, R¹⁶, R¹⁷, R²⁴ und R²⁵ jeweils die vorstehend genannte Bedeutung mit Ausnahme von Wasserstoff haben; und B, D und F die Bedeutung gemäß einem oder mehreren der Ansprüche 1 bis 12 haben; umgesetzt wird und diese ggf. gereinigt und/oder isoliert wird.

Ebenfalls ein Gegenstand der vorliegenden Erfindung ist ein Verfahren zur Herstellung von Verbindungen der allgemeinen Formel I gemäß dem wenigstens eine Verbindung der allgemeinen Formel II, 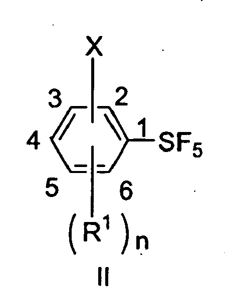 worin R¹ und n die vorstehend genannte Bedeutung haben und X für -NH₂ steht, in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, mit wenigstens einer Verbindung der Formel LG-G, worin G die vorstehend genannte Bedeutung hat und LG für eine Abgangsgruppe, vorzugsweise ein Halogenatom, besonders bevorzugt ein Fluoratom oder ein Chloratom steht, zu wenigstens einer Verbindung der allgemeinen Formel I, worin R¹ und n die vorstehend genannte Bedeutung haben und R² für -N(H)-G, wobei G die vorstehend genannte Bedeutung hat, steht, umgesetzt wird und diese ggf. gereinigt und/oder isoliert wird.

Ebenfalls ein Gegenstand der vorliegenden Erfindung ist ein Verfahren zur Herstellung von Verbindungen der allgemeinen Formel I gemäß dem wenigstens eine Verbindung der allgemeinen Formel III, 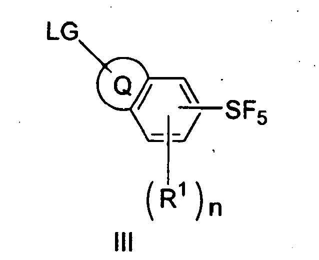 worin R¹, n und Q die vorstehend genannte Bedeutung haben und LG für eine Abgangsgruppe, vorzugweise für ein Halogenatom, besonders bevorzugt für ein Fluoratom oder Chloratom steht, in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, mit wenigstens einer Verbindung der allgemeinen Formel H-B-D; wobei die Gruppe H-B wenigstens eine -N(H)-Gruppe aufweist und B und D die vorstehend genannte Bedeutung haben, zu wenigstens einer Verbindung der allgemeinen Formel I, worin R¹ und n die vorstehend genannte Bedeutung haben und R² für -Q-B-D steht, wobei Q, B und D die vorstehend genannte Bedeutung haben, umgesetzt wird und diese ggf. gereinigt und/oder isoliert wird.

Ebenfalls ein Gegenstand der vorliegenden Erfindung ist ein Verfahren zur Herstellung von Verbindungen der allgemeinen Formel I gemäß dem wenigstens eine Verbindung der allgemeinen Formel II, 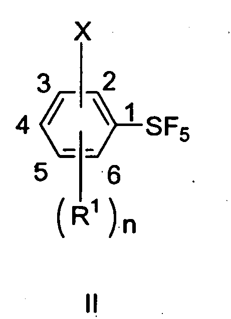 worin R¹ und n die vorstehend genannte Bedeutung haben und X für -C(=O)-LG, - (CH₂)-C(=O)-LG, -(CH₂)-(CH₂)-C(=O)-LG, -CH(CH₃)-C(=O)-LG, -CH=CH-C(=O)-LG und -N(R²⁰)-CH₂-C(=O)-LG steht, wobei LG für eine Abgangsgruppe, vorzugweise für ein Halogenatom, besonders bevorzugt für ein Chloratom steht und R²⁰ die vorstehend genannte Bedeutung hat, in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, in Gegenwart wenigstens einer Base, bevorzugt in Gegenwart wenigstens einer organischen Base ausgewählt aus der Gruppe bestehend aus Triethylamin, Diisopropylethylamin, N-Methylmorpholin, Pyridin und Dimethylaminopyridin, mit wenigstens einer Verbindung der allgemeinen Formel H-B-D; wobei die Gruppe H-B wenigstens eine -N(H)-Gruppe aufweist, H₂N-B-D, H₂N-F, H₂N-(CH₂)-F, H₂N-(CH₂)-(CH₂)-F oder H₂N-CH(CH₃)-F; wobei B, D und F die vorstehend genannte Bedeutung haben;
oder wenigstens eine Verbindung der allgemeinen Formel II, worin R¹ und n die vorstehend genannte Bedeutung haben und X für-NH_{2,} -(CH₂)-NH₂, -CH(CH₃)-NH₂, -(CH₂)-(CH₂)-NH₂ oder -CH=CH-NH₂ steht, in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, in Gegenwart wenigstens einer Base, bevorzugt in Gegenwart wenigstens einer organischen Base ausgewählt aus der Gruppe bestehend aus Triethylamin, Diisopropylethylamin, N-Methylmorpholin, Pyridin und Dimethylaminopyridin, mit wenigstens einer Verbindung der allgemeinen Formel F-C(=O)-LG, F-(CH₂)-C(=O)-LG, F-(CH₂)-(CH₂)-C(=O)-LG, F-CH(CH₃)-C(=O)-LG, F-CH=CH-C(=O)-LG und F-N(R²⁰)-CH₂-C(=O)-LG; wobei F, R²⁰ und LG die vorstehend genannte Bedeutung haben;
zu wenigstens einer Verbindung der allgemeinen Formel I, worin R¹ und n die vorstehend genannte Bedeutung haben und R² für -A-B-D oder -E-F steht, wobei A für -N(R⁵)-C(=O)- steht; E für einen Rest ausgewählt aus der Gruppe bestehend aus -CH=CH-C(=O)-N(R¹¹), -(CH₂)-N(R¹⁸)-C(=O)-CH(CH₃)-, -C(=O)-N(R¹⁹)-(CH₂)-, - N(R²⁰)-(CH₂)-C(=O)-N(R²¹)-, -(CH₂)-(CH₂)-N(R²²)-C(=O)- und -CH(CH₃)-N(R²³⁼) C(=O)- steht; wobei R⁵, R¹¹, R¹⁸, R¹⁹, R²⁰, R²¹, R²² und R²³ jeweils für einen Wasserstoff-Rest stehen; und B, D und F die vorstehend genannte Bedeutung haben; umgesetzt wird und diese ggf. gereinigt und/oder isoliert wird;
und ggf. wenigstens eine Verbindung der allgemeinen Formel I, worin R¹ und n die vorstehend genannte Bedeutung haben und R² für -A-B-D oder -E-F steht, wobei A für -N(R5)-C(=O) steht; E für einen Rest ausgewählt aus der Gruppe bestehend aus -CH=CH-C(=O)-N(R¹¹), -(CH₂)-N(R¹⁸)-C(=O)-CH(CH₃)-, -C(=O)-N(R¹⁹)-(CH₂)-N(R²⁰)-(CH₂)-C(=O)-N(R²¹)-, -(CH₂)-(CH₂)-N(R²²)-C(=O)- und -CH(CH₃)-N(R²³)-C(=O)- steht; wobei R⁵, R¹¹, R¹⁸, R¹⁹, R²⁰, R¹¹, R²² und R²³ jeweils für einen Wasserstoff-Rest stehen; und B, D und F die vorstehend genannte Bedeutung haben; in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, in Gegenwart einer Base, vorzugsweise in Gegenwart eines Metallhydridsalzes, besonders bevorzugt in Gegenwart von Kalium- und/oder Natriumhydrid mit wenigstens einer Verbindung der allgemeinen Formel LG-R⁵, LG-R¹¹, LG-R¹⁸, LG-R¹⁹, LG-R²⁰, LG-R²¹, LG-R²² und LG-R²³, wobei R⁵, R¹¹, R¹⁸, R¹⁹, R²⁰, R²¹, R²² und R²³ die vorstehend genannte Bedeutung mit Ausnahme von Wasserstoff haben, zu wenigstens einer Verbindung der allgemeinen Formel I, worin R¹ und n die vorstehend genannte Bedeutung haben und R² für A-B-D oder -E-F steht, wobei A für -N(R⁵)-C(=O)- steht; E für einen Rest ausgewählt aus der Gruppe bestehend aus .CH=CH.C(=O)-N(R¹¹), -(CH₂)-N(R¹⁸)-C(=O)-CH(CH₃)-, -C(=O)-N(R¹⁹)-(CH₂)-, -N(R²-(CH₂)-C(=O)-N(R²¹)-, -(CH₂)-(CH₂)-N(R²²)-C(=O)- und -CH(CH₃)-N(R²³)-C(=O)- steht; wobei R⁵, R¹¹, R¹⁸, R¹⁹, R²⁰, R²¹, R²² und R²³ die Bedeutung gemäß einem oder mehreren der Ansprüche 1 bis 12 haben; und B, D und F die Bedeutung gemäß einem oder mehreren der Ansprüche 1 bis 12 haben; umgesetzt wird und diese ggf. gereinigt und/oder isoliert wird;
und ggf. eine Verbindung der allgemeinen Formel I, worin R¹ und n die vorstehend genannte Bedeutung haben und R² für -A-B-D oder -E-F steht, wobei A für -N(R⁵)-C(=O)- steht; E für einen Rest ausgewählt aus der Gruppe bestehend aus -CH=CH-C(=O)-N(R¹¹), -(CH₂)-N(R¹⁸)-C(=O)-CH(CH₃)-, -C(=O)-N(R¹⁹)-(CH₂)-, -N(R²⁰)-(CH₂)-C(=O)-N(R²¹)-, -(CH₂)-(CH₂)-N(R²²)-C(=O)- und -CH(CH₃)-N(R²³)-C(=O) steht; wobei R⁵, R¹¹, R¹⁸, R¹⁹, R²⁰, R²¹, R²² und R²³ die vorstehend genannte Bedeutung haben; und B, D und F die vorstehend genannte Bedeutung haben; in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, mit wenigstens einer Verbindung der allgemeinen Formel IV, 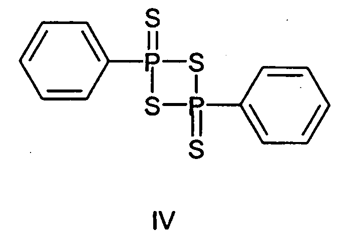 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid,
zu wenigstens einer Verbindung der allgemeinen Formel I, worin R¹ und n die vorstehend genannte Bedeutung haben und R² für -A-B-D oder -E-F steht, wobei A für -N(R⁵)-C(=S)- steht; E für einen Rest ausgewählt aus der Gruppe bestehend aus -CH=CH-C(=S)-N(R¹¹), -(CH₂)-N(R¹⁸)-C(=S)-CH(CH₃)-, -C(=S)-N(R¹⁹)-(CH₂)-, - N(R²⁰)-(CH₂)-C(=S)-N(R²¹)-, -(CH₂)-(CH₂)-N(R²²)-C(=S)- und -CH(CH₃)-N(R²³)-C(=S)- steht; wobei R⁵, R¹¹, R¹⁸, R¹⁹, R²⁰, R²¹, R²² und R²³ die vorstehend genannte Bedeutung haben; und B, D und F die Bedeutung gemäß einem oder mehreren der Ansprüche 1 bis 12 haben; umgesetzt wird und diese ggf. gereinigt und/oder isoliert wird.

Ebenfalls ein Gegenstand der vorliegenden Erfindung ist ein Verfahren zur Herstellung von Verbindungen der allgemeinen Formel I gemäß dem wenigstens einer Verbindung der allgemeinen Formel II, 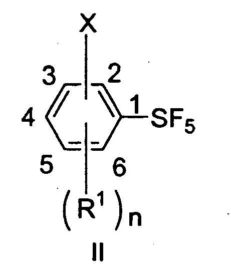 worin R¹ und n die vorstehend genannte Bedeutung haben und X für -NH₂ oder - NHR⁶ steht; wobei R⁶ die vorstehend genannte Bedeutung hat; in einem Reaktionsmedium mit wenigstens einer Verbindung der allgemeinen Formel A-B-D, worin B und D die vorstehend genannte Bedeutung haben und A für einen Rest ausgewählt aus der Gruppe bestehend aus 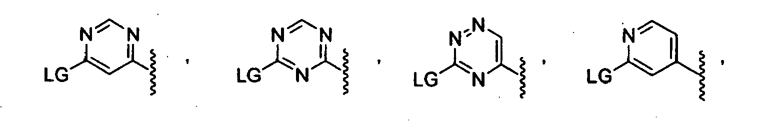 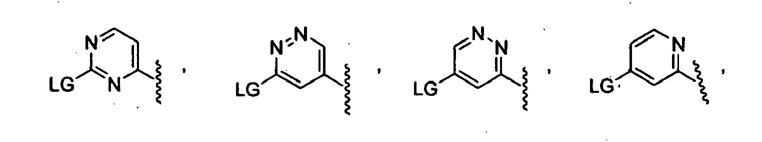 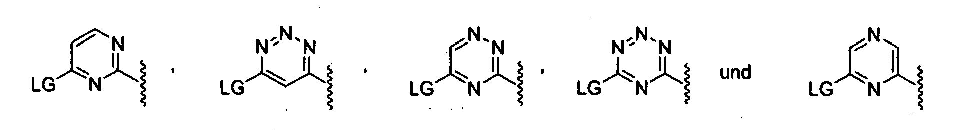 steht; wobei LG für eine Abgangsgruppe, vorzugsweise für ein Halogenatom, besonders bevorzugt für ein Fluoratom oder Chloratom steht, in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, zu wenigstens einer Verbindung der allgemeinen Formel I, worin R¹, R⁶ und n die vorstehend genannte Bedeutung haben und R² für-A-B-D steht, wobei B und D die vorstehend genannte Bedeutung haben und A die vorstehend genannte Bedeutung mit Ausnahme von -N(R³)-C(=O)-N(R⁴)-, -N(R³)-C(=S)-N(R⁴)-, -N(R⁵)-C(=O)- und - N(R⁵)-C(=S)-, umgesetzt wird und diese ggf. gereinigt und/oder isoliert wird.

Die Verbindungen der vorstehend angegebenen Formeln II, III, IV, A-B-D; worin A für einen Rest ausgewählt aus der Gruppe bestehend aus 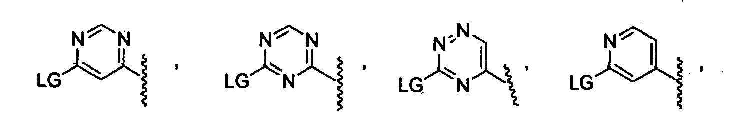 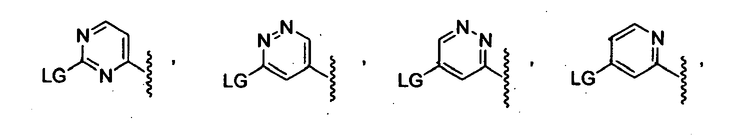 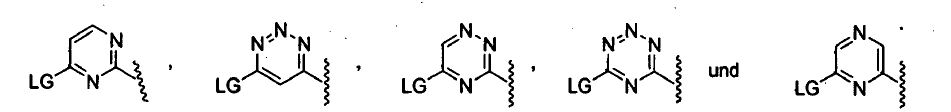 steht; H-B-D, H₂N-B-D, H₂N-F, H₂N-(CH₂)-F, H₂N-(CH₂)-(CH₂)-F, H₂N-CH(CH₃)-F, F-N=C=O, F-N=C=S, F-(CH₂)-N=C=O, F-(CH₂)-N=C=S, F-(CH)₂-(CH₂)-N=C=O, F-(CH₂)-(CH₂)-N=C=S, F-CH(CH₃)-N=C=O, F-CH(CH₃)-N=C=S, F-C(=O)-LG, F-(CH₂)-C(=O)-LG, F-(CH₂)-(CH₂)-C(=O)-LG, F-CH(CH₃)-C(=O)-LG, F-CH=CH-C(=O)-LG, F-N(R²⁰)-CH₂-C(=O)-LG, LG-R³, LG-R⁴, LG-R⁷, LG-R⁸, LG-R⁹, LG-R¹⁰, LG-R¹², LG-R¹³, LG-R¹⁴, LG-R¹⁵, LG-R¹⁶, LG-R¹⁷, LG-R²⁴, LG-R²⁵, LG-G, LG-R⁵, LG-R¹¹, LG-R¹⁸, LG-R¹⁹, LG-R²⁰, LG-R²¹, LG-R²² und LG-R²³ sind ggf. jeweils am Markt käuflich erhältlich und können auch nach üblichen, dem Fachmann bekannten Verfahren hergestellt werden.

Die Herstellung der erfindungsgemäßen Verbindungen kann analog zu den in den folgenden Druckschriften beschriebenen Verfahren erfolgen WO 2002/8221, WO 2002/16318, WO 2002/16319, WO 2003/70247, WO 2003/80578, WO 2003/95420, WO 2004/24710, WO 2004/74290, WO 2004/89877, WO 2004/103954, WO 2004/108133, WO 2005/3084, WO 2005/7646, WO 2005/9980, WO 2005/9987, WO 2005/9977, WO 2005/16890, Park et al. Bioorg. Med. Chem. Lett. 2004, 14, 787-791, Ryu et al. Bioorg. Med. Chem. Lett. 2004; 14, 1751-1755, Park et al. Bioorg. Med. Chem. Lett. 2005, 15, 631-634 und Shao et al. Bioorg. Med. Chem. Lett. 2005, 15, 719-723 entnehmen. Die entsprechenden Beschreibungen werden hiermit als Referenz eingeführt und gelten als Teil der vorliegenden Offenbarung.

Die vorstehend beschriebenen Umsetzungen können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend genannten Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen pentafluorsulfanyl-substituierten Verbindungen der vorstehend genannten allgemeinen Formel I, sowie entsprechende Stereoisomere, können sowohl in Form ihrer freien Basen, ihrer freien Säuren wie auch in Form entsprechender Salze, insbesondere physiologisch verträglicher Salze, isoliert werden. Die freien Basen der jeweiligen erfindungsgemäßen pentafluorsulfanyl-substituierten Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen pentafluorsulfanyl-substituierten Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der pentafluorsulfanyl-substituierten Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen pentafluorsulfanyl-substituierten Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen pentafluorsulfanyl-substituierten Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen pentafluorsulfanyl-substituierten Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine pentafluorsulfanyl-substituierte Verbindungen der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, insbesondere zur Vanilloid-Rezeptor 1-(VR1/TRPV1)-Hemmung oder zur Vanilloid-Rezeptor 1(VR1/TRPV1)-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen pentafluorsulfanyl-substituierten Verbindung sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1 (VR1/TRPV1)-Stimulation.

Bevorzugt ist die Verwendung wenigstens einer erfindungsgemäßen pentafluorsulfanyl-substituierten Verbindung sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens einer erfindungsgemäßen pentafluorsulfanyl-substituierten Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenäbhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens einer erfindungsgemäßen pentafluorsulfanyl-substituierten Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens einer erfindungsgemäßen pentafluorsulfanyl-substituierten Verbindung sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Das erfindungsgemäße Arzneimittel eignet sich zur Verabreichung an Erwachsene und Kinder einschließlich Kleinkindern und Säuglingen. Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer pentafluorsulfanyl-substituierten Verbindung der vorstehend angegebenen allgemeinen Formel I, ggf. in Form eines ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen pentafluorsulfanyl-substituierten Verbindungen können, in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige erfindungsgemäße pentafluorsulfanyl-substituierte Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen pentafluorsulfanyl-substituierten Verbindung der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg, vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium:
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (1 µM) auf die Zellen pipettiert und der Ca²⁺-Einstrom mit der Kontrolle (Capsaicin 1 µM) verglichen. Daraus ergibt sich die Angabe in % Aktivierung bezogen auf das Ca²⁺-Signal nach Zugabe von 1 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden,. Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37°C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37 °C inkubiert. Anschließend werden die Platten 3 mal mit HBSS-Puffer gewaschen und nach einer weiteren Inkubation von 15 Minuten bei Raumtemperatur zur Ca²⁺⁻-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsazepin berechnet.

### III. Formalin-Test an der Maus

Die Untersuchung zur Bestimmung der antinociceptiven Wirkung der erfindungsgemäßen Verbindungen wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Verbindungen werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen Verbindungen vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lösung) vor Formalinapplikation erhalten. Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

### IV. Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten hatten. Alle Verbindungen wurden in der Standarddosierung von 10 mg/kg getestet.

Im Folgenden wird die Erfindung anhand von Beispielen erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, Solvias(Base, Schweiz für SF₅-haltige Reagenzien) AG, TCI etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt.
Die Mischungsverhältnisse von Lösungsmitteln, Laufmitteln oder für chromatographische Untersuchungen sind stets in Volumen/Volumen angegeben.
Die Analytik erfolgte durch Massenspektroskopie und NMR.

### Synthese von Beispielverbindung 1:

### N-{4-[3-(4-pentafluorsulfanyl-benzyl)-thioureidomethyl]-2-fluor-phenyl}-methansulfonamid

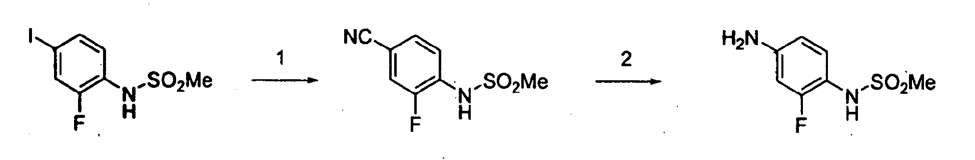

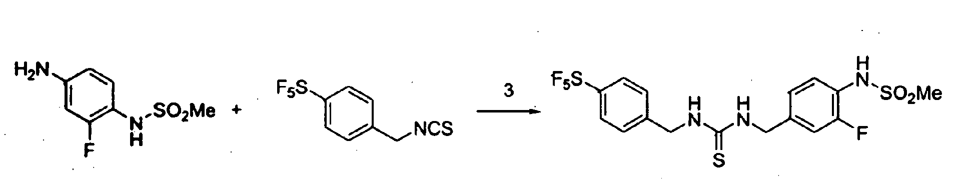

### 1. Synthese von N-(4-Cyano-2-fluo-phenyl)-methansulfonamid

Eine Suspension aus N-(2-Fluoro-4-iodo-phenyl)-methansulfonamid (2.57 g, 8.15 mmol), Zink(II)cyanid (570 mg, 4.89 mmol) und Tetrakis(triphenylphosphin)palladium (470 mg, 0.41 mmol) in Dimethylformamid (15 ml) wurde 8 h bei 80 °C und 40 h bei Raumtemperatur gerührt.
Die Reaktionsmischung wurde mit Wasser (15 ml) versetzt, der ausgefallene Niederschlag abgesaugt und das Filtrat mehrfach mit Ethylacetat extrahiert. Die vereinigten organischen Phasen wurden mit Wasser gewaschen, getrocknet und das Lösungsmittel im Vakuum entfernt. Rückstände an Dimethylformamid wurden durch Kodestillation mit Toluol entfernt. Der Rückstand wurde zur Kristallisation stehen gelassen. Es wurde das gewünschte Produkt als ein weißer Feststoff (1.15 g) erhalten.

### 2. Synthese von N-(4-Aminomethyl-2-fluor-phenyl)-methansulfonamid

In eine Hydrieranlage wurden der Katalysator (0.151 g Raney Nickel), N-(4-Cyano-2-fluo-phenyl)-methansulfonamid (1.15 g) und Methanol (25 ml) gegeben. Das Gemisch wurde bei 2 bar Wasserstoffdruck über Nacht gerührt. Es wurden 0.26 ml Wasserstoff aufgenommen. Die Reaktionsmischung wurde über Filtererde abgesaugt und der Filterkuchen mit Methanol gewaschen. Das Lösungsmittel wurde im Vakuum entfernt und der Rückstand getrocknet. Es wurde das gewünschte Produkt als Feststoff (476 mg) erhalten.

### 3. Synthese von N-{4-[3-(4-pentafluorsulfanyl-benzyl)-thioureidomethyl]-2-fluor-phenyl}-methansulfonamid

N-(4-Aminomethyl-2-fluor-phenyl)-methansulfonamid (237 mg, 1.09 mmol) wurde in Dimethylformamid (1 ml) gelöst und mit Triethylamin (132 mg, 1.30 mmol) und 4-(Pentafluorsulfanyl)benzylisothiocyanat (300 mg, 1.09 mmol) gelöst in Dimethylformamid (1 ml) versetzt. Die Reaktionsmischung wurde über Nacht bei Raumtemperatur gerührt, das Lösungsmittel im Vakuum entfernt und der Rückstand in wenig Methanol gelöst. Nach säulenchromatographischer Reinigung (Kieselgel, Hexan/Ethylacetat 1:5) wurde das Produkt als Feststoff (305 mg, 57 % der Theorie) erhalten.

### Synthese von Beispielverbindung 2:

### 4-(3-Chlor-pyridin-2-yl)-piperazin-1-carbonsäure-(4-pentafluorsulfanyl-phenyl)-amid

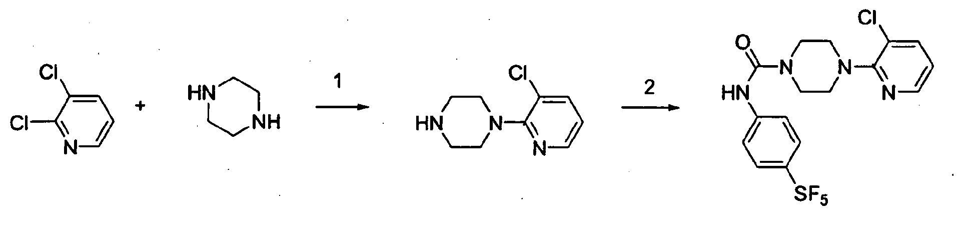

### 1. Synthese von 1-(3-Chlor-pyridin-2-yl)-piperazin

Piperazin (15 g, 174 mmol) wurde in Dimethylsulfoxid (309 g) gelöst und mit 2,3-Dichlorpyridin (23.4 g, 158 mmol) versetzt. Das Reaktionsgemisch wurde 60 h bei 110 °C gerührt. Das Reaktionsgemisch wurde portionsweise mit ges. aq. Natriumhydrogencarbonat-Lösung (insgesamt 250 ml) versetzt und der entstandene Niederschlag wurde abgesaugt. Das Filtrat wurde mehrfach mit Ethylacetat (jeweils 150 ml) extrahiert und die vereinigten organischen Phasen wurden getrocknet und das Lösungsmittel im Vakuum entfernt. Der Rückstand wurde bei 2,1⁻¹ mbar destilliert. Das Produkt wurde bei 95 °C Kopftemperatur und 155 °C Sumpftemperatür erhalten. Ausbeute: 9.6 g (28 % der Theorie)

### 2. Synthese von 4-(3-Chlor-pyridin-2-yl)-piperazin-1-carbonsäure-(4-pentafluorsulfanyl-phenyl)-amid

1-(3-Chlor-pyridin-2-yl)-piperazin (403 mg, 2.04 mmol) wurde in Dimethylformamid (1 ml) gelöst und mit Triethylamin (247 mg, 2.48 mmol) und 4-(Pentafluorsulfanyl)-phenylisocyanat (500 mg, 2.039 mmol) gelöst in Dimethylformamid (1 ml) versetzt. Die Reaktionsmischung wurde über Nacht bei Raumtemperatur gerührt, das Lösungsmittel im Vakuum entfernt und der Rückstand in wenig Methanol gelöst. Nach säulenchromatographischer Reinigung (Kieselgel, Hexan/Ethylacetat 1:1) wurde das Produkt als Feststoff (684 mg, 75 % der Theorie) erhalten.

### Synthese von Beispielverbindung 3:

### 4-(3-Chlor-pyridin-2-yl)-piperazin-1-thiocarbonsäure-(4-pentafluorsulfanyl-phenyl)-amid

1-(3-Chlor-pyridin-2-yl)-piperazin (226 mg, 1,14 mmol) wurde in Dimethylformamid (1 ml) gelöst und mit Triethylamin (140 mg, 1.38 mmol) und 4-(Pentafluorsulfanyl)-phenylisothiocyanat (300 mg, 1.14mmol) gelöst in Dimethylformamid (1 ml) versetzt. Die Reaktionsmischung wurde über Nacht bei Raumtemperatur gerührt, das Lösungsmittel im Vakuum entfernt und der Rückstand in wenig Methanol gelöst. Nach säulenchromatographischer Reinigung (Kieselgel, Hexan/Ethylacetat 1:1) wurde das Produkt als Feststoff (315 mg, 60 % der Theorie) erhalten.

Die folgenden Beispielverbindungen 9, 10, 18, 19, 20, 21 und 22 wurden analog zu den Beispielverbindungen 1 bis 3 hergestellt. Die benötigten Ausgangsstoffe sind dem Fachmann bekannt.

| | |
|---|---|
| 1 | N-{4-[3-(4-Pentafluorsulfanyl-benzyl)-thioureidomethyl]-2-fluor-phenyl}-methansulfonamid |
| 2 | 4-(3-Chlor-pyridin-2-yl)-piperazin-1-carbonsäure-(4-pentafluorsulfanyl-phenyl)-amid |
| 3 | 4-(3-Chlor-pyridin-2-yl)-piperazin-1-thiocarbonsäure-(4-pentafluorsulfanyl-phenyl)-amid |
| 4 | 3-(4-Pentafluorsulfanyl-phenyl)-N-(2,3-dihydro-benzo[1,4]dioxin-6-yl)-acrylamid |
| 5 | N-(4-Pentafluorsulfanyl-benzyl)-2-(3-fluor-4-methansulfonylamino-phenyl)-propionamid |
| 6 | (2R)-N-(4-Pentafluorsulfanyl-benzyl)-2-(3-fluor-4-methansulfonylamino-phenyl)-propionamid |
| 7 | (2S)-N-(4-Pentafluorsulfanyl-benzyl)-2-(3-fluor-4-methansulfonylamino-phenyl)-propionamid |
| 8 | N-(4-Pentafluorsulfanyl-phenyl)-4-pyridin-2-yl-benzamid |
| 9 | 1-(4-Pentafluorsulfanyl-benzyl)-3-(7-hydroxy-naphthalen-1-yl)-hamstoff |
| 10 | 4-(3-Chlor-pyrazin-2-yl)-3,6-dihydro-2H-pyridin-1-carbonsäure-(4-pentafluorsulfanyl-phenyl)-amid |
| 11 | (4-Pentafluorsulfanyl-phenyl)-[4-(4-methyl-piperazin-1-yl)-6-(2-trifluormethyl-benzyloxy)-[1,3,5]triazin-2-yl]-amin |
| 12 | (4-Pentafluorsulfanyll-phenyl)-[6-(4-chlor-phenyl)-pyrimidin-4-yl]-amin |
| 13 | 4-Pentafluorsulfanyl-N-(2-oxo-2,3-dihydro-1H-benzoimidazol-5-ylmethyl)-benzamid |
| 14 | (4-Pentafluorsulfanyl-phenyl)-{6-[4-(3-chlor-pyridin-2-yl)-2-(R)-methyl-piperazin-1-yl]-pymidin-4-yl}-amin |
| 15 | 3'-Chlor-3,6-dihydro-2H-[1,2']bipyridinyl-4-carbonsäure-(4-pentafluorsulfanyl-phenyl)-amid |
| 16 | 2-(4-Pentafluorsulfanyl-phenylamino)-N-(7-hydroxy-naphthalen-1-yl)-acetamid |
| 17 | (4-Pentafluorsulfanyl-phenyl)-[7-(3-trifluormethyl-pyridin-2-yl)-[1,2,4]triazolo[4,3-b]pyridazin-3-yl]-amin |
| 18 | 1-(4-Pentafluorsulfanyl-benzyl)-3-isochinolin-5-yl-harnstoff |
| 19 | 1-[2-(4-Pentafluorsulfanyl-phenyl)-ethyl]-3-isochinolin-5-yl-harnstoff |
| 20 | 1-(4-Pentafluorsulfanyl-phenyl)-3-(7-hydroxy-5,6,7,8-tetrahydro-naphthalen-1-yl)-harnstoff |
| 21 | 1-[2-(4-Pentafluorsulfanyl-phenyl)-ethyl]-3-chinolin-5-yl-harnstoff |
| 22 | 1-(4-Pentafluorsulfanyl-phenyl)-3-(1-isochinolin-5-yl-pyrrolidin-3-yl)-harnstoff |
| 23 | 5-Pentafluorsulfanyl-2-[4-(3-chlor-pyridin-2-yl)-piperazin-1-yl]-benzooxazol |
| 24 | N-{4-[3-(4-Pentafluorsulfanyl-benzyl)-ureidomethyl]-2-fluor-phenyl}-methansulfonamid |

### Pharmakologische Daten

Die Affinität der erfindungsgemäßen pentafluorsulfanyl-substituierten Verbindungen zum Vanilloid-Rezeptor 1 (VR1/TRPV1-Rezeptor) wurde wie vorstehend beschrieben bestimmt.

| **Verbindung gemäß Beispiel** | **VR1 (Ratte) (% Stimulation im Vgl. zu 1 µM CP)** | **VR1 (Ratte) (% Hemmung im Vgl. zu 1 µM CP)** | **VR1 (Mensch) (% Stimulation im Vgl. zu 1 µM CP)** | **VR1 (Mensch) (% Hemmung im Vgl. zu 1 µM CP)** |
|---|---|---|---|---|
| **1** | 0,02 | 90,27 | 0,68 | 87,67 |
| **2** | 0,63 | 101,93 | 3,72 | 105,44 |

| **Verbindung gemäß Beispiel** | **IC₅₀ (Ratte) (µM)** | **IC₅₀ (Mensch) (µM)** | **Bindung Kᵢ(µM)** |
|---|---|---|---|
| 1 | 0,36 | 0,8 | 0,084 |
| 2 | 0,048 | 0,25 | 0,012 |
| 3 | | | 0,094 |

## Patentansprüche

1. Pentafluorsulfanyl-substituierte Verbindungen der allgemeinen Formel I, 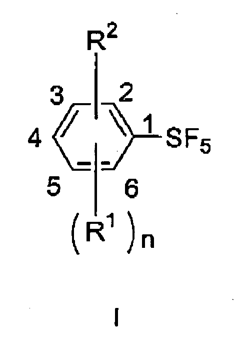 worin
n gleich 0, 1, 2, 3 oder 4 ist;
R¹ für einen oder mehrere Reste unabhängig voneinander ausgewählt aus der Gruppe bestehend aus H, F, Cl, Br, I, -CN, -NC, -NO₂, -SO₃H, -S(=O₂)NH₂, -NH₂, -OH, -SH, Methyl, Ethyl, n-Propyl, Isopropyl, n-Butyl, sec-Butyl, Isobutyl, tert-Butyl, n-Pentyl, sec-Pentyl, n-Hexyl, -O-CH₃, -O-C₂H₅, -O-CH(CH₃)₂, -O-C(CH₃)₃, -S-CH₃, -S-C₂H₆, -S-CH(CH₃)₂, -S-C(CH₃)₃, -N(CH₃)₂, -N(C₂H₅)₂, -NH-CH₃, -NH-C₂H₅, -NH-C(=O)-CH₃, -NH-C(=O)-C₂H₅, -C(=O)-NH₂, -C(=O)-CH₃, -C(=O)-C₂H₅, -C(=O)-OH, -C(=O)-OCH₃ und -C(=O)-OC₂H₅ steht;
R² für eine Gruppe A-B-D, -E-F oder -N(H)-G steht, die jeweils an die 2-, 3-, 4-, 5- oder 6-Position des Phenyl-Rests der allgemeinen Formel I gebunden sein kann;
oder für eine Gruppe -Q-B-D steht; wobei Q für einen Rest ausgewählt aus der Gruppe bestehend aus Oxazolyl, Thiazolyl, Isoxazolyl, Isothiazolyl und Imidazolyl steht, der mit dem Phenyl-Rest der allgemeinen Formel I kondensiert ist und somit einen ggf. substituierten Rest ausgewählt aus der Gruppe bestehend aus Benzoxazolyl, Benzothiazolyl, Benzisothiazolyl und Benzimidazolyl bildet;
A für einen Rest ausgewählt aus der Gruppe bestehend aus -N(R³)-C(=O)-N(R⁴)-, -N(R³)-C(=S)-N(R⁴)-, -N(R⁵)-C(=O)-, -N(R⁵)-C(=S)-, 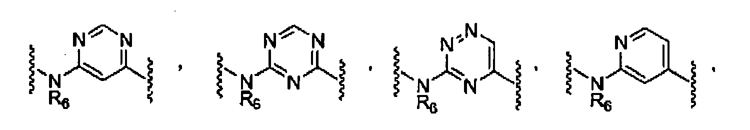 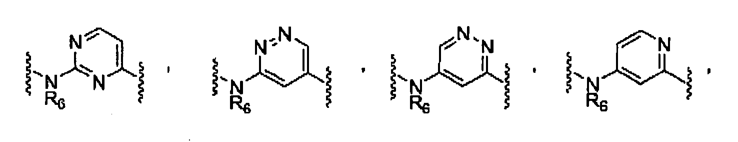 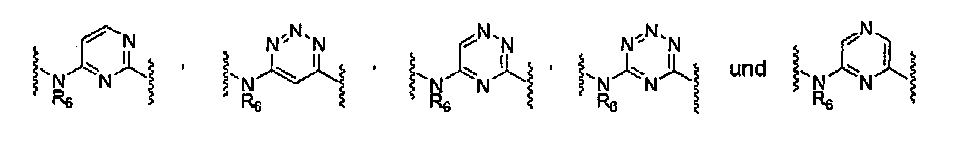 steht,
B für einen Rest ausgewählt aus der Gruppe bestehend aus 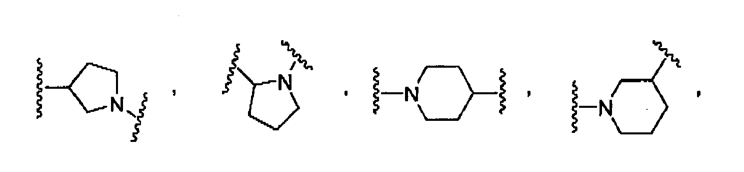 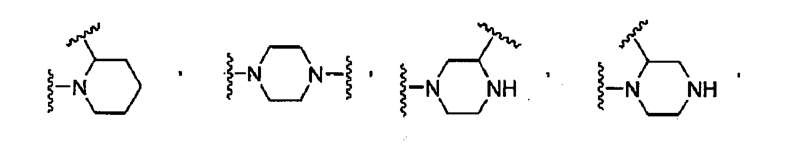 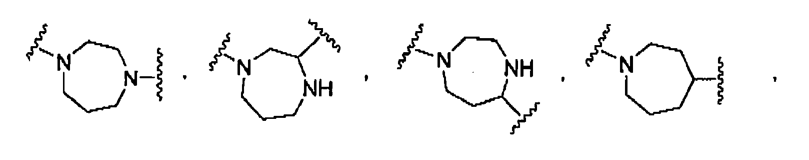 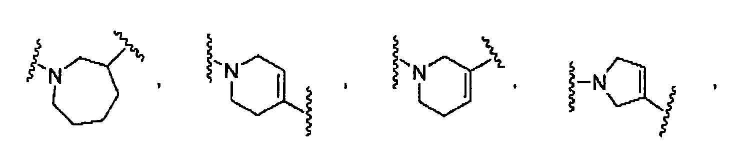 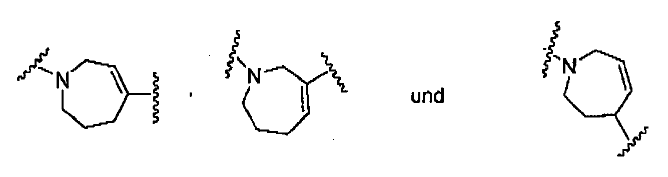 steht, der ggf. mit 1, 2, 3 oder 4 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl und n-Hexyl substituiert sein kann;
oder für einen Phenylen-Rest steht, der mit 1, 2, 3 oder 4 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl und tert-Butyl substituiert sein kann;
D für einen Rest ausgewählt aus der Gruppe bestehend aus Phenyl, [1,3,5]-Triazinyl, Pyridinyl, Pyridazinyl, Pyrimidinyl, Chinolinyl, Isochinolinyl und Pyrazinyl steht, der ggf. mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert sein kann;
E für einen Rest ausgewählt aus der Gruppe bestehend aus -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R¹⁸)-C(=O)-CH(CH₃)-, -(CH₂)-N(R¹⁸)-C(=S)-CH(CH₃)-, -1-C(=O)-N(R¹⁹)-(CH₂)-, -C(=S)-N(R¹⁹)-(CH₂)-, -N(R²⁰)-(CH₂)-C(=O)-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)-, -(CH₂)-N(R²⁴)-C(=O)-N(R²⁵)-CH(CH₃)- und -(CH₂)-N(R²⁴)-C(=S)-N(R²⁵)-CH(CH₃)- steht;
F für einen Rest ausgewählt aus der Gruppe bestehend aus Chinolinyl, Isochinolinyl, Chinoxalinyl, Chinazolinyl, (1,4)-Benzodioxanyl, (1,3)-Benzdioxolyl, (1,2,3,4)-Tetrahydronaphthyl, (1,2,3,4)-Tetrahydrochinolinyl, (1,2,3,4)-Tetrahydroisochinolinyl, Benzoxazolyl, Benzothiazolyl, Benzisoxazolyl, Benzisothiazolyl, Benzimidazolyl, 2-Oxo-2,3-dihydro-1H-benzimidazolyl, 2-Benzoxazolinonyl und 2-Benzothiazolinonyl steht, der ggf. mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert sein kann;
für einen Naphthyl-Rest steht, der mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert ist;
oder für einen Phenyl-Rest steht, der mit 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂. Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert ist;
G für einen Rest ausgewählt aus der Gruppe bestehend aus 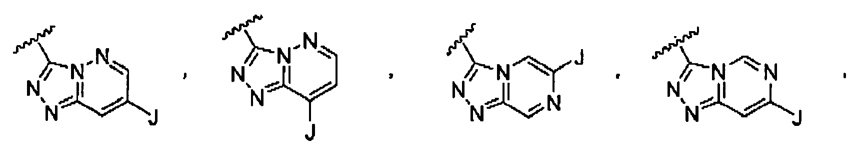 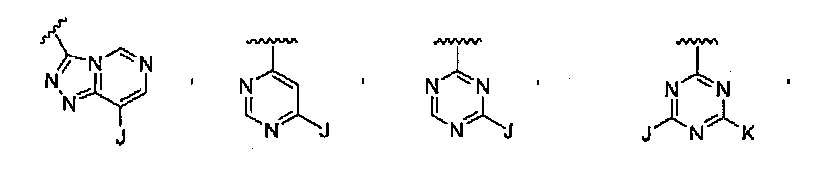 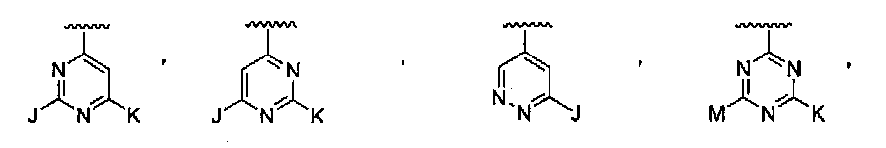 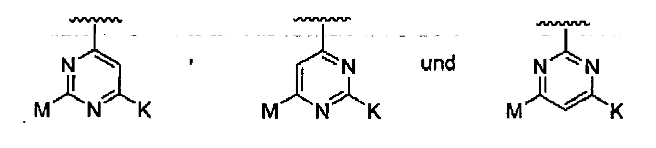 steht;
J für einen Rest ausgewählt aus der Gruppe bestehend aus Phenyl, [1,3,5]-Triazinyl, Pyridinyl, Pyrimidinyl, Pyridazinyl, Chinolinyl, Isochinolinyl und Pyrazinyl steht, der ggf. mit 1, 2, 3, 4 oder 5 Substituenten unabhängig voneinander ausgewählt aus der Gruppe bestehend aus F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl, tert-Butyl, n-Pentyl, sec-Pentyl, n-Hexyl, -NH-S(=O)₂-CH₃ und -NH-S(=O)₂-C₂H₅ substituiert sein kann;
K für einen Rest ausgewählt aus der Gruppe bestehend aus Piperidinyl, Piperazinyl, Pyrrolidinyl, Morpholinyl, Thiomorpholinyl, (1,2,3,6)-Tetrahydropyridinyl, Cyclohexyl, Cyclopentyl, Cycloheptyl, Azepanyl und Diazepanyl steht, der mit 1, 2, 3 oder 4 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, -NH-CH₃, -NH-C₂H₅, -N(CH₃)₂ und -N(C₂H₅) substituiert sein kann;
oder für eine -NR²⁶R²⁷-Gruppe steht;
M für einen Phenyl-Rest steht, der mit 1, 2, 3, 4 oder 5 Substituenten unabhängig voneinander ausgewählt aus der Gruppe bestehend aus -SF₅, F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, Methyl, Ethyl, n-Propyl, Isopropyl, n-Butyl, sec-Butyl, Isobutyl und tert-Butyl substituiert und/oder über eine -O-CH₂-, -S-CH₂-, -CH₂-CH₂-, -CH=CH- oder -CH₂-Gruppe gebunden sein kann;
R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴ und R²⁵, unabhängig voneinander, jeweils
für einen Wasserstoff-Rest
oder für einen Rest ausgewählt aus der Gruppe bestehend aus Wasserstoff, Methyl, Ethyl, n-Propyl, lsopropyl, n-Butyl, sec-Butyl, Isobutyl, tert-Butyl, n-Pentyl, sec-Pentyl und n-Hexyl stehen;
und
R²⁶ und R²⁷, unabhängig voneinander, jeweils
für einen Rest ausgewählt aus der Gruppe bestehend aus Wasserstoff, Methyl, Ethyl, n-Propyl, Isopropyl, n-Butyl, sec-Butyl, Isobutyl, tert-Butyl, n-Pentyl, sec-Pentyl und n-Hexyl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass** n gleich 0 ist.

3. Verbindungen gemäß Anspruch 1 oder 2, **dadurch gekennzeichnet, dass** R² für eine Gruppe -A-B-D, -E-F oder -N(H)-G steht, die jeweils an die 4-Position des Phenyl-Rests der allgemeinen Formel I gebunden ist,
oder für eine Gruppe -Q-B-D steht; wobei Q für einen Rest ausgewählt aus der Gruppe bestehend aus Oxazolyl, Thiazolyl, Isoxazolyl, Isothiazolyl und Imidazolyl steht, der mit dem Phenyl-Rest der allgemeinen Formel I in der 4- und 5-Position kondensiert ist und somit einen ggf. substituierten Rest ausgewählt aus der Gruppe bestehend aus Benzoxazolyl, Benzothiazolyl, Benzisothiazolyl und Benzimidazolyl bildet.

4. Verbindungen gemäß einem oder mehreren der Ansprüche 1 bis 3, **dadurch gekennzeichnet, dass**
B für einen Rest ausgewählt aus der Gruppe bestehend aus 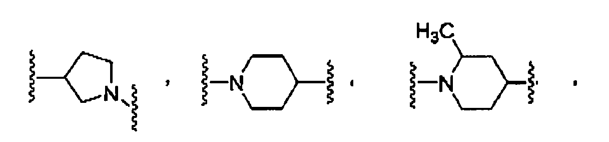 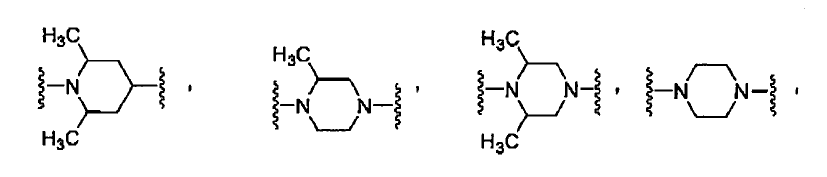 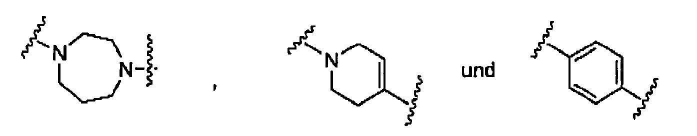 steht.

5. Verbindungen gemäß einem oder mehreren der Ansprüche 1 bis 4, **dadurch gekennzeichnet, dass**
D für einen Rest ausgewählt aus der Gruppe bestehend aus 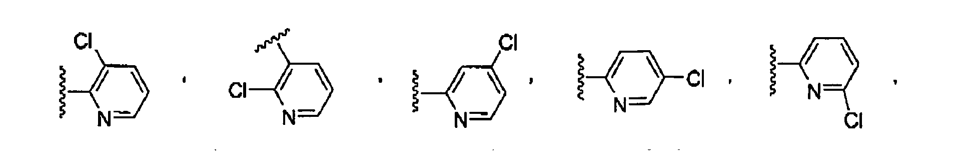 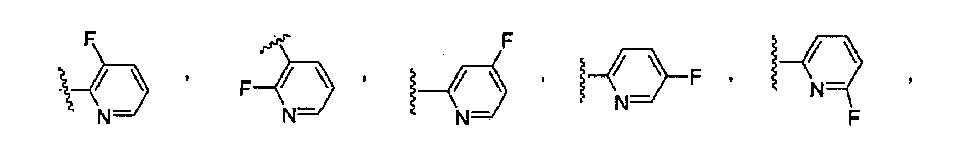 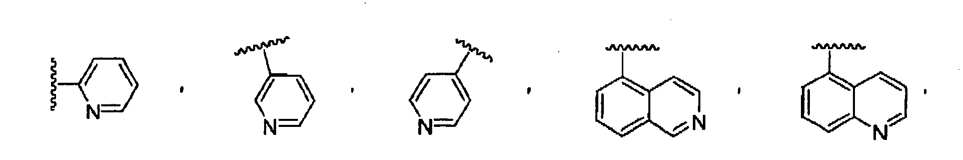 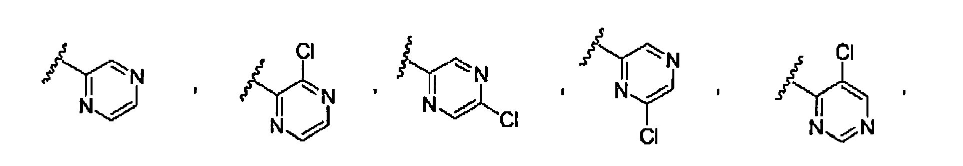 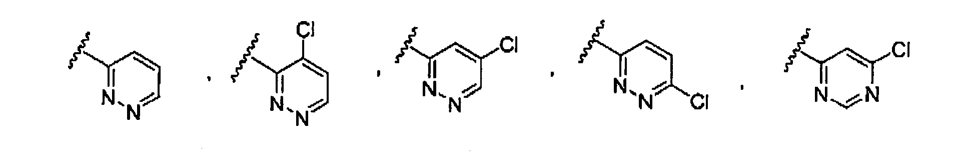 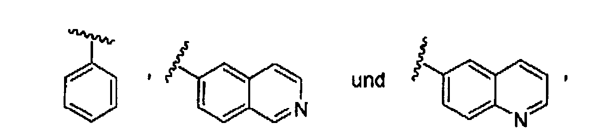 steht.

6. Verbindungen gemäß einem oder mehreren der Ansprüche 1 bis 5, **dadurch gekennzeichnet, dass**
F für einen Rest ausgewählt aus der Gruppe bestehend aus 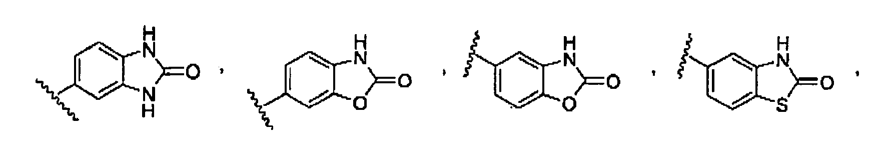 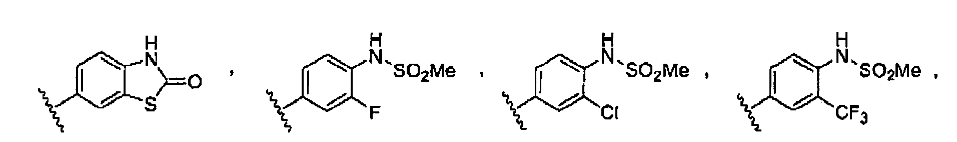 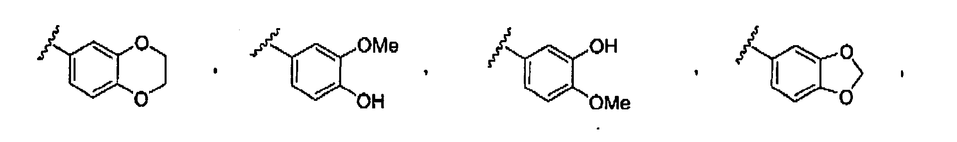 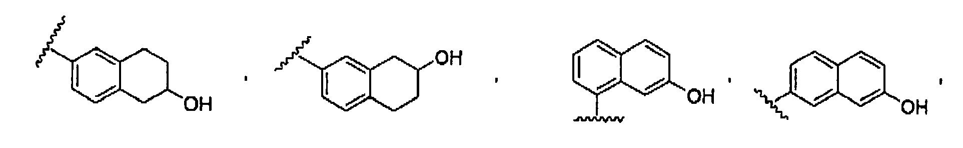 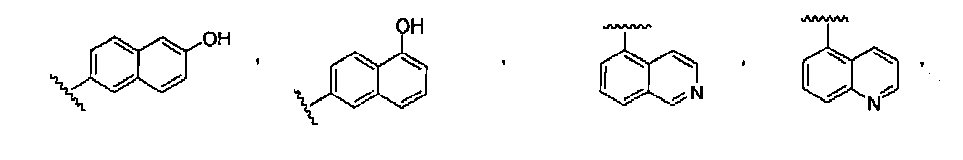 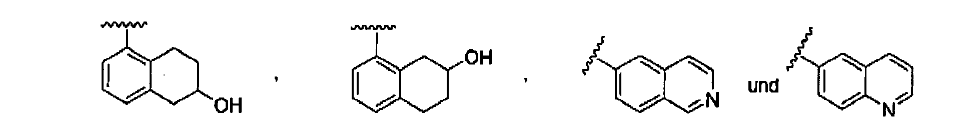 steht.

7. Verbindungen gemäß einem oder mehreren der Ansprüche 1 bis 6, **dadurch gekennzeichnet, dass**
J für einen Rest ausgewählt aus der Gruppe bestehend aus 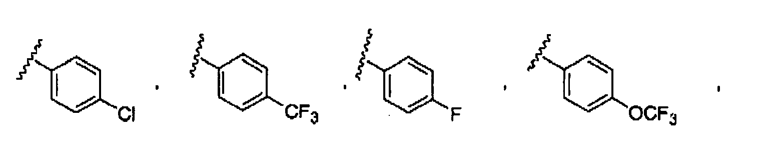 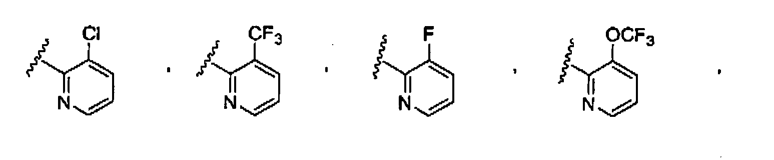 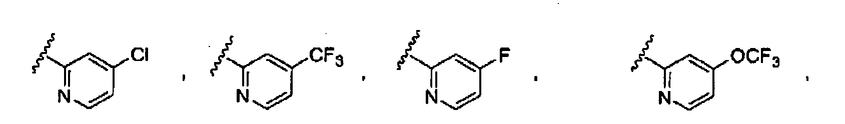 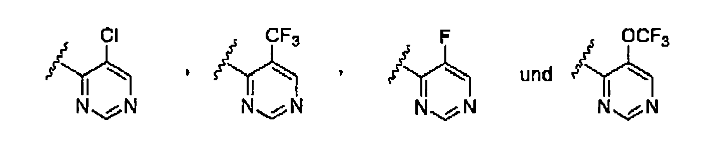 steht.

8. Verbindungen gemäß einem oder mehreren der Ansprüche 1 bis 7, **dadurch gekennzeichnet, dass**
K für einen Rest ausgewählt aus der Gruppe bestehend aus 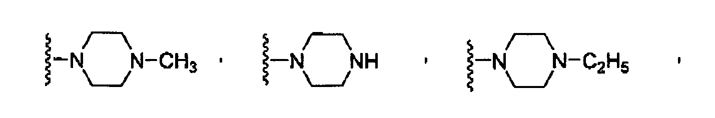 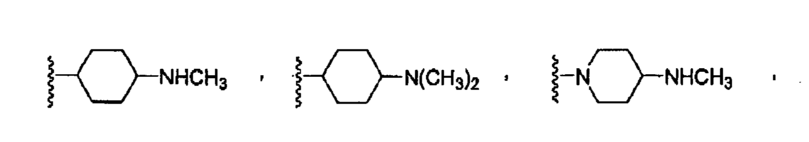 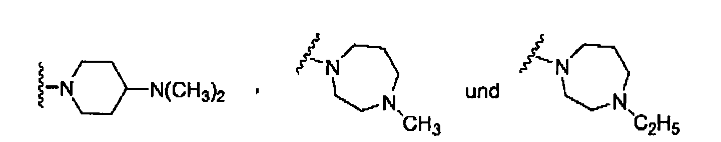 steht.

9. Verbindungen gemäß einem oder mehreren der Ansprüche 1 bis 8, **dadurch gekennzeichnet, dass**
M für einen Rest ausgewählt aus der Gruppe bestehend aus 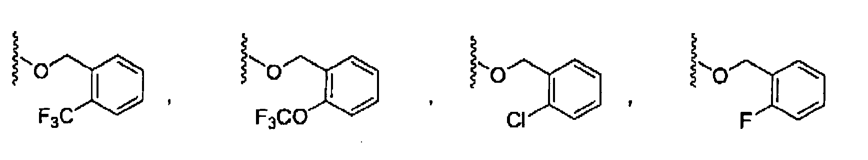 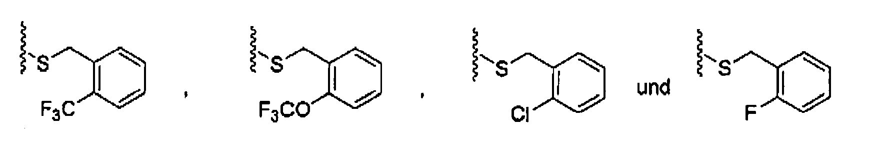 steht.

10. Verbindungen gemäß einem oder mehreren der Ansprüche 1 bis 9, **dadurch gekennzeichnet, dass**
n gleich 0 ist,
R² für eine Gruppe A-B-D, -E-F oder -N(H)-G steht, die jeweils an die 4-Position des Phenyl-Rests der allgemeinen Formel I gebunden ist,
oder für eine Gruppe -Q-B-D steht; wobei Q für einen Rest ausgewählt aus der Gruppe bestehend aus Oxazolyl, Thiazolyl, Isoxazolyl, Isothiazolyl und Imidazolyl steht, der mit dem Phenyl-Rest der allgemeinen Formel I in der 4- und 5-Position kondensiert ist und somit einen ggf. substituierten Rest ausgewählt aus der Gruppe bestehend aus Benzoxazolyl, Benzothiazolyl, Benzisothiazolyl und Benzimidazolyl bildet,
A für einen Rest ausgewählt aus der Gruppe bestehend aus -N(R³)-C(=O)-N(R⁴)-, -N(R³)-C(=S)-N(R⁴)-, -N(R⁵)-C(=O)-, -N(R⁵)-C(=S)-, steht;
B für einen Rest ausgewählt aus der Gruppe bestehend aus 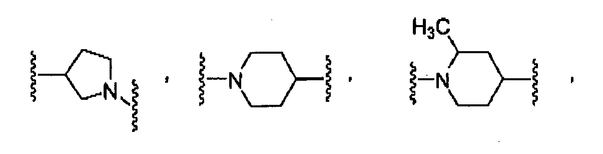 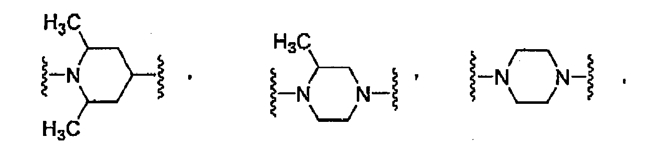 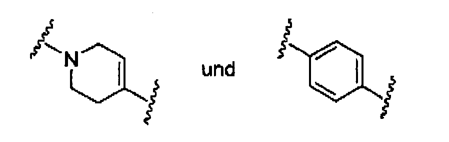 steht;
D für einen Rest ausgewählt aus der Gruppe bestehend aus 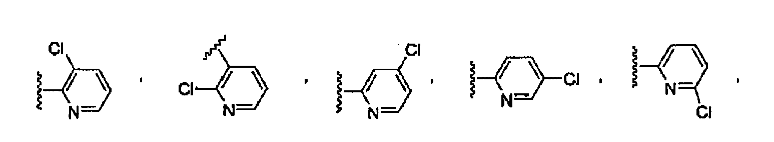 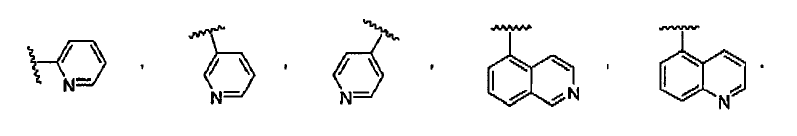 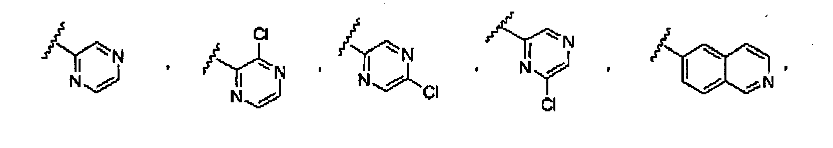 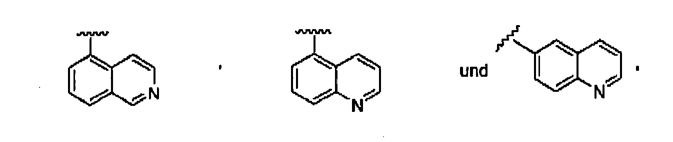 steht;
E für einen Rest ausgewählt aus der Gruppe bestehend aus -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)C(=S)-N(R¹⁰)-, -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R¹⁸)-C(=O)-CH(CH₃)-, -(CH₂)-N(R¹⁸)-C(=S)-CH(CH₃)-, -C(=O)-N(R¹⁹)-(CH₂)-,-C(=S)-N(R¹⁹)-(CH₂)-, -N(R²⁰)-(CH₂)-C(=O)-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)-, -(CH₂)-N(R²⁴)-C(=O)-N(R²⁵)-CH(CH₃)- und -(CH₂)-N(R²⁴)-C(=S)-N(R²⁵)-CH(CH₃)- steht;
F für einen Rest ausgewählt aus der Gruppe bestehend aus 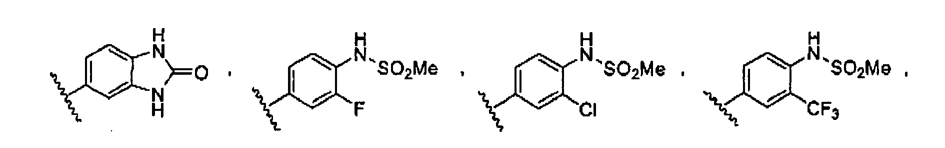 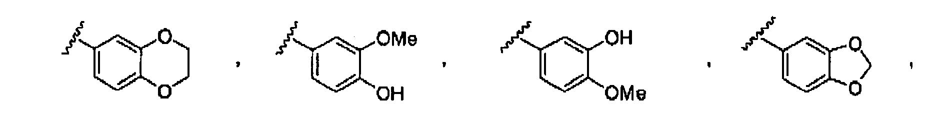 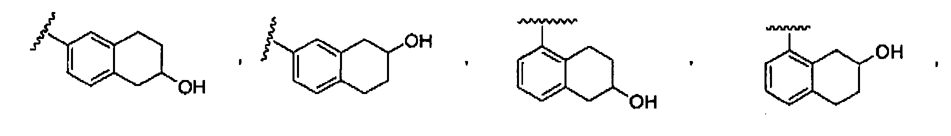 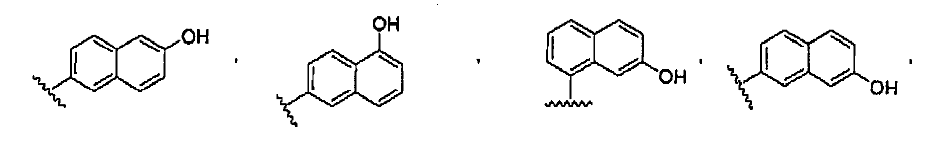 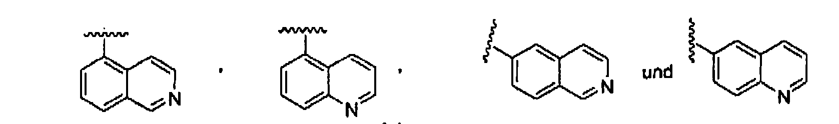 steht;
G für einen Rest ausgewählt aus der Gruppe bestehend aus 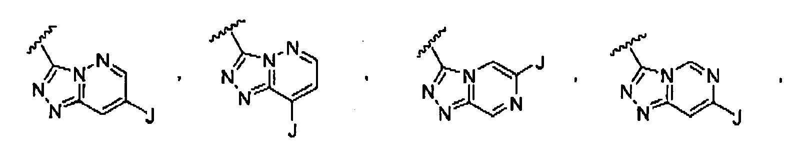 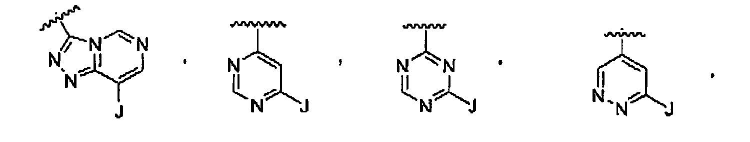 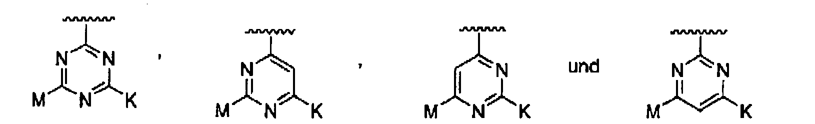 steht;
J für einen Rest ausgewählt aus der Gruppe bestehend aus 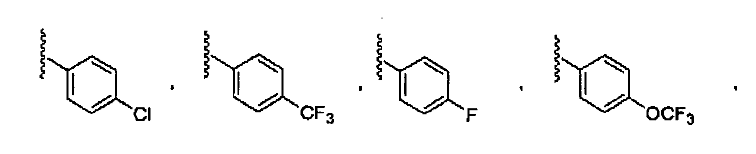 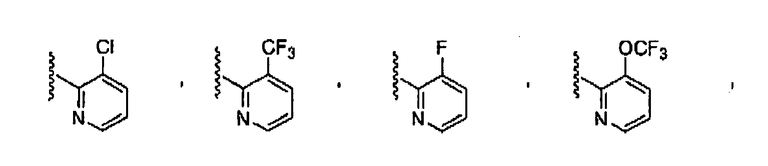 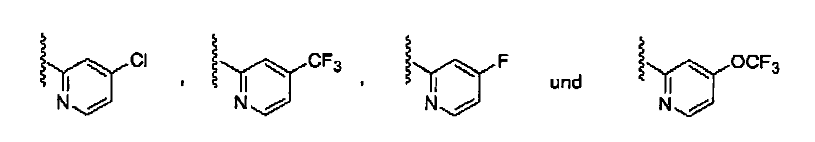 steht;
K für einen Rest ausgewählt aus der Gruppe bestehend aus 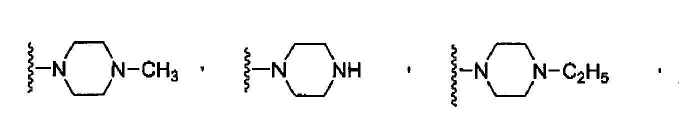 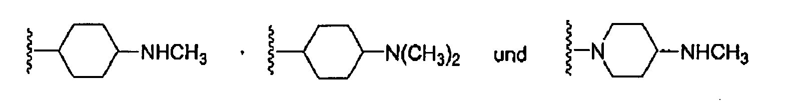 steht;
M für einen Rest ausgewählt aus der Gruppe bestehen aus 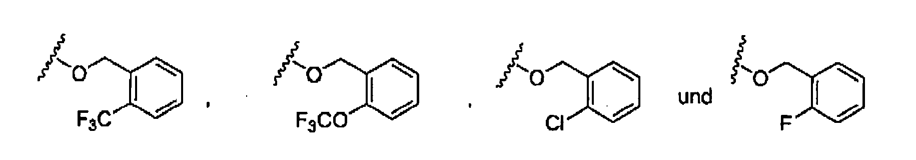 steht;
und
R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴ und R²⁵ jeweils für einen Wasserstoff-Rest stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

11. Verbindungen gemäß einem oder mehreren der Ansprüche 1 bis 10 ausgewählt aus der Gruppe bestehend aus
[1] N-{4-[3-(4-Pentafluorsulfanyl-benzyl)-thioureidomethyl]-2-fluor-phenyl}-methansulfonamid
[2] 4-(3-Chlor-pyridin-2-yl)-piperazin-1-carbonsäure-(4-pentafluorsulfanyl-phenyl)-amid
[3] 4-(3-Chlor-pyridin-2-yl)-piperazin-l-thiocarbonsäure-(4-pentafluorsulfanyl-phenyl)-amid
[4] 3-(4-Pentafluorsulfanyl-phenyl)-N-(2,3-dihydro-benzo[1,4]dioxin-6-yl)-acrylamid
[5] N-(4-Pentafluorsulfanyl-benzyl)-2-(3-fluor-4-methansulfonylamino-phenyl)-propionamid
[6] (2R)-N-(4-Pentafluorsulfanyl-benzyl)-2-(3-fluor-4-methansulfonylamino-phenyl)-propionamid
[7] (2S)-N-(4-Pentafluorsulfanyl-benzyl)-2-(3-fluor-4-methansulfonylamino-phenylrpropionamid
[8] N-(4-Pentafluorsulfanyl-phenyl)4-pyridin-2-yl-benzamid
[9] 1-(4-Pentafluorsulfanyl)-benzyl)-3-(7-hydroxy-naphthalen-1-yl))-harnstoff
[10] 4-(3-Chlor-pyrazin-2-yl)-3,6-dihydro-2H-pyridin-1-carbonsäure-(4-pentafluorsulfanyl-phenyl)-amid
[11] (4-Pentafluorsulfanyl-phenyl)-[4-(4-methyl-piperazin-1-yl)-6-(2-trifluorrnethyl-benzyloxy)-[1,3,5]triazin-2-yl]-amin
[12] (4-Pentafluorsulfanyll-phenyl)-[6-(4-chlor-phenyl)-pyrimidin-4-yl]-amin
[13] 4-Pentafluorsulfanyl-N-(2-oxo-2,3-dihydro-1H-benzoimidazol-5-ylmethyl)-benzamid
[14] (4-Pentafluorsulfanyl-phenyl)-{6-[4-(3-chlor-pyridin-2-yl)-2-(R)-methylpiperazin-1-yl]-pyrimidin-4-yl}-amin
[15] 3'-Chlor-3,6-dihydro-2H-[1,2']bipyridinyl-4-carbonsäure-(4-pentafluorsulfanyl-phenyl)-amid
[16] 2-(4-Pentaftuorsulfany-phenylamino)-N-(7-hydroxy-naphthalen-1-yl)-acetamid
[17] (4-Pentafluorsutfanyl-phenyl)-[7-(3-trifluormethyl-pyridin-2-yl)-[1,2,4]triazolo[4,3-b]pyridazin-3-yl]-amin
[18] 1-(4-Pentafluorsulfanyl-benzyl)-3-isochinolin-5-yl-harnstoff
[19] 1-[2-(4-Pentafluorsulfanyl-phenylrethyl]-3-isochinolin-5-yl-harnstoff
[20] 1-(4-Pentafluorsulfanyl-phenyl)-3-(7-hydroxy-5,6,7,8-tetrahydro-naphthalen-1-yl)-harnstoff
[21] 1-[2-(4-Pentafluorsulfanyl-phenyl)-ethyl]-3-chinolin-5-yl-harnstoff
[22] 1-(4-Pentafluorsulfanyl-phenyl)-3-(1-isochinolin-5-yl-pyrrofidin-3-yl)-harnstoff
[23] 5-Pentafluorsulfanyl-2-[4-(3-chlor-pyridin-2-ylypiperazin-1-yl]-benzooxazol und
[24] N-{4-[3-(4-Pentafluorsulfanyl-benzyl)-ureidomethyl]-2-fluor-phenyl}-methansulfonamid.

12. Verfahren zur Herstellung von Verbindungen der allgemeinen Formel I gemäß einem oder mehreren der Ansprüche 1 bis 11, **dadurch gekennzeichnet, dass** wenigstens eine Verbindung der allgemeinen Formel II 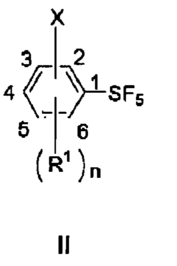 worin R¹ und n die Bedeutung gemäß einem oder mehreren der Ansprüche 1 bis 11 haben und X für -N=C=O, -N=C=S, -(CH₂)-N=C=O, -(CH₂)-N=C=S, - (CH₂)-N=C=S, -(CH₂)-(CH₂)-N=C=O, -(CH₂)-(CH₂)-N=C=S, -CH(CH₃)-N=G=O oder-CH(CH₃)-N=C=S steht, in einem Reaktionsmedium, in Gegenwart wenigstens einer Base, bevorzugt in Gegenwart wenigstens einer organischen Base ausgewählt aus der Gruppe bestehend aus Triethylamin, Diisopropylethylamin, N-Methylmorpholin, Pyridin und Dimethylaminopyridin, mit wenigstens einer Verbindung der allgemeinen Formel H-B-D; wobei die Gruppe H-B wenigstens eine -N(H)-Gruppe aufweist, H₂N-B-D, H₂N-F, H₂N-(CH₂)-F, H₂N-(CH₂)-(CH₂)-F oder H₂N-CH(CH₃)-F; wobei B, D und F die Bedeutung gemäß einem oder mehreren der Ansprüche 1 bis 11 haben;
oder wenigstens eine Verbindung der allgemeinen Formel II, worin R¹ und n die Bedeutung gemäß einem oder mehreren der Ansprüche 1 bis 11 haben und X für-NH_{2,} -(CH₂)NH₂, -CH(CH₃)-NH₂ oder-(CH₂)-(CH₂)-Nh₂ steht, in einem Reaktionsmedium, in Gegenwart wenigstens einer Base, bevorzugt in Gegenwart wenigstens einer organischen Base ausgewählt aus der Gruppe bestehend aus Triethylamin, Düsopropylethylamin, N-Methylmorpholin, Pyridin und Dimethylaminopyridin, mit wenigstens einer Verbindung der allgemeinen Formel F-N=C=O, F-N=C=S, F-(CH₂)N=C=O, F-(CH₂)-N=C=S, F-(CH)₂-(CH₂)-N=C=O, F-(CH₂)-(CH₂)-N=C=S. F-CH(CH₃)N=C=O oder F-CH(CH₃)-N=C=S; wobei F die vorstehend genannte Bedeutung hat;
zu wenigstens einer Verbindung der allgemeinen Formel I; worin R¹ und n die vorstehend genannte Bedeutung haben und R² für A-B-D oder -E-F steht, wobei A für -N(R³)-C(=O)-N(R⁴)- oder -N(R³)-C(=S)-N(R⁴)- steht; E für einen Rest ausgewählt aus der Gruppe bestehend aus -(CH₂)-(CH₂)-N(R⁷)-C(=O)-N(R⁸)-, -(CH₂)-(CH₂)-N(R⁷)-C(=S)-N(R⁸)-, -(CH2)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂) -(CH₂)-(CH₂)-N(R¹²)C(=S)-N(R¹³)-CH₂)-(CH₂)-, -(CH₂)-N(R¹⁴)-C( =O)-N(R¹⁵)-(CH₂)-,-(CH₂)-N(R¹⁴)-(=S)-N(R¹⁵)-(CH₂)-, -N(R¹⁶)-C(=O)-N(R¹⁷)-, -N(R¹⁶)-C(=S)-N(R¹⁷), -(CH₂)-N(R²⁴)-C(=O)-N(R²⁵)-CH(CH₃)- und -(CH₂)-N(R²⁴)-C(=S)_ N(R²⁵)-CH(CH₃)- steht; wobei R³, R⁴, R⁷, R⁸, R⁹, R¹⁰, R¹², R¹³, R¹⁴, R¹⁵ R¹⁶, R¹⁷, R²⁴ und R²⁵ jeweils für Wasserstoff stehen; und B, D und F die Bedeutung gemäß einem oder mehreren der Ansprüche 1 bis 11 haben; umgesetzt wird und diese ggf. gereinigt und/oder isoliert wird,
und ggf. wenigstens einer Verbindung der allgemeinen Formel 1₋ worin R¹ und n die vorstehend genannte Bedeutung haben und R² für -A-B-D oder -E-F steht, wobei A für -N(R³)-C(=O)-N(R⁴)- oder -N(R³)-C(=S)-N(R⁴)- steht; E für einen Rest ausgewählt aus der Gruppe bestehend aus -(CH₂)-(CH₂)-N(R⁷)-C(=O)-N(R⁸)-, -(CH₂)-(CH₂)-N(R⁷-C(=S)-N(R⁸)- -(CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=CO)-N(R¹³)-(CH₂)-(CH₂)-, -(CH₂)-(CH₂)-N(R¹²)-C(=S)-N(R¹³)-(CH₂)-(CH₂)- -(CH₂)-N(R¹⁴)-C(=O)-N(R¹⁵)-(CH₂)-(CH₂)-N(R¹⁴)-C(=S)-N(R¹⁵)-(CH₂)-, -N(R¹⁶)-C(=O)-N(R¹⁷)- -N(R¹⁶)-C(=S)-N(R¹⁷)-, -(CH₂)-N(R²⁴)-C(=O)-N(R²⁵)-CH(CH₃)- und -(CH₂)-N(R²⁴)-C(=S)-N(R²⁵)-CH(CH₃)- steht; wobei R³, R⁴, R⁷, R⁸, R⁹, R^{1o}, R¹², R¹³, R¹⁴ , R¹⁵, R¹⁶, R¹⁷ , R²⁴ und R²⁵ jeweils für Wasserstoff stehen; und B, D und F die Bedeutung gemäß einem oder mehreren der Ansprüche 1 bis 11 haben; in einem Reaktionsmedium in Gegenwart einer Base, bevorzugt in Gegenwart eines Metallhydridsalzes, besonders bevorzugt in Gegenwart von Kalium- und/oder Natriumhydrid, mit wenigstens einer Verbindung der allgemeinen Formel LG-R³, LG-R⁴, LG-R⁷, LG-R⁸, LG-R⁹, LG-R¹⁰, LG-R¹² , LG-R¹³, LG-R¹⁴ , LG-R¹⁵, LG-R¹⁶, LG-R¹⁷, LG-R²⁴ und LG-R²⁵; wobei R³, R⁴, R⁷, R⁸_{.} R⁹, R¹⁰, R¹², R¹³ R¹⁴, R¹⁵, R¹⁶, R¹⁷, R²⁴ und R²⁵ die Bedeutung gemäß einem oder mehreren der Ansprüche 1 bis 11 mit Ausnahme von Wasserstoff haben und LG für eine Abgangsgruppe, vorzugsweise für ein Halogenatom, besonders bevorzugt für ein Chloratom steht, zu wenigstens einer Verbindung der allgemeinen Formel 1, worin R¹ und n die vorstehend genannte Bedeutung haben und R² für A-B-D oder-E-F steht, wobei A für -N(R³)-C(=O)-N(R⁴)- oder -N(R³)-C(=S)-N(R⁴)-steht; E für einen Rest ausgewählt aus der Gruppe bestehend aus -(CH₂)-(CH₂)-N(R⁷)-C(=O)-N(R⁸h -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂(CH₂)-, -(CH₂)-(CH₂)-N(R¹²)C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-CH₂)- -N(R¹⁶)-C( =O)-N(R¹⁷)-, -N(R¹⁶)-C(=S)-N(R¹⁷)-, -(CH₂)-N(R²⁴)-C(=O)-N(R²⁵)-CH(CH₃)- und -(CH₂)-N(R²⁴)-C(=S)-N(R²⁵)-CH(CH₃)- steht; wobei R³, R⁴, R⁷, R⁸, R⁹, R¹⁰, R¹², R¹³, R¹⁴, R¹⁵, R¹⁶, R¹⁷, R²⁴ und R²⁵ jeweils die Bedeutung gemäß einem oder mehreren der Ansprüche 1 bis 11 mit Ausnahme von Wasserstoff haben; und B, D und F die Bedeutung gemäß einem oder mehreren der Ansprüche 1 bis 11haben; umgesetzt wird und diese ggf. gereinigt und/oder isoliert wird.

13. Verfahren zur Herstellung von Verbindungen der allgemeinen Formel I gemäß einem oder mehreren der Ansprüche 1 bis 11, **dadurch gekennzeichnet, dass** wenigstens einer Verbindung der allgemeinen Formel II, 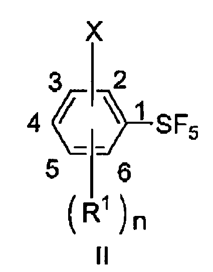 worin R¹ und n die Bedeutung gemäß einem oder mehreren der Ansprüche 1 bis 11 haben und X für -NH₂ steht, in einem Reaktionsmedium mit wenigstens einer Verbindung der Formel LG-G, worin G die Bedeutung gemäß einem oder mehreren der Ansprüche 1 bis 11 hat und LG für eine Abgangsgruppe, vorzugsweise ein Halogenatom, besonders bevorzugt ein Fluoratom oder ein Chloratom steht, zu wenigstens einer Verbindung der allgemeinen Formel 1, worin R¹ und n die vorstehend genannte Bedeutung haben und R²für-N(H)-G, wobei G die vorstehend genannte Bedeutung hat, steht, umgesetzt wird und diese ggf. gereinigt undloder/isoliert wird.

14. Verfahren zur Herstellung von Verbindungen der allgemeinen Formel gemäß einem oder mehreren der Ansprüche 1 bis 11, **dadurch gekennzeichnet, dass** wenigstens einer Verbindung der allgemeinen Formel III, 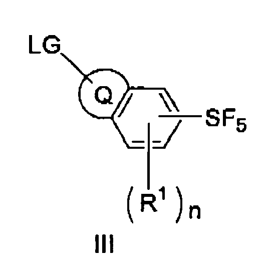 worin R¹, n und Q die Bedeutung gemäß einem oder mehreren der Ansprüche 1 bis 11 haben und LG für eine Abgangsgruppe, vorzugweise für ein Halogenatom, besonders bevorzugt für ein Fluoratom oder Chloratom steht, in einem Reaktionsmedium mit wenigstens einer Verbindung der allgemeinen Formel H-B-D; wobei die Gruppe H-B wenigstens eine -N(H)-Gruppe aufweist und B und D die Bedeutung gemäß einem oder mehreren der Ansprüche 1 bis 11 haben, zu wenigstens einer Verbindung der allgemeinen Formel I, worin R¹ und n die vorstehend genannte Bedeutung haben und R² für -Q-B-D steht, wobei Q, B und D die vorstehend genannte Bedeutung haben, umgesetzt wird und diese ggf. gereinigt und/oder isoliert wird.

15. Verfahren zur Herstellung von Verbindungen der allgemeinen Formell gemäß einem oder mehreren der Ansprüche 1 bis 11, **dadurch gekennzeichnet, dass** wenigstens einer Verbindung der allgemeinen Formel II, 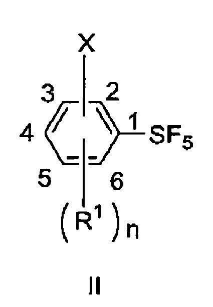 worin R¹ und n die Bedeutung gemäß einem oder mehreren der Ansprüche 1 bis 11 haben und X für -C(=O)-LG, -(CH₂)-C(=O)-LG, -(CH₂).(CH₂).C(=OFLG, -CH(CH,₃)-C(=O)-LG, -CH=CH-C(=O)-LG und -N(R²⁰)-CH₂₋C(=O)-LG steht, wobei LG für eine Abgangsgruppe, vorzugweise für ein Halogenatom, besonders bevorzugt für ein Chloratom steht und R²⁰ die Bedeutung gemäß einem oder mehreren der Ansprüche 1 bis 11 hat, in einem Reaktionsmedium, in Gegenwart wenigstens einer Base, bevorzugt in Gegenwart wenigstens einer organischen Base ausgewählt aus der Gruppe bestehend aus Triethylamin, Diisopropylethylamin, N-Methylmorpholin, Pyridin und Dimethylaminopyridin, mit wenigstens einer Verbindung der allgemeinen Formel H-B-D; wobei die Gruppe H-B wenigstens eine -N(H)-Gruppe aufweist, H₂N-B-D, H₂N-F, H₂N-(CH₂)-F, H₂N-(CH₂)-(CH₂)-F oder H₂N-CH(CH₃)-F; wobei B, D und F die Bedeutung gemäß einem oder mehreren der Ansprüche 1 bis 1 haben;
oder wenigstens eine Verbindung der allgemeinen Formel II, worin R¹ und n die Bedeutung gemäß einem oder mehreren der Ansprüche 1 bis 11 haben und X für -NH₂, -(CH₂)-NH₂, -CH(CH₃)-NH₂, -(CH₂)-(CH₂)-NH₂ oder -CH=CH-NH₂ steht, in einem Reaktionsmedium, in Gegenwart wenigstens einer Base, bevorzugt in Gegenwart wenigstens einer organischen Base ausgewählt aus der Gruppe bestehend aus Triethylamin, Diisopropylethylamin, N-Methylmorpholin, Pyridin und Dimethylaminopyridin, mit wenigstens einer Verbindung der allgemeinen Formel F-C(=O)-LG, F-(CH₂)-C(O)-LG, F-(CH₂)-(CH₂)-C(=O)-LG, F-CH(CH₃)-C(=O)-LG, F-CH=CH-C(=O)-LG und F-N(R²⁰)-CH₂-C(=O)-LG; wobei F, R²⁰ und LG die vorstehend genannte Bedeutung haben;
zu wenigstens einer Verbindung der allgemeinen Formel I, worin R¹ und n die vorstehend genannte Bedeutung haben und R² für A-B-D oder -E-F steht, wobei A für-N(R⁵)-C(=_{O})- steht; E für einen Rest ausgewählt aus der Gruppe bestehend aus -CH=CH-C(=O)-N(R¹¹), -(CH₂)-N(R¹⁸)-C(=O)-CH(CH₃)- -C(=O)-N(R¹⁹)-(CH₂)-, -N(R²⁰)-(CH₂)-C(=O)-N(R²¹)-, -(CH₂)-(CH₂hN(R²²)-C(=O)- und - CH(CH₃)-N(R²³)-C(=O(steht; wobei R^{5,} R¹¹, R¹⁸, R¹⁹, R²⁰, R²¹, R²² und R²³ jeweils für einen Wasserstoff-Rest stehen; und B, D und F die Bedeutung gemäß einem oder mehreren der Ansprüche 1 bis 11 haben; umgesetzt wird und diese ggf. gereinigt und/oder isoliert wird;
und ggf. wenigstens eine Verbindung der allgemeinen Formel I, worin R¹ und n die vorstehend genannte Bedeutung haben und R² für A-B-D oder -E-F steht, wobei A für-N(R⁵)-C(=O)- steht; E für einen Rest ausgewählt aus der Gruppe bestehend aus -CH=CH-C(=O)-N(R¹¹), -(CH₂)-N(R¹⁸)-C(=O)-CH(CH₃)-, -C(=O)-N(R¹⁹)-(CH₂)-,-N(R²⁰(CH₂)-C(=O)-N(R²¹)-, -(CH₂HCH₂)-N(R²²)-C(=O)- und - CH(CH₃)-N(R²³)-C(=O)- steht; wobei R⁵, R¹¹, R¹⁸, R¹⁹, R²⁰ , R²¹, R²² und R²³ jeweils für einen Wasserstoff-Rest stehen; und B, D und F die Bedeutung gemäß einem oder mehreren der Ansprüche 1 bis 11 haben; in einem Reaktionsmedium in Gegenwart einer Base, vorzugsweise in Gegenwart eines Metallhydridsalzes, besonders bevorzugt in Gegenwart von Kalium- und/oder Natriumhydrid mit wenigstens einer Verbindung der allgemeinen Formel LG-R⁵, LG-R¹¹, LG-R¹⁸, LG-R¹⁹, LG-R²⁰, LG-R²¹, LG-R²² und LG-R²³, wobei R⁵, R¹¹, R¹⁸, R¹⁹, R²⁰, R²¹, R²² und R²³ die Bedeutung gemäß einem oder mehreren der Ansprüche 1 bis 11 mit Ausnahme von Wasserstoff haben, zu wenigstens einer Verbindung der allgemeinen Formel I, worin R¹ und n die vorstehend genannte Bedeutung haben und R² für -A-B-D oder -E-F steht, wobei A für -N(R⁵)-C(=O)- steht; E für einen Rest ausgewählt aus der Gruppe bestehend aus - CH=CH.C(=O).N(R¹¹), -(CH₂)-N(R¹⁸)-C(=O)-CH(CH₃)-, -C(=O)-N(R¹⁹)-(CH₂)-, - N(R²⁰)-(CH₂)-C(=O)-N(R²¹)-, -(CH)₂-(CH₂)-N(R²²)-C(=O)- und -CH(CH₃)-N(R²³)-C(=O)- steht; wobei R⁵, R¹¹, R¹⁸, R¹⁹, R²⁰, R²¹, R²² und R²³ die Bedeutung gemäß einem oder mehreren der Ansprüche 1 bis 11 haben; und B, D und F die Bedeutung gemäß einem oder mehreren der Ansprüche 1 bis 11 haben; umgesetzt wird und diese ggf. gereinigt und/oder isoliert wird;
und ggf. eine Verbindung der allgemeinen Formel I, worin R¹ und n die vorstehend genannte Bedeutung haben und R² für-A-B-D oder-E-F steht, wobei A für -N(R⁵)-C(=O)- steht; E für einen Rest ausgewählt aus der Gruppe bestehend aus -CH=CH-C(=O)-N(R¹¹, -(CH₂)-N(R¹⁸)-C(=O)-CH(CH₃)-, - C(=O)-N(R¹⁹)-(CH₂)-, -N(R²⁰)-(CH₂)-C(=O)-N(R²¹)-, -(CH₂)-(CH₂)-N(R²²)-C(=O)- und -CH(CH₃)-N(R²³)-C(=O)- steht; wobei R⁵, R¹¹, R¹⁸, R¹⁹, R²⁰, R²¹, R²² und R²³ die Bedeutung gemäß einem oder mehreren der Ansprüche 1 bis 11 haben; und B, D und F die Bedeutung gemäß einem oder mehreren der Ansprüche 1 bis 11 haben; in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, mit wenigstens einer Verbindung der allgemeinen Formel IV, 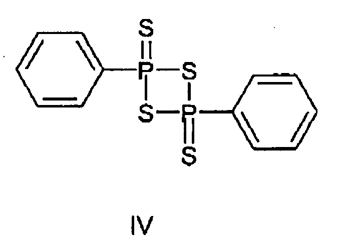 worin die Phenyl-Reste jeweils mit 1 oder 2 Substituenten unabhängig voneinander ausgewählt aus der Gruppe bestehend aus Methoxy, Phenoxy, CI, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfd,
zu wenigstens einer Verbindung der allgemeinen Formel 1, worin R¹ und n die vorstehend genannte Bedeutung haben und R² für -A-B-D oder -E-F steht, wobei A für -N(R⁵)-C(=S)- steht; E für einen Rest ausgewählt aus der Gruppe bestehend aus -CH=CH-C(=S)-N(R¹¹), -(CH₂)-N(R¹⁸)-C(=S)-CH(CH₃)-, -C(=S)-N(R¹⁹)-(CH₂)-, -N(R²⁰)-(CH₂)-C(=S)-N(R²¹)-, -(CH₂)-(CH₂)-N(R²²)-C(=S)- und - CH(CH₃)-N(R²³)-C(=S)- steht; wobei R⁵, R¹¹, R¹⁸, R¹⁹, R²⁰, R²¹, R²² und R²³ die Bedeutung gemäß einem oder mehreren der Ansprüche 1 bis 11 haben; und B, D und F die Bedeutung gemäß einem oder mehreren der Ansprüche 1 bis 11 haben; umgesetzt wird und diese ggf. gereinigt und/oder isoliert wird.

16. Verfahren zur Herstellung von Verbindungen der allgemeinen Formel Gemäß einem-oder mehreren-der Ansprüche 1 bis 11, **dadurch gekennzeichnet, dass** wenigstens einer Verbindung der allgemeinen Formel II, 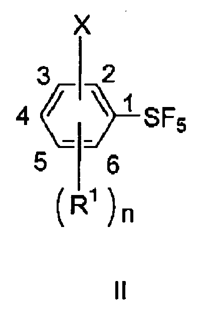 worin R¹ und n die Bedeutung gemäß einem oder mehreren der Ansprüche 1 bis 11 haben und X für-NH₂ oder -NHR⁶ steht; wobei R⁶ die Bedeutung gemäß einem oder mehreren der Ansprüche 1 bis 11 hat; in einem Reaktionsmedium mit wenigstens einer Verbindung der allgemeinen Formel AB-D, worin B und D die Bedeutung gemäß einem oder mehreren der Ansprüche 1 bis 11 haben und A für einen Rest ausgewählt aus der Gruppe bestehend aus 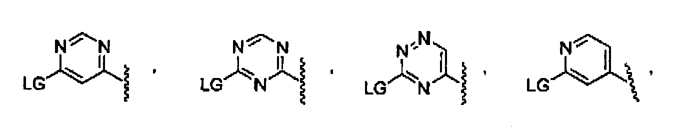 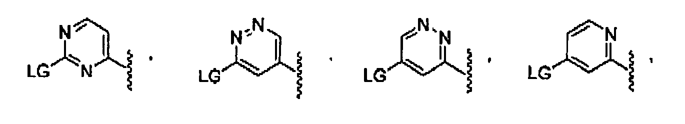 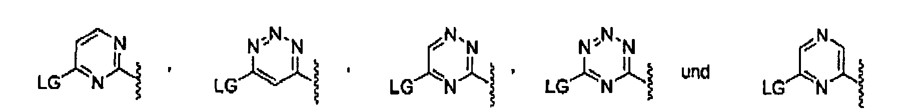 steht; wobei LG für eine Abgangsgruppe, vorzugsweise für ein Halogenatom, besonders bevorzugt für ein Fluoratom oder Chloratom steht, in einem Reaktionsmedium, vorzugsweise in einem Reaktionsmedium ausgewählt aus der Gruppe bestehend aus Dimethylformamid, Xylol, Mesitylen, Toluol, Acetonitril, Dichlormethan, Diethylether, Tetrahydrofuran und Dimethylsulfoxid, zu wenigstens einer Verbindung der allgemeinen Formel I, worin R¹, R⁶ und n die vorstehend genannte Bedeutung haben und R² für A-B-D steht, wobei B und D die vorstehend genannte Bedeutung haben und A die Bedeutung gemäß einem oder mehreren der Ansprüche 1 bis 11 mit Ausnahme von -N(R³)-C(=O)-N(R⁴)-, -N(R³)-C(=S)-N(R⁴)-, -N(R⁵)-C(=O)- und -N(R⁵)-C(=S)-, umgesetzt wird und diese ggf. gereinigt und/oder isoliert wird.

17. Arzneimittel enthaltend wenigstens eine Verbindung gemäß einem oder mehreren der Ansprüche 1 bis 11 und ggf. einen oder mehrere physiologisch verträgliche Hilfsstoffe.

18. Arzneimittel gemäß Anspruch 17 zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

19. Arzneimittel gemäß Anspruch 17 zur Prophylaxe und/oder Behandlung von einer oder mehreren Erkrankungen ausgewählt aus der Gruppe bestehend aus Migräne; Depressionen; Harninkontinenz; Stressinkontinenz; überaktive Blase (overactive bladder, OAB); Husten; Osteoporose; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; oder zur Prophylaxe und/oder Behandlung von Alkohol- und/oder Drogen- und/oder Medikamentenmißbrauch und Alkohol- und/oder Drogen- und/oder Nikotin- und/oder Medikamentenabhängigkeit, vorzugsweise zur Prophylaxe und/oder Verminderung von Entzugserscheinungen bei Alkohol- und/oder Drogen- und/oder Nikotin- und/oder Medikamentenabhängigkelt; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

20. Verwendung wenigstens einer Verbindung gemäß einem oder mehreren der Ansprüche 1 bis 11 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

21. Verwendung wenigstens einer Verbindung gemäß einem oder mehreren der Ansprüche 1 bis 11 zur Herstellung eines Arzneimittels zur Prophylaxe und/oder Behandlung von einer oder mehreren Erkrankungen ausgewählt aus der Gruppe bestehend aus Migräne; Depressionen; Harninkontinenz; Stressinkontinenz; überaktive Blase (overacitve bladder, OAB); Husten; Osteoporose; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; zur Prophylaxe und/oder Behandlung von Alkohol- und/oder Drogen- und/oder Medikamentenmißbrauch und Alkohol- und/oder Drogen- und/oder Nikotin- und/oder Medikamentenabhängigkeit, vorzugsweise zur Prophylaxe und/oder Verminderung von Entzugserscheinungen bei Alkohol- und/oder Drogen- und/oder Nikotin- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

## Claims

1. Pentafluorosulphanyl-substituted compounds of the general formula I, 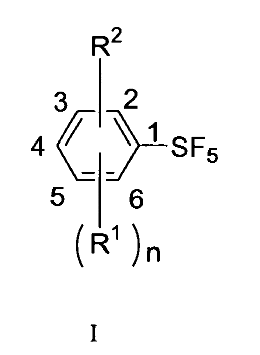 wherein
n is 0,1,2, 3 or 4;
R¹ represents one or more mutually independent groups selected from the following: H, F, Cl, Br, I, -CN, -NC, -NO₂, -SO₃H, -S(=O₂)NH₂, -NH₂, -OH, -SH, methyl, ethyl, n-propyl, isopropyl, n-butyl, sec-butyl, isobutyl, tert-butyl, n-pentyl, sec-pentyl, n-hexyl, -O-CH₃, -O-C₂H₅, -O-CH(CH₃)₂, -O-C(CH₃)₃, -S-CH₃, -S-C₂H₅, - S-CH(CH₃)₂, -S-C(CH₃)₃, -N(CH₃)₂, -N(C₂H₅)₂, -NH-CH₃, -NH-C₂H₅, -NH-C(=O)-CH₃, -NH-C(=O)-C₂Hs, -C(=O)-NH₂, -C(=O)-CH₃, -C(=O)-C₂Hₛ, -C(=O)-OH, - C(=O)-OCH₃ and -C(=O)-OC₂H₅;
R² represents a group -A-B-D, -E-F or -N(H)-G, which can be bound to position 2, 3, 4, 5 or 6 of the phenyl group of the general formula I;
or a group -Q-B-D; wherein Q represents a group selected from the following: oxazolyl, thiazolyl, isoxazolyl, isothiazolyl and imidazolyl, which is condensed with the phenyl group of the general formula I and thereby forms a possibly substituted group selected from the following: benzoxazolyl, benzothiazolyl, benzisothiazolyl and benzimidazolyl;
A represents a group selected from the following: -N(R³)-C(=O)-N(R⁴)-, -N(R³)-C(=S)-N(R⁴)-, -N(R⁵)-C(=O)-, -N(R⁵)-C(=S)-, 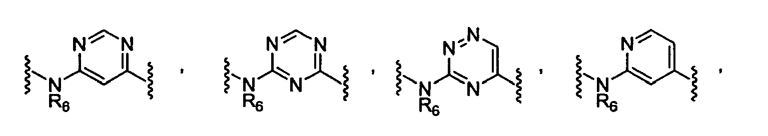 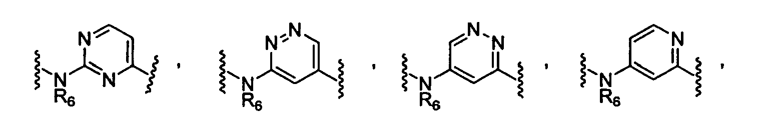 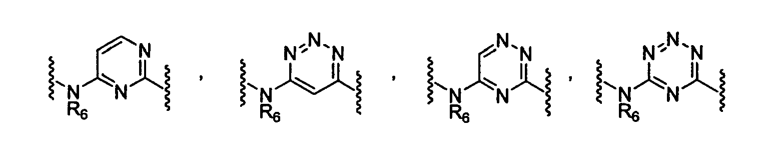 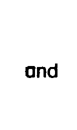 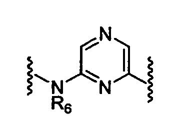
B represents a group selected from the following: 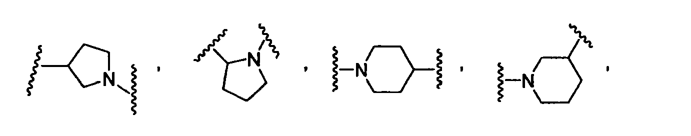 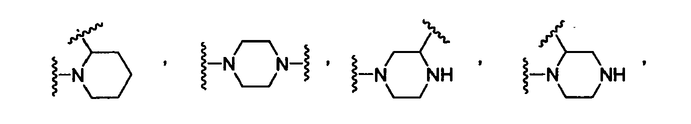 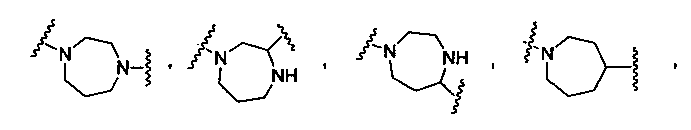 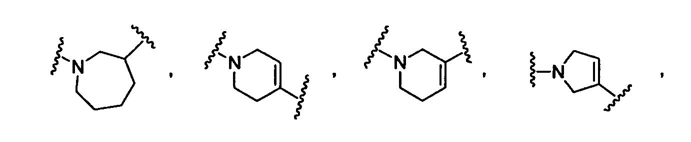 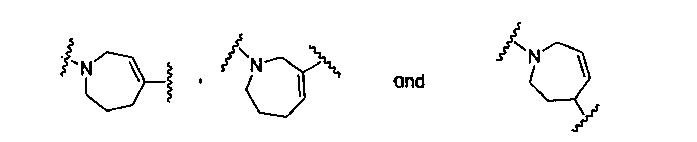 which may possibly be substituted with 1, 2, 3 or 4 substituents selected independently of one another from the following: methyl, ethyl, n-propyl, isopropyl, n-butyl, sec-butyl, isobutyl, tert-butyl, n-pentyl, sec-pentyl and n-hexyl;
or represents a phenylene group, which may be substituted with 1, 2, 3 or 4 substituents selected independently of one another from the following: F, Cl, Br, -CF₃, -OH, -O-CH₃, -O-C₂Hs, -O-CF₃, -O-CHF₂, -O-CF₂H, -S-CF₃, -S-CHF₂, -S-CF₂H, - NO₂, methyl, ethyl, n-propyl, isopropyl, n-butyl, sec-butyl, isobutyl and tert-butyl;
D represents a group selected from the following: phenyl, [1,3,5]-triazinyl, pyridinyl, pyridazinyl, pyrimidinyl, quinolinyl, isoquinolinyl and pyrazinyl, which may possibly be substituted with 1, 2, 3, 4 or 5 substituents selected independently of one another from the following: F, Cl, Br, -CF₃, -OH, -O-CH₃, -O-C₂Hₛ, -O-CF₃, -O-CHF₂, -O-CF₂H, -S-CF₃, -S-CHF₂, -S-CF₂H, -NO₂, methyl, ethyl, n-propyl, isopropyl, n-butyl, sec-butyl, isobutyl, tert-butyl, n-pentyl, sec-pentyl, n-hexyl, -NH-S(=O)₂-CH₃ and -NH-S(=O)₂-C₂Hs;
E represents a group selected from the following: -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰) -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, N(R¹⁶)-C(=O)-N(R¹⁷)-, -N(R¹⁶)-C(=S)-N(R¹⁷)-, (CH₂)-N(R¹⁸)-C(=O)-CH(CH₃)-, -(CH₂)-N(R¹⁸)-C(=S)-CH(CH₃)-, -C(=O)-N(R¹⁹)-(CH₂)-, -C(=S)-N(R¹⁹)-(CH₂)-, -N(R²⁰)-(CH₂)-C(=_{O})-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)₋, -(CH₂)-N(R²⁴)-C(=O)-N(R²⁵)-CH(CH₃)- and -(CH₂)-N(R²⁴)-C(=S)-N(R²⁵)-CH(CH₃)-;
F represents a group selected from the following: quinolinyl, isoquinolinyl, quinoxalinyl, quinazolinyl, (1,4)-benzodioxanyl, (1,3)-benzdioxolyl, (1,2,3,4)-tetrahydronaphthyl, (1,2,3,4)-tetrahydroquinolinyl, (1,2,3,4)-tetrahydroisoquinolinyl, benzoxazolyl, benzothiazolyl, benzisoxazolyl, benzisothiazolyl, benzimidazolyl, 2-oxo-2,3-dihydro-1H-benzimidazolyl, 2-benzoxazolinonyl and 2-benzothiazolinonyl, which may possibly be substituted with 1, 2, 3, 4 or 5 substituents selected independently of one another from the following: F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, methyl, ethyl, n-propyl, isopropyl, n-butyl, sec-butyl, isobutyl, tert-butyl, n-pentyl, sec-pentyl, n-hexyl, -NH-S(=O)₂-CH₃ and -NH-S(=O)₂-C₂Hs;
or represents a naphthyl group, which is substituted with 1, 2, 3, 4 or 5 substituents, selected independently of one another from the following: F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, methyl, ethyl, n-propyl, isopropyl, n-butyl, sec-butyl, isobutyl, tert-butyl, n-pentyl, sec-pentyl, n-hexyl, -NH-S(=O)₂-CH₃ or -NH-S(=O)₂-C₂Hs;
or represents a phenyl group, which is substituted with 2, 3, 4 or 5 substituents, selected independently of one another from the following: F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, methyl, ethyl, n-propyl, isopropyl, n-butyl, sec-butyl, isobutyl, tert-butyl, n-pentyl, sec-pentyl, n-hexyl, -NH-S(=O)₂-CH₃ or -NH-S(=O)₂-C₂H₅;
G represents a group selected from the following: 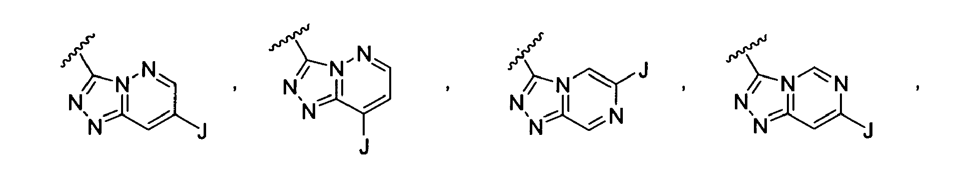 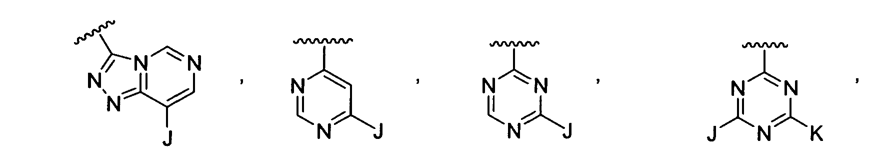 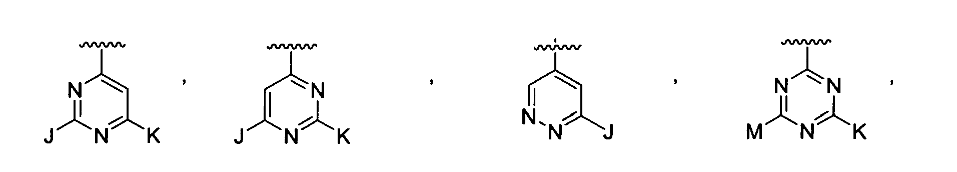 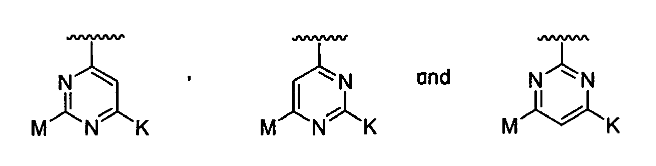
J represents a group selected from the following: phenyl, [1,3,5]-triazinyl, pyridinyl, pyrimidinyl, pyridazinyl, quinolinyl, isoquinolinyl and pyrazinyl, which may possibly be substituted with 1, 2, 3, 4 or 5 substituents selected independently of one another from the following: F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, methyl, ethyl, n-propyl, isopropyl, n-butyl, sec-butyl, isobutyl, tert-butyl, n-pentyl, sec-pentyl, n-hexyl, -NH-S(=O)₂-CH₃ and -NH-S(=O)₂-C₂Hs;
K represents a group selected from the following: piperidinyl, piperazinyl, pyrrolidinyl, morpholinyl, thiomorpholinyl, (1,2,3,6)-tetrahydropyridinyl, cyclohexyl, cyclopentyl, cycloheptyl, azepanyl and diazepanyl, which may be substituted with 1, 2, 3 or 4 substituents selected independently of one another from the following: methyl, ethyl, n-propyl, isopropyl, n-butyl, sec-butyl, isobutyl, tert-butyl, n-pentyl, sec-pentyl, n-hexyl, -NH-CH₃, -NH-C₂H₅, -N(CH₃)₂ and -N(C₂Hs);
or represents a -NR²⁶R²⁷-group;
M represents a phenyl group, which may be substituted with 1, 2, 3, 4 or 5 substituents selected independently of one another from the following: -SF₅, F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, methyl, ethyl, n-propyl, isopropyl, n-butyl, sec-butyl, isobutyl and tert-butyl and/or can be bound via a -O-CH₂-, -S-CH₂-, -CH₂-CH₂-, -CH=CH- or -CH₂-group;
R³ R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴ and R²⁵ represent, independently of one another,
a hydrogen group
or a group selected from the following: hydrogen, methyl, ethyl, n-propyl, isopropyl, n-butyl, sec-butyl, isobutyl, tert-butyl, n-pentyl, sec-pentyl and n-hexyl; and
R²⁶ and R²⁷ represent, independently of one another,
a group selected from the following: hydrogen, methyl, ethyl, n-propyl, isopropyl, n-butyl, sec-butyl, isobutyl, tert-butyl, n-pentyl, sec-pentyl and n-hexyl;
each possibly in the form of its pure stereoisomers, in particular enantiomers or diastereoisomers, its racemates or in the form of a mixture of stereoisomers, in particular enantiomers and/or diastereoisomers in any desired mixing ratio or in the form of corresponding salts, or respectively in the form of corresponding solvates.

2. Compounds according to claim 1, **characterised in that** n is equal to 0.

3. Compounds according to claims 1 or 2, **characterised in that** R² denotes a group -A-B-D, -E-F or -N(H)-G, which is bound to position 4 of the phenyl group of the general formula I,
or a group -Q-B-D; wherein Q represents a group selected from the following: oxazolyl, thiazolyl, isoxazolyl, isothiazolyl and imidazolyl, which is condensed with the phenyl group of the general formula I in positions 4 and 5 and therefore forms a possibly substituted group selected from the following: benzoxazolyl, benzothiazolyl, benzisothiazolyl and benzimidazolyl.

4. Compounds according to one or more of claims 1 to 3, **characterised in that**
B represents a group selected from the following:

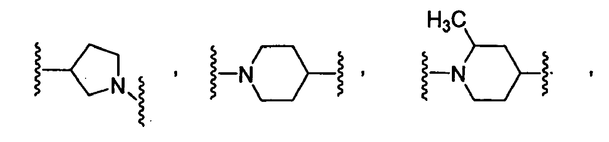

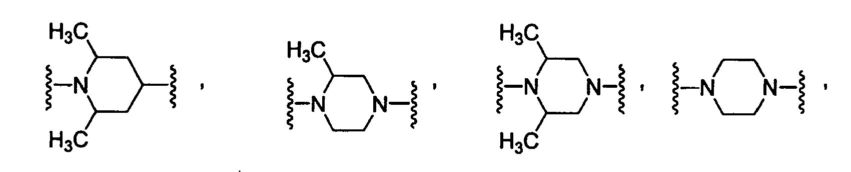

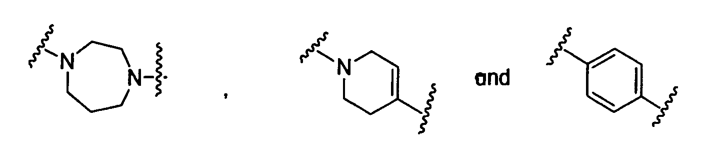

5. Compounds according to one or more of claims 1 to 4, **characterised in that**
D represents a group selected from the following:

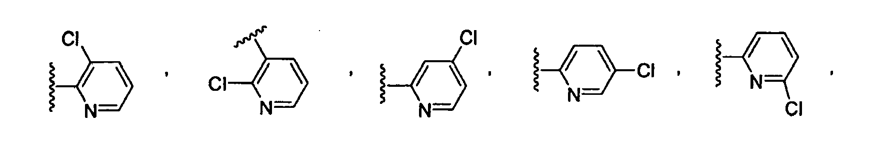

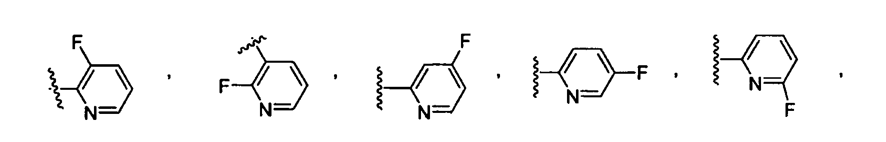

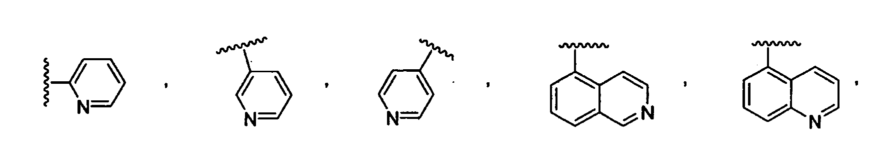

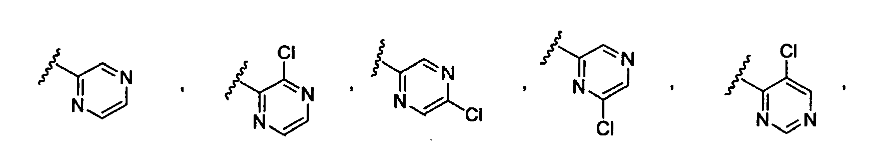

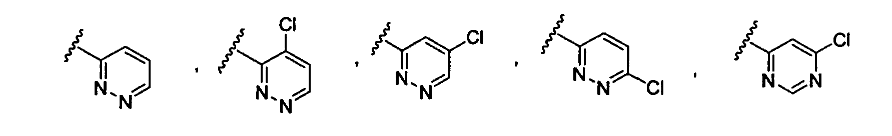

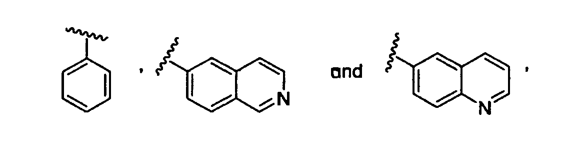

6. Compounds according to one or more of claims 1 to 5, **characterised in that**
F represents a group selected from the following:

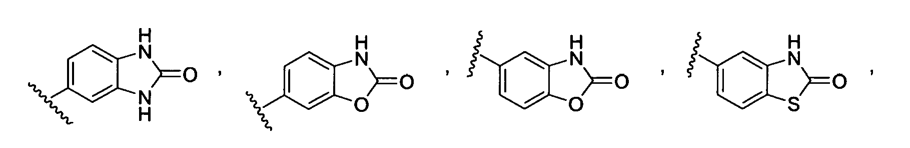

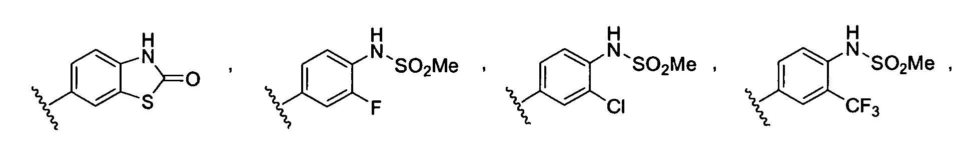

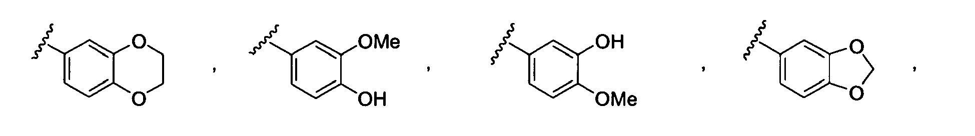

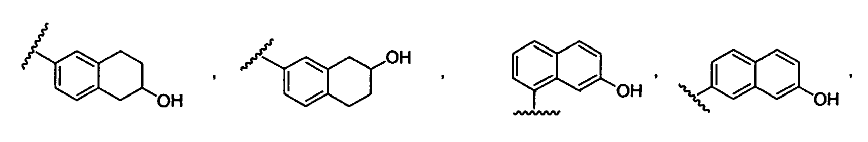

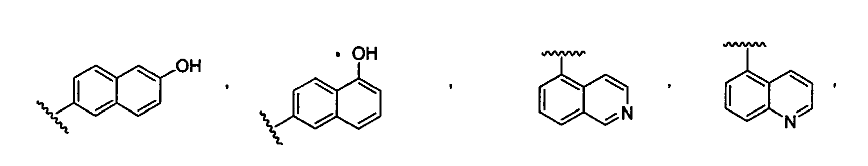

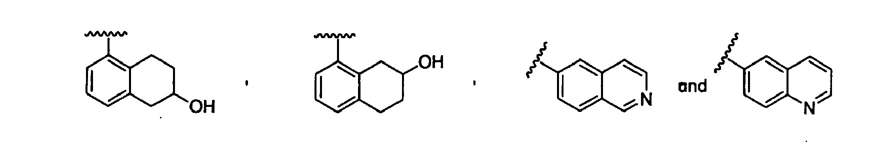

7. Compounds according to one or more of claims 1 to 6, **characterised in that**
J represents a group selected from the following:

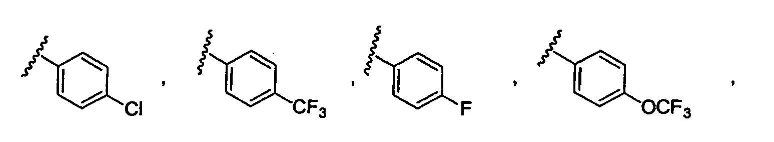

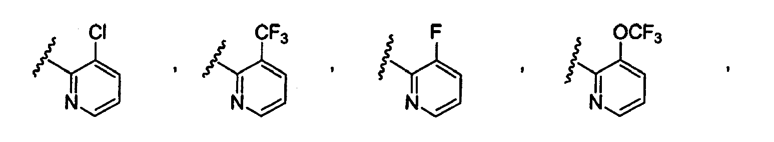

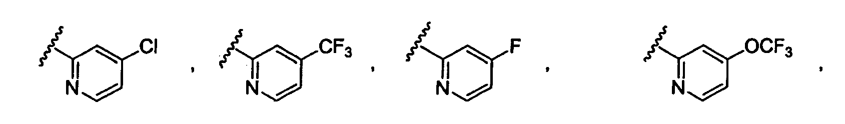

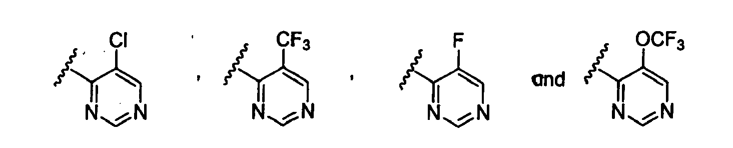

8. Compounds according to one or more of claims 1 to 7, **characterised in that**
K represents a group selected from the following:

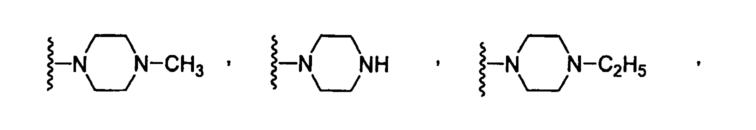

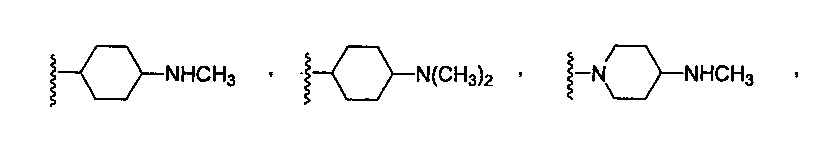

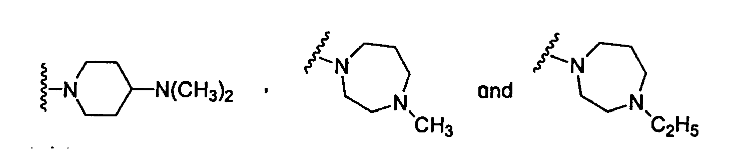

9. Compounds according to one or more of claims 1 to 8, **characterised in that**
M represents a group selected from the following:

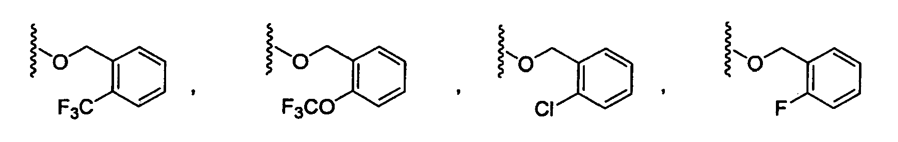

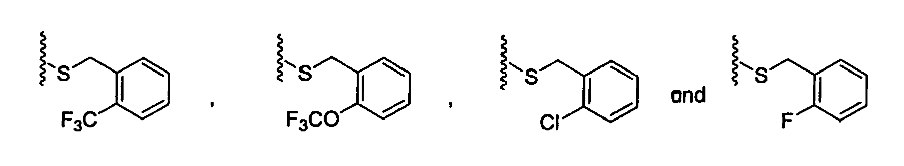

10. Compounds according to one or more of claims 1 to 9, **characterised in that**
n is equal to 0,
R² represents a group -A-B-D, -E-F or -N(H)-G, which can be bound to position 4 of the phenyl group of the general formula I;
or a group -Q-B-D; wherein Q represents a group selected from the following: oxazolyl, thiazolyl, isoxazolyl, isothiazolyl and imidazolyl, which is condensed with the phenyl group of the general formula I in positions 4 and 5 and thereby forms a possibly substituted group selected from the following: benzoxazolyl, benzothiazolyl, benzisothiazolyl and benzimidazolyl;
A represents a group selected from the following: -N(R³)-C(=O)-N(R⁴)-, -N(R³)-C(=S)-N(R⁴)-, -N(R⁵)-C(=O)-, -N(R⁵)-C(=S)-, 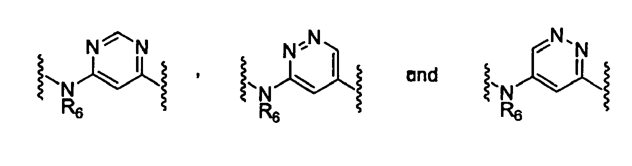
B represents a group selected from the following: 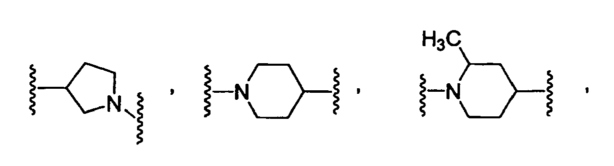 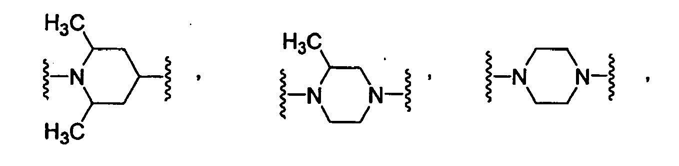 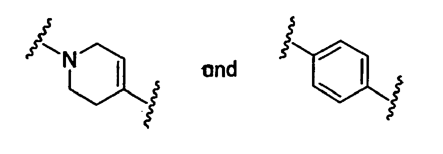
D represents a group selected from the following: 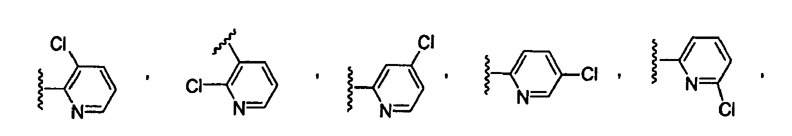 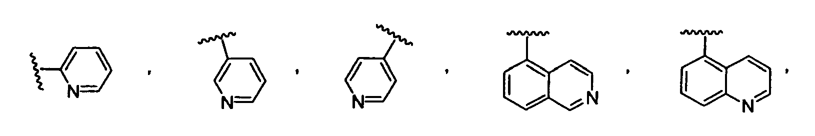 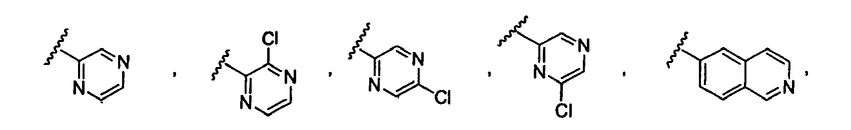 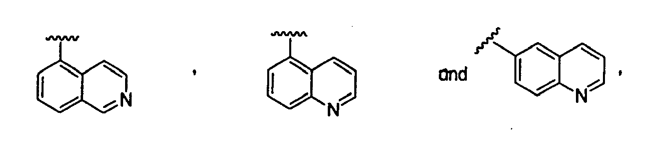
E represents a group selected from the following: -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴ )-C(=S)-N(R¹⁵)-(CH₂)-,N(R¹⁶)-C(=O)-N(R¹⁷)-, -N(R¹⁶)-C(=S)-N(R¹⁷)-, -(CH₂)-N(R¹⁸)-C(=O)-CH(CH₃)-, -(CH₂)-N(R¹⁸)-C(=S)-CH(CH₃)-, -C(=O)-N(R¹⁹)-(CH₂)-, -C(=S)-N(R¹⁹)-(CH₂)-, -N(R²⁰)-(CH₂)-C(=O)-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)-, -(CH₂)-N(R²⁴)-C(=O)-N(R^{2s})-CH(CH₃)- and -(CH₂)-N(R²⁴)-C(=S)-N(R²⁵)-CH(CH₃)-
F represents a group selected from the following: 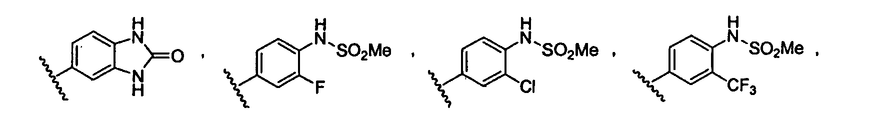 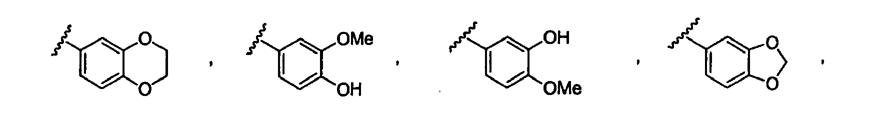 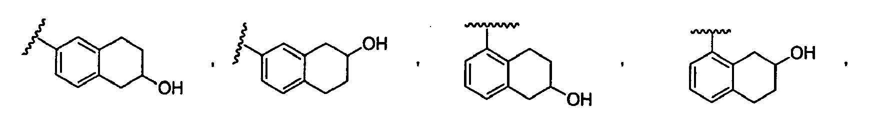 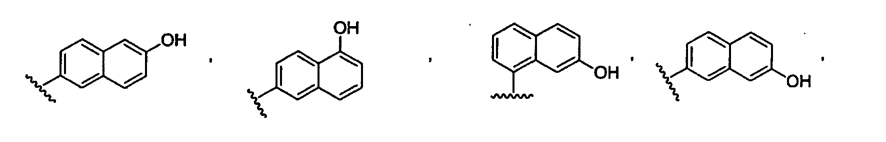 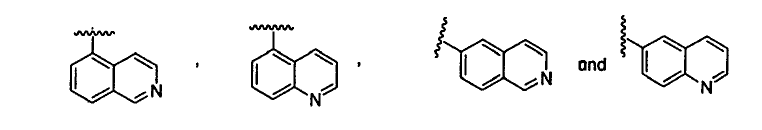
G represents a group selected from the following: 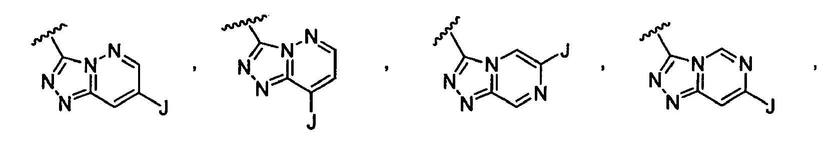 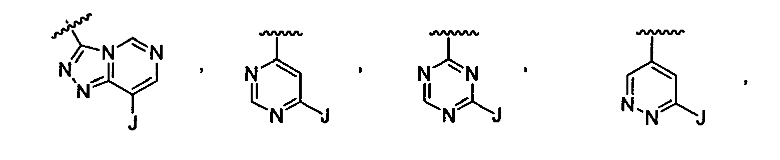 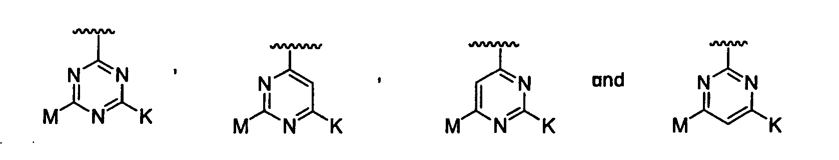
J represents a group selected from the following: 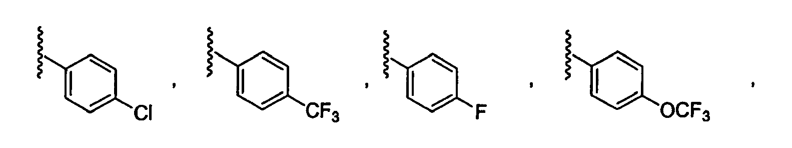 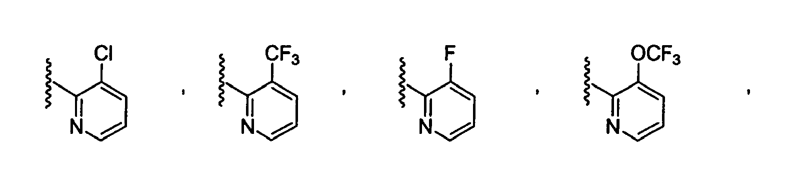 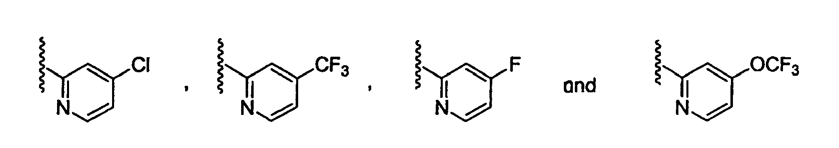
K represents a group selected from the following: 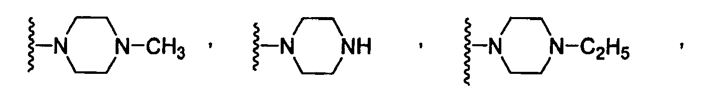 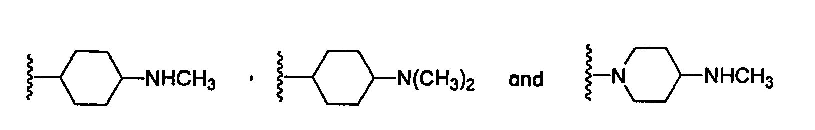
M represents a group selected from the following: 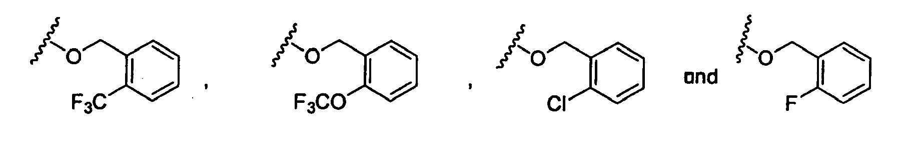 and
R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ R²¹ R²², R²³, R^{2a} and R²⁵ each represent a hydrogen group;
each possibly in the form of its pure stereoisomers, in particular enantiomers or diastereoisomers, its racemates or in the form of a mixture of stereoisomers, in particular enantiomers and/or diastereoisomers in any desired mixing ratio or in the form of corresponding salts, or respectively in the form of corresponding solvates.

11. Compounds according to one or more of claims 1 to 10, selected from the following:
[1] N-{4-[3-(4-pentafluorosulphanyl-benzyl)-thioureidomethyl]-2-fluoro-phenyl}-methane sulphonamide
[2] 4-(3-chloropyridin-2-yl)-piperazine-1-carboxy-(4-pentafluorosulphanyl-phenyl)-amide
[3] 4-(3-chloropyridin-2-yl)-piperazine-1-thiocarboxy-(4-pentafluorosulphanyl-phenyl)-amide
[4] 3-(4-pentafluorosulphanyl-phenyl)-N-(2,3-dihydro-benzo[1,4]dioxin-6-yl)-acrylamide
[5] N-(4-pentafluorosulphanyl-benzyl)-2-(3-fluoro-4-methanesulphonylamino-phenyl)-propionamide
[6] (2R)-N-(4-pentafluorosulphanyl-benzyl)-2-(3-fluoro-4-methanesulphonylamino-phenyl)-propionamide
[7] (2S)-N-(4-pentafluorosulphanyl-benzyl)-2-(3-fluoro-4-methanesulphonylamino-phenyl)-propionamide
[8] N-(4-pentafluorosulphanyl-phenyl)-4-pyridin-2-yl-benzamide
[9] 1-(4-pentafluorosulphanyl-benzyl)-3-(7-hydroxy-naphthalen-1-yl)-urea
[10] 4-(3-chloro-pyrazin-2-yl)-3,6-dihydro-2H-pyridine-1-carboxy-(4-pentafluorosulphanyl-phenyl)-amide
[11] (4-pentafluorosulphanyl-phenyl)-[4-(4-methyl-piperazin-1-yl)-6-(2-trifluoromethyl-benzyloxy)-[1,3,5]triazin-2-yl]-amine
[12] (4-pentafluorosulphanyl-phenyl)-[6-(4-chloro-phenyl)-pyrimidin-4-yl]-amine
[13] 4-pentafluorosulphanyl-N-(2-oxo-2,3-dihydro-1H-benzoimidazol-5-ylmethyl)-benzamide
[14] (4-pentafluorosulphanyl-phenyl)-{6-[4-(3-chloropyridin-2-yl)-2-(R)-methyl-piperazin-1-yl]-pyrimidin-4-yl}-amine
[15] 3'-chloro-3,6-dihydro-2H-[1,2']bipyridinyl-4-carboxy-(4-pentafluorosulphanyl-phenyl)-amide
[16] 2-(4-pentafluorosulphanyl-phenylamino)-N-(7-hydroxy-naphthalen-1-yl)-acetamide
[17] (4-pentafluorosulphanyl-phenyl)-[7-(3-trifluoromethyl-pyridin-2-yl)-[1,2,4]triazolo[4,3-bipyridazin-3-yl]-amine
[18] 1-(4-pentafluorosulphanyl-benzyl)-3-isoquinolin-5-yl-urea
[19] 1-[2-(4-pentafluorosulphanyl-phenyl)-ethyl]-3-isoquinolin-5-yl-urea
[20] 1-(4-pentafluorosulphanyl-phenyl)-3-(7-hydroxy-5,6,7,8-tetrahydro-naphthalen-1-yl)-urea
[21] 1-[2-(4-pentafluorosulphanyl-phenyl)-ethyl]-3-quinolin-5-yl-urea
[22] 1-(4-pentafluorosulphanyl-phenyl)-3-(1-isoquinolin-5-yl-pyrrolidin-3-yl)-urea
[23] 5-pentafluorosulphanyl-2-[4-(3-chloro-pyridin-2-yl)-piperazin-1-yl]-benzooxazole
and
[24] N-{4-[3-(4-pentafluorosulphanyl-benzyl)-ureidomethyl]-2-fluoro-phenyl}-methanesulphonamide.

12. Method for producing compounds of the general formula I according to one or more of claims 1 to 11, **characterised in that** at least one compound of the general formula II, 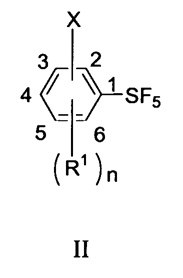
wherein R¹ and n denote the same as in one or more of claims 1 to 11 and
X represents -N=C=O, -N=C=S, -(CH₂)-N=C=O, -(CH₂)-N=C=S, -(CH₂)-N=C=S, - (CH₂)-(CH₂)-N=C=O, -(CH₂)-(CH₂)-N=C=S, -CH(CH₃)-N=C=O or -CH(CH₃)-N=C=S, is reacted in a reaction medium in the presence of at least one base, preferably in the presence of at least one organic base selected from the group comprising triethylamine, diisopropylethylamine, N-methylmorpholine, pyridine and dimethylaminopyridine, with at least one compound of the general formula H-B-D; wherein the group H-B has at least one -N(H)-group, H₂N-B-D, H₂N-F, H₂N-(CH₂)-F, H₂N-(CH₂)-(CH₂)-F or H₂N-CH(CH₃)-F; wherein B, D and F denote the same as in one or more of claims 1 to 11;
or at least one compound of the general formula II, wherein R¹ and n denote the same as in one or more of claims 1 to 11 and X represents -NH₂, -(CH₂)-NH₂, -CH(CH₃)-NH₂ or -(CH₂)-(CH₂)-NH₂, is reacted in a reaction medium in the presence of at least one base, preferably in the presence of at least one organic base selected from the group comprising triethylamine, diisopropylethylamine, N-methylmorpholine, pyridine and dimethylaminopyridine, with at least one compound of the general formula F-N=C=O, F-N=C=S, F-(CH₂)-N=C=O, F-(CH₂)-N=C=S, F-(CH)₂-(CH₂)-N=C=O, F-(CH₂)-(CH₂)-N=C=S, F-CH(CH₃)-N=C=O or F-CH(CH₃)-N=C=S; wherein F denotes the same as above;
and is converted to at least one compound of the general formula I; wherein R¹ and n denote the same as above and R² represents -A-B-D or -E-F, wherein A represents - N(R³)-C(=O)-N(R⁴)- or -N(R³)-C(=S)-N(R⁴)-; E represents a group selected from the following: -(CH₂)-(CH₂)-N(R⁷)-C(=O)-N(R⁸)-, -(CH₂)-(CH₂)-N(R⁷)-C(=S)-N(R⁸)-, - (CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, - (CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R²⁴)-C(=O)-N(R²⁵)-CH(CH₃)- and - (CH₂)-N(R²⁴)-C(=S)-N(R²⁵)-CH(CH₃)-; wherein R³, R⁴, R⁷, R⁸, R⁹, R¹⁰, R¹², R¹³, R¹⁴, R¹⁵, R¹⁶, R¹⁷ R²⁴ and R²⁵ each represent hydrogen; and B, D and F denote the same as in one or more of claims 1 to 11; and this compound is possibly purified and/or isolated,
and possibly at least one compound of the general formula I; wherein R¹ and n denote the same as above and R² represents -A-B-D or -E-F, wherein A represents -N(R³)-C(=O)-N(R⁴)- or -N(R³)-C(=S)-N(R⁴)-; E represents a group selected from the following: -(CH₂-(CH₂-N(R⁷)-C(=O)-N(R⁸)-, -(CH₂)-(CH₂)-N(R⁷)-C(=S)-N(R⁸)-, (CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, - (CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=_{O})-N(R¹⁷)-, -N(R¹⁶)-C(=S)-N(R¹⁷)-, -(CH₂)-N(R²⁴)-C(=O)-N(R²⁵)-CH(CH₃)- and - (CH₂)-N(R²⁴)-C(=S)-N(R²⁵)-CH(CH₃)-; wherein R³, R⁴, R⁷, R⁸, R⁹, R¹⁰, R¹², R¹³, R¹⁴, R¹⁵ R¹⁶ R¹⁷ R²⁴ and R²⁵ each represent hydrogen; and B, D and F denote the same as in one or more of claims 1 to 11; is reacted in a reaction medium, in the presence of a base, preferably in the presence of a metal hydride salt, particularly preferred in the presence of potassium hydride and/or sodium hydride, with at least one compound of the general formula LG-R³, LG-R⁴, LG-R ⁷, LG-R⁸, LG-R⁹, LG-R¹⁰, LG-R¹², LG-R¹³, LG-R¹⁴, LG-R¹⁵ LG-R¹⁶ LG-R¹⁷ LG-R²⁴ and LG-R²5. wherein R³, R⁴, R⁷, R⁸ R⁹, R¹⁰ R¹², R¹³, R¹⁴, R¹⁵, R¹⁶, R¹⁷, R²⁴ and R²⁵ denote the same as in one or more of claims 1 to 11 with the exception of hydrogen and LG denotes a leaving group, preferably a halogen atom, particularly preferred a chlorine atom, and is converted to at least one compound of the general formula I, wherein R¹ and n denote the same as above and R² represents -A-B-D or -E-F, wherein A represents -N(R³)-C(=O)-N(R⁴)- or -N(R³)-C(=S)-N(R⁴)-; E represents a group selected from the following: -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-,-N(R¹⁶)-C(=S)-N(R¹⁷)-, -(CH₂)-N(R²⁴)-C(=O)-N(R²⁵)-CH(CH₃)- and -(CH₂)-N(R2⁴⁾_ C(=S)-N(R²⁵)-CH(CH₃)-; wherein R³, R⁴, R⁷, R⁸, R⁹, R¹⁰, R¹² , R¹³, R¹⁴, R¹⁵, R¹⁶, R¹⁷, R²⁴ and R²⁵ each denote the same as in one or more of claims 1 to 11 with the exception of hydrogen; and B, D and F denote the same as in one or more of claims 1 to 11; and this compound is possibly purified and/or isolated.

13. Method for producing compounds of the general formula I according to one or more of claims 1 to 11, **characterised in that** at least one compound of the general formula II, 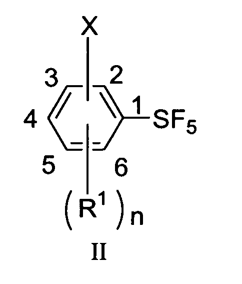 wherein R¹ and n denote the same as in one or more of claims 1 to 11 and X represents -NH₂, is reacted in a reaction medium with at least one compound of the formula LG-G, wherein G denotes the same as in one or more of claims 1 to 11 and LG denotes a leaving group, preferably a halogen atom, particularly preferred a fluorine atom or a chlorine atom, and is converted to at least one compound of the general formula I, wherein R¹ and n denote the same as above and R² represents -N(H)-G, wherein G denotes the same as above, and this compound is possibly purified and/or isolated.

14. Method for producing compounds of the general formula I according to one or more of claims 1 to 11, **characterised in that** at least one compound of the general formula III, 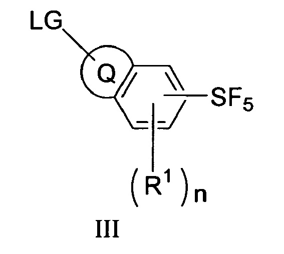 wherein R¹, n and Q denote the same as in one or more of claims 1 to 11 and LG denotes a leaving group, preferably a halogen atom, particularly preferred a fluorine atom or a chlorine atom, is reacted in a reaction medium with at least one compound of the general formula H-B-D; wherein the group H-B has at least one -N(H)- group and B and D denote the same as in one or more of claims 1 to 11, and is converted to at least one compound of the general formula I, wherein R¹ and n denote the same as above and R² represents -Q-B-D, wherein Q, B and D denote the same as above, and this compound is possibly purified and/or isolated.

15. Method for producing compounds of the general formula I according to one or more of claims 1 to 11, **characterised in that** at least one compound of the general formula II, 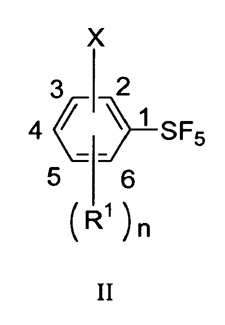 wherein R¹ and n denote the same as in one or more of claims 1 to 11 and X represents -C(=O)-LG, -(CH₂)-C(=O)-LG, -(CH₂)-(CH₂)-C(=O)-LG, -CH(CH₃)-C(=O)-LG, - CH=CH-C(=O)-LG and -N(R²⁰)-CH₂-C(=O)-LG, wherein LG denotes a leaving group, preferably a halogen atom, particularly preferred a chlorine atom and R²⁰ denotes the same as in one or more of claims 1 to 11, is reacted in a reaction medium in the presence of at least one base, preferably in the presence of at least one organic base selected from the group comprising triethylamine, diisopropylethylamine, N-methylmorpholine, pyridine and dimethylaminopyridine, with at least one compound of the general formula H-B-D; wherein the group H-B has at least one -N(H)- group, H₂N-B-D, H₂N-F, H₂N-(CH₂)-F, H₂N-(CH₂)-(CH₂)-F or H₂N-CH(CH₃)-F; wherein B, D and F denote the same as in one or more of claims 1 to 11;
or at least one compound of the general formula II, wherein R¹ and n denote the same as in one or more of claims 1 to 11 and X represents -NH₂, -(CH₂)-NH₂, -CH(CH₃)-NH₂, -(CH₂)-(CH₂)-NH₂ or -CH=CH-NH₂, is reacted in a reaction medium, in the presence of at least one base, preferably in the presence of at least one organic base selected from the group comprising triethylamine, diisopropylethylamine, N-methylmorpholine, pyridine and dimethylaminopyridine, with at least one compound of the general formula F-C(=O)-LG, F-(CH₂)-C(=O)-LG, F-(CH₂)-(CH₂)-C(=O)-LG, F-CH(CH₃)-C(=O)-LG, F-CH=CH-C(=O)-LG and F-N(R²¹)-CH₂-C(=O)-LG; wherein F, R²⁰ and LG denote the same as above;
to at least one compound of the general formula I, wherein R¹ and n denote the same as above and R² represents -A-B-D or -E-F, wherein A represents -N(R⁵)-C(=O)-; E represents a group selected from the following: -CH=CH-C(=O)-N(R¹¹), -(CH₂)-N(R¹⁸)-C(=O)-CH(CH₃)-, -C(=O)-N(R¹⁹)-(CH₂)-, -N(R²⁰)-(CH₂)-C(=O)-N(R²¹)-, - (CH₂)-(CH₂)-N(R²²)-C(=O)- and -CH(CH₃)-N(R²³)-C(=O)-; wherein R⁵, R¹¹, R¹⁸, R¹⁹, R²⁰, R²¹, R²² and R²³ each represent a hydrogen group; and B, D and F denote the same as in one or more of claims 1 to 11; and this compound is possibly purified and/or isolated;
and possibly at least one compound of the general formula I, wherein R¹ and n denote the same as above and R² represents -A-B-D or -E-F, wherein A represents -N(R⁵)-C(=O)-; E represents a group selected from the following: -CH=CH-C(=O)-N(R¹¹), - (CH₂)-N(R¹⁸)-C(=O)-CH(CH₃)-, -C(=O)-N(R¹⁹)-(CH₂)-, -N(R²⁰)-(CH₂)-C(=O)-N(R²¹)-, -(CH₂)-(CH₂)-N(R²²)-C(=O)- and -CH(CH₃)-N(R²³)-C(=O)-; wherein R⁵, R¹¹, R¹⁸, R¹⁹, R²⁰, R²¹, R²² and R²³ each represent a hydrogen group; and B, D and F denote the same as in one or more of claims 1 to 11; is reacted in a reaction medium in the presence of a base, preferably in the presence of a metal hydride salt, particularly preferred in the presence of potassium hydride and/or sodium hydride with at least one compound of the general formula LG-R⁵, LG-R¹¹, LG-R¹⁸, LG-R¹⁹, LG-R20, LG-R²¹, LG-R²² and LG-R²³, wherein R⁵, R¹¹, R¹⁸, R¹⁹, R²⁰, R²¹, R²² and R²³ denote the same as in one or more of claims 1 to 11 with the exception of hydrogen, and is converted to at least one compound of the general formula I, wherein R¹ and n denote the same as above and R² represents -A-B-D or -E-F, wherein A represents -N(R⁵)-C(=O)-; E represents a group selected from the following: -CH=CH-C(=O)-N(R¹¹), -(CH₂)-N(R¹⁸)-C(=O)-CH(CH₃)-, -C(=O)-N(R¹⁹)-(CH₂)-, -N(R²⁰)-(CH₂)-C(=O)-N(R²¹)-, - (CH₂)-(CH₂)-N(R²²)-C(=O)- and -CH(CH₃)-N(R²³)-C(=O)-; wherein R⁵, R¹¹, R¹⁸, R¹⁹, R²⁰, R²¹, R²² and R²³ denote the same as in one or more of claims 1 to 11; and B, D and F denote the same as in one or more of claims 1 to 11; and this compound is possibly purified and/or isolated;
and possibly a compound of the general formula I, wherein R¹ and n denote the same as above and R² represents -A-B-D or -E-F, wherein A represents -N(R⁵)-C(=O)-; E represents a group selected from the following: -CH=CH-C(=O)-N(R¹¹), -(CH₂)-N(R¹⁸)-C(=O)-CH(CH₃)-, -C(=O)-N(R¹⁹)-(CH₂)-, -N(R²⁰)-(CH₂)-C(=O)-N(R²¹)-, - (CH₂)-(CH₂)-N(R²²)-C(=O)- and -CH(CH₃)-N(R²³)-C(=O)-; wherein R⁵, R¹¹, R¹⁸ , R¹⁹, R²⁰, R²¹, R²² and R²³ denote the same as in one or more of claims 1 to 11; and B, D and F denote the same as in one or more of claims 1 to 11; is reacted in a reaction medium, preferably in a reaction medium selected from the group comprising dimethylformamide, xylene, mesitylene, toluene, acetonitrile, dichloromethane, diethyl ether, tetrahydrofuran and dimethyl sulphoxide, with at least one compound of the general formula IV, 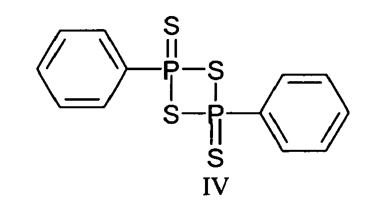 wherein the phenyl groups are each substituted with 1 or 2 substituents selected independently of one another from the group comprising methoxy, phenoxy, Cl, methyl and Br, preferably with a phenoxy group or a methoxy group and particularly preferred with a methoxy group in the para-position, or with phosphorus pentasulphide,
and is converted to at least one compound of the general formula I, wherein R¹ and n denote the same as above and R² represents -A-B-D or -E-F, wherein A represents - N(R⁵)-C(=S)-; E represents a group selected from the following: -CH=CH-C(=S)-N(R"), -(CH₂)-N(R¹⁸)-C(=S)-CH(CH₃)-, -C(=S)-N(R¹⁹)-(CH₂)-, -N(R²⁰)-(CH₂)-C(=S)-N(R²¹)-, -(CH₂)-(CH₂)-N(R²²)-C(=S)- and -CH(CH₃)-N(R²³)-C(=S)-; wherein R⁵, R¹¹, R¹⁸, R¹⁹, R²⁰, R²¹, R²² and R²³ denote the same as in one or more of claims 1 to 11; and B, D and F denote the same as in one or more of claims 1 to 11; and this compound is possibly purified and/or isolated.

16. Method for producing compounds of the general formula I according to one or more of claims 1 to 11, **characterised in that** at least one compound of the general formula II, 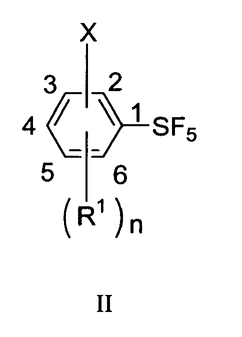 wherein R¹ and n denote the same as in one or more of claims 1 to 11 and X represents -NH₂ or -NHR⁶; wherein R⁶ denotes the same as in one or more of claims 1 to 11; is reacted in a reaction medium with at least one compound of the general formula A-B-D, wherein B and D denote the same as in one or more of claims 1 to 11 and A represents a group selected from the following: 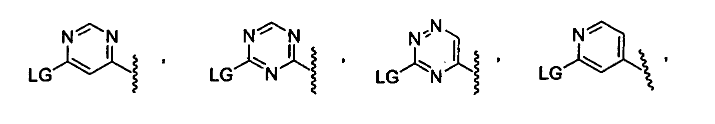 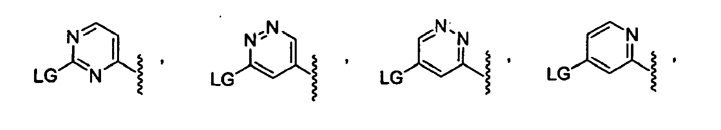 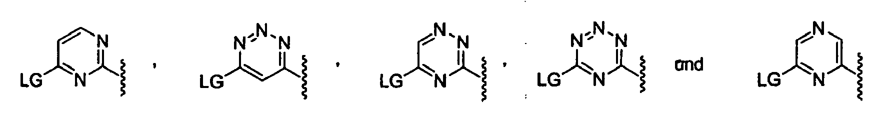 wherein LG denotes a leaving group, preferably a halogen atom, particularly preferred a fluorine atom or a chlorine atom, in a reaction medium, preferably in a reaction medium selected from the group comprising dimethylformamide, xylene, mesitylene, toluene, acetonitrile, dichloromethane, diethyl ether, tetrahydrofuran and dimethyl sulphoxide, and is converted to at least one compound of the general formula I, wherein R¹, R⁶ and n denote the same as above and R² represents -A-B-D, wherein B and D denote the same as above and A denotes the same as in one or more of claims 1 to 11 with the exception of -N(R³)-C(=O)-N(R⁴)-, -N(R³)-C(=S)-N(R⁴)-, -N(R⁵)-C(=O)- and -N(R⁵)-C(=S)-, and this compound is possibly purified and/or isolated.

17. Medication comprising at least one compound according to one or more of claims 1 to 11 and possibly one or more physiologically compatible adjuvants.

18. Medication according to claim 17 for the prophylaxis and/or treatment of pain, preferably pain selected from the group comprising acute pain, chronic pain, neuropathic pain and visceral pain.

19. Medication according to claim 17 for the prophylaxis and/or treatment of one or more disorders selected from the group comprising migraine; depression; urinary incontinence; stress incontinence; overactive bladder (OAB); coughing; osteoporosis; neurodegenerative diseases, preferably from the group comprising Parkinson's disease, Huntington's disease, Alzheimer's disease and multiple sclerosis; eating disorders, preferably selected from the group comprising bulimia, anorexia, obesity and cachexia; anxiety conditions; cognitive dysfunctions, preferably memory deficiencies; cognitive deficiencies (attention deficit syndrome, ADS); epilepsy; diarrhoea and pruritus; or for the prophylaxis and/or treatment of alcohol and/or drug and/or medication misuse and alcohol and/or drug and/or nicotine and/or medication dependency, preferably for the prophylaxis and/or alleviation of withdrawal symptoms in the case of alcohol and/or drug and/or nicotine and/or medication dependency; for the prophylaxis and/or alleviation in the development of tolerance to medications, in particular opioid-based medications; for the regulation of nutrient intake; for modulating motor activity; for regulating the cardiovascular system; for local anaesthesia; for increasing alertness; for heightening libido; for diuresis and/or for antinatriuresis.

20. Use of at least one compound according to one or more of claims 1 to 11 for producing a medication for the prophylaxis and/or treatment of pain, preferably pain selected from the group comprising acute pain, chronic pain, neuropathic pain and visceral pain.

21. Use of at least one compound according to one or more of claims 1 to 11 for producing a medication for the prophylaxis and/or treatment of one or more disorders selected from the group comprising migraine; depression; urinary incontinence; stress incontinence; overactive bladder (OAB); coughing; osteoporosis; neurodegenerative diseases, preferably selected from the group comprising Parkinson's disease, Huntington's disease, Alzheimer's disease and multiple sclerosis; eating disorders, preferably selected from the group comprising bulimia, anorexia, obesity and cachexia; anxiety conditions; cognitive dysfunctions, preferably memory deficiencies; cognitive deficiencies (attention deficit syndrome, ADS); epilepsy; diarrhoea and pruritus; for the prophylaxis and/or treatment of alcohol and/or drug and/or medication misuse and alcohol and/or drug and/or nicotine and/or medication dependency, preferably for the prophylaxis and/or alleviation of withdrawal symptoms in the case of alcohol and/or drug and/or nicotine and/or medication dependency; for the prophylaxis and/or alleviation in the development of tolerance to medications, in particular opioid-based medications; for the regulation of nutrient intake; for modulating motor activity; for regulating the cardiovascular system; for local anaesthesia; for increasing alertness; for heightening libido; for diuresis and/or for antinatriuresis.

## Revendications

1. Composés substitués par pentafluorosulfanyle, de formule générale I, 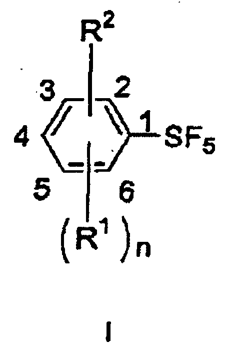 dans laquelle
n est 0, 1, 2, 3 ou 4 ;
R¹ représente un ou plusieurs radicaux choisis, indépendamment les uns des autres, dans l'ensemble constitué par H, F, Cl, Br, I, -CN, -NC, -NO₂, -SO₃H, -S(=O₂) NH₂, -NH₂, -OH, -SH, méthyle, éthyle, n-propyle, isopropyle, n-butyle, sec-butyle, isobutyle, tert-butyle, n-pentyle, sec-pentyle, n-hexyle, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -N(CH₃)₂, -N (C₂H₅)₂, -NH-CH₃, -NH-C₂H₅, -NH-C (=O) -CH₃, -NH-C(=O)-C₂H₅, -C(=O)-NH₂, -C(=O)-CH₃, -C(=O)-C₂H₅, -C(=O)-OH, -C(=O)-OCH₃ et -C(=O)-OC₂H₅ ;
R² représente un groupe -A-B-D, -E-F ou -N(H)-G, qui peut chaque fois être lié en la position 2, 3, 4, 5 ou 6 du radical phényle de la formule générale I ;
ou un groupe -Q-B-D; Q représentant un radical choisi dans l'ensemble constitué par les groupes oxazolyle, thiazolyle, isoxazolyle, isothiazolyle et imidazolyle, qui est condensé avec le radical phényle de la formule générale I et forme donc un radical éventuellement substitué choisi dans l'ensemble constitué par les groupes benzoxazolyle, benzothiazolyle, benzisothiazolyle et benzimidazolyle ;
A représente un radical choisi dans l'ensemble constitué par -N(R³)-C(=O)-N(R⁴)-, -N(R³)-C(=S)-N(R⁴)-, -N(R⁵)-C(=O)-, -N(R⁵)-C(=S)-, 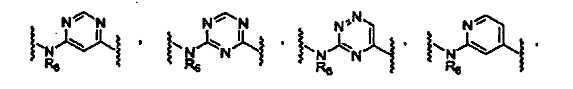 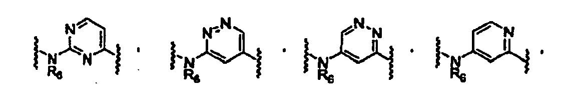 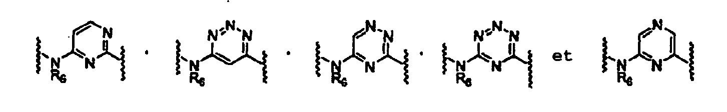
B représente un radical choisi dans l'ensemble constitué par 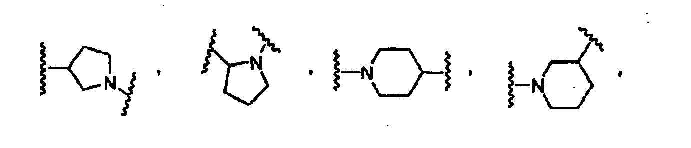 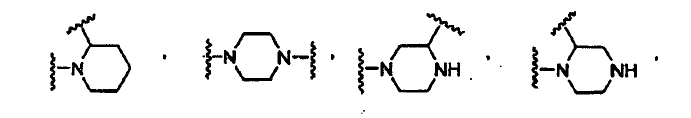 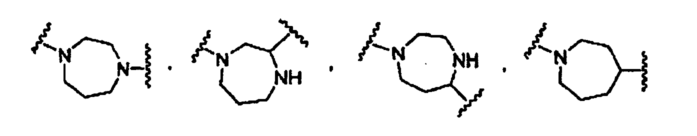 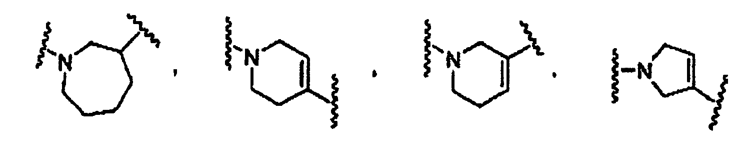 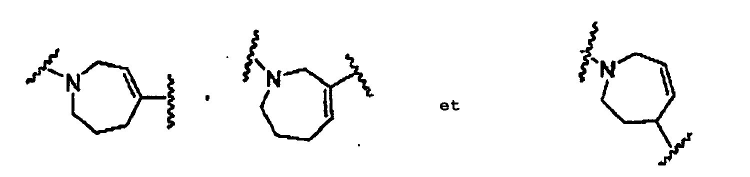 qui peut éventuellement porter 1, 2, 3 ou 4 substituants choisis, indépendamment les uns des autres, dans l'ensemble constitué par les groupes méthyle, éthyle, n-propyle, isopropyle, n-butyle, sec-butyle, isobutyle, tert-butyle, n-pentyle, sec-pentyle et n-hexyle ;
ou représente un radical phénylène qui peut porter 1, 2, 3 ou 4 substituants choisis, indépendamment les uns des autres, dans l'ensemble constitué par F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, méthyle, éthyle, n-propyle, isopropyle, n-butyle, sec-butyle, isobutyle et tert-butyle ;
D représente un radical choisi dans l'ensemble constitué par les groupes phényle, [1,3,5]-triazinyle, pyridinyle, pyridazinyle, pyrimidinyle, quinoléinyle, isoquinoléinyle et pyrazinyle, qui peut éventuellement porter 1, 2, 3, 4 ou 5 substituants choisis, indépendamment les uns des autres, dans l'ensemble constitué par F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, méthyle, éthyle, n-propyle, isopropyle, n-butyle, sec-butyle, isobutyle, tert-butyle, n-pentyle, sec-pentyle, n-hexyle, -NH-S(=O)₂-CH₃ et -NH-S(=O)₂-C₂H₅ ;
E représente un radical choisi dans l'ensemble constitué par -(CH₂)-(CH₂)-N(R⁷)-C(=O)-N (R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R¹⁸)-C(=O)-CH(CH₃)-, -(CH₂)-N(R¹⁸) -C(=S)-CH(CH₃)-, -C(=O)-N(R¹⁹)-(CH₂)-, -C(=S)-N(R¹⁹)-(CH₂)-, -N(R²⁰)-(CH₂)-C(=O)-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)-, -(CH₂)-N(R²⁴)-C(=O)-N(R²⁵)-CH(CH₃)- et -(CH₂)-N(R²⁴)-C(=S)-N(R²⁵)-CH(CH₃)-;
F représente un radical choisi dans l'ensemble constitué par les groupes quinoléinyle, isoquinoléinyle, quinoxalinyle, quinazolinyle, (1,4)-benzodioxanyle, (1,3)-benzodioxolyle, (1,2,3,4)-tétrahydronaphtyle, (1,2,3,4)-tétrahydroquinoléinyle, (1,2,3,4)-tétrahydro-isoquinoléinyle, benzoxazolyle, benzothiazolyle, benzisoxazolyle, benzisothiazolyle, benzimidazolyle, 2-oxo-2,3-dihydro-1H-benzimidazolyle, 2-benzoxazolinonyle et 2-benzothiazolinonyle, qui peut éventuellement porter 1, 2, 3, 4 ou 5 substituants choisis, indépendamment les uns des autres, dans l'ensemble constitué par F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, méthyle, éthyle, n-propyle, isopropyle, n-butyle, sec-butyle, isobutyle, tert-butyle, n-pentyle, sec-pentyle, n-hexyle, -NH-S(=O)₂-CH₃ et -NH-S(=O)₂-C₂H₅ ;
ou représente un radical naphtyle qui porte 1, 2, 3, 4 ou 5 substituants choisis, indépendamment les uns des autres, dans l'ensemble constitué par F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, méthyle, éthyle, n-propyle, isopropyle, n-butyle, sec-butyle, isobutyle, tert-butyle, n-pentyle, sec-pentyle, n-hexyle, -NH-S(=O)₂-CH₃ et -NH-S(=O)₂-C₂H₅ ;
ou représente un radical phényle qui porte 2, 3, 4 ou 5 substituants choisis, indépendamment les uns des autres, dans l'ensemble constitué par F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, méthyle, éthyle, n-propyle, isopropyle, n-butyle, sec-butyle, isobutyle, tert-butyle, n-pentyle, sec-pentyle, n-hexyle, -NH-S(=O)₂-CH₃ et -NH-S(=O) ₂-C₂H₅ ;
G représente un radical choisi dans l'ensemble constitué par 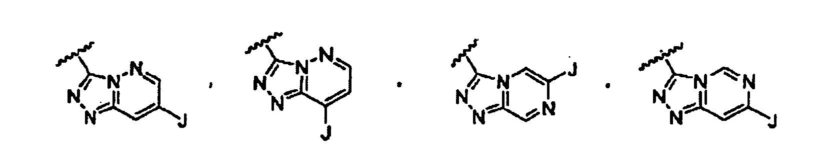 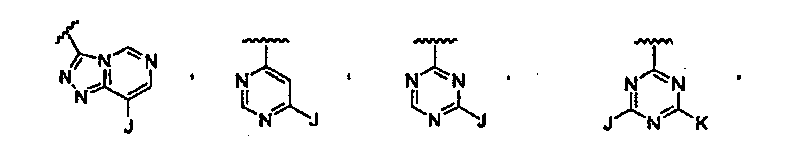 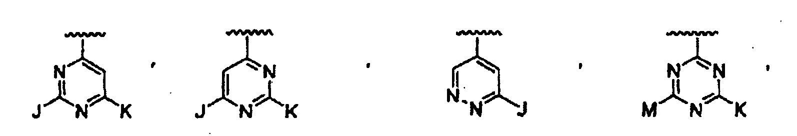 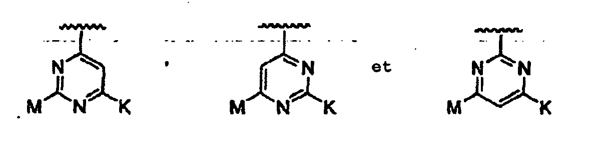
J représente un radical choisi dans l'ensemble constitué par un groupe phényle, [1,3,5]-triazinyle, pyridinyle, pyrimidinyle, pyridazinyle, quinoléinyle, isoquinoléinyle et pyrazinyle, qui peut éventuellement porter 1, 2, 3, 4 ou 5 substituants choisis, indépendamment les uns des autres, dans l'ensemble constitué par F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, méthyle, éthyle, n-propyle, isopropyle, n-butyle, sec-butyle, isobutyle, tert-butyle, n-pentyle, sec-pentyle, n-hexyle, -NH-S(=O)₂-CH₃ et -NH-S(=O)₂-C₂H₅ ;
K représente un radical choisi dans l'ensemble constitué par les groupes pipéridinyle, pipérazinyle, pyrrolidinyle, morpholinyle, thiomorpholinyle, (1,2,3,6)-tétrahydropyridinyle, cyclohexyle, cyclopentyle, cycloheptyle, azépanyle et diazépanyle, qui peut porter 1, 2, 3 ou 4 substituants choisis, indépendamment les uns des autres, dans l'ensemble constitué par les groupes méthyle, éthyle, n-propyle, isopropyle, n-butyle, sec-butyle, isobutyle, tert-butyle, n-pentyle, sec-pentyle, n-hexyle, -NH-CH₃, -NH-C₂H₅, -N(CH₃)₂ et -N (C₂H₅) ;
ou représente un groupe -NR²⁶R²⁷ ;
M représente un radical phényle qui peut porter 1, 2, 3, 4 ou 5 substituants choisis, indépendamment les uns des autres, dans l'ensemble constitué par les groupes -SF₅, F, Cl, Br, -CF₃, -OH, -O-CH₃, -O-C₂H₅, -O-CF₃, -O-CHF₂, -O-CF₂H, -S-CF₃, -S-CHF₂, -S-CF₂H, -NO₂, méthyle, éthyle, n-propyle, isopropyle, n-butyle, sec-butyle, isobutyle et tert-butyle et/ou lié par l'intermédiaire d'un groupe -O-CH₂-, -S-CH₂-, -CH₂-CH₂-, -CH=CH- ou -CH2- ;
R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²², R²¹, R²², R²³, R²⁴ et R²⁵ représentent chacun, indépendamment les uns des autres,
un atome d'hydrogène
ou un radical choisi dans l'ensemble constitué par l'atome d'hydrogène et les groupes méthyle, éthyle, n-propyle, isopropyle, n-butyle, sec-butyle, isobutyle, tert-butyle, n-pentyle, sec-pentyle et n-hexyle ;
et
R²⁶ et R²⁷ représentent chacun, indépendamment l'un de l'autre,
un radical choisi dans l'ensemble constitué par l'atome d'hydrogène et les groupes méthyle, éthyle, n-propyle, isopropyle, n-butyle, sec-butyle, isobutyle, tert-butyle, n-pentyle, sec-pentyle et n-hexyle ;
chacun éventuellement sous forme de ses stéréoisomères, en particulier énantiomères ou diastéréoisomères, purs, de leurs racémates ou sous forme d'un mélange de stéréoisomères, en particulier des énantiomères et/ou diastéréoisomères, en un rapport de mélange quelconque, ou chacun sous forme des sels correspondants, ou chacun sous forme de produits de solvatation correspondants.

2. Composés selon la revendication 1, **caractérisés en ce que** n est égal à 0.

3. Composés selon la revendication 1 ou 2, **caractérisés en ce que** R² représente un groupe -A-B-D, - E-F ou -N(H)-G, qui est chaque fois lié en la position 4 du radical phényle de la formule générale I,
ou représente un groupe -Q-B-D ; Q représentant un radical choisi dans l'ensemble constitué par les groupes oxazolyle, thiazolyle, isoxazolyle, isothiazolyle et imidazolyle, qui est condensé en la position 4 ou 5 avec le radical phényle de la formule générale I et forme par conséquent un radical éventuellement substitué, choisi dans l'ensemble constitué par les groupes benzoxazolyle, benzothiazolyle, benzisothiazolyle et benzimidazolyle.

4. Composés selon une ou plusieurs des revendications 1 à 3, **caractérisés en ce que**
B représente un radical choisi dans l'ensemble constitué par

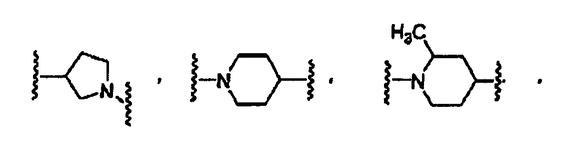

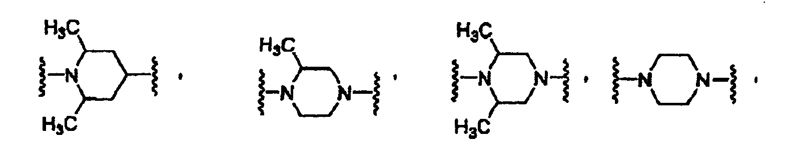

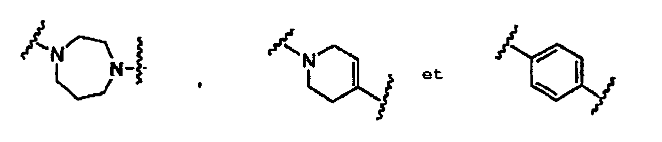

5. Composés selon une ou plusieurs des revendications 1 à 4, **caractérisés en ce que**
D représente un radical choisi dans l'ensemble constitué par

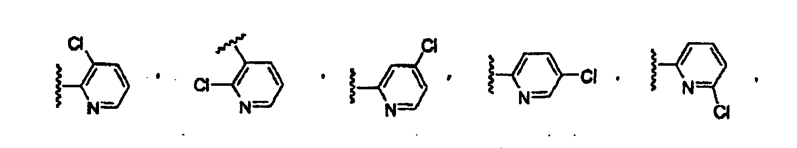

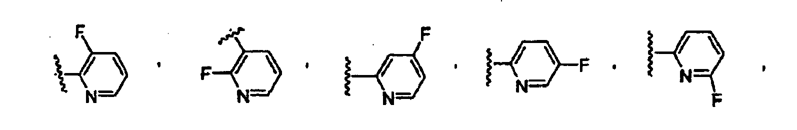

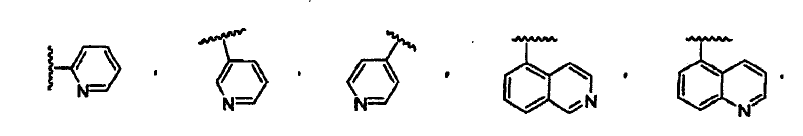

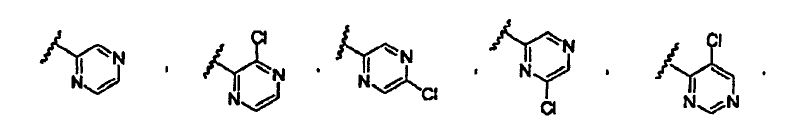

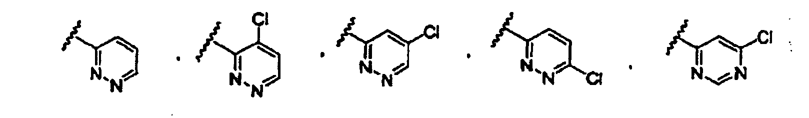

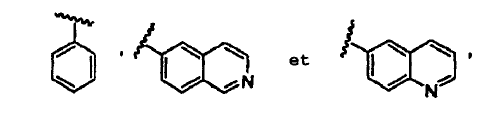

6. Composés selon une ou plusieurs des revendications 1 à 5, **caractérisés en ce que**
F représente un radical choisi dans l'ensemble constitué par

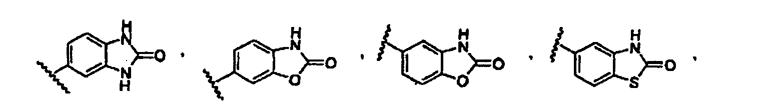

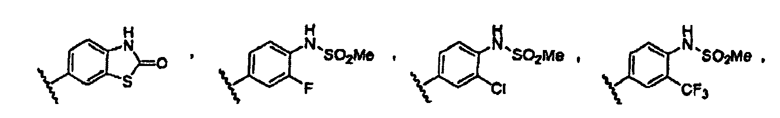

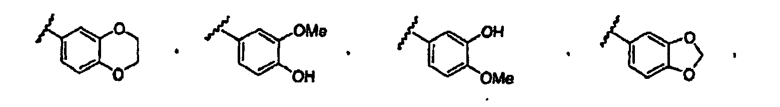

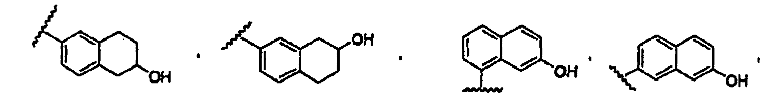

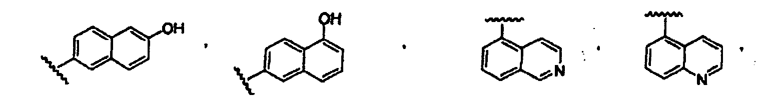

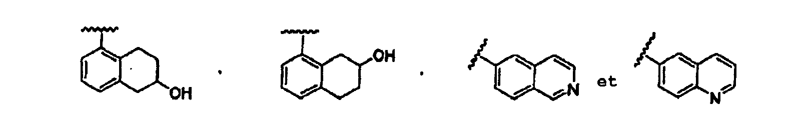

7. Composés selon une ou plusieurs des revendications 1 à 6, **caractérisés en ce que**
J représente un radical choisi dans l'ensemble constitué par

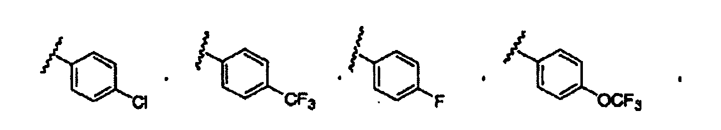

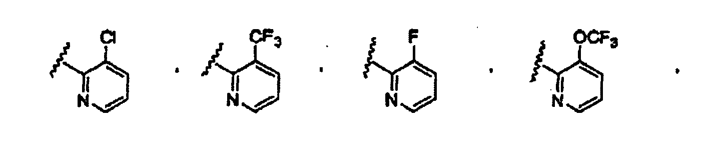

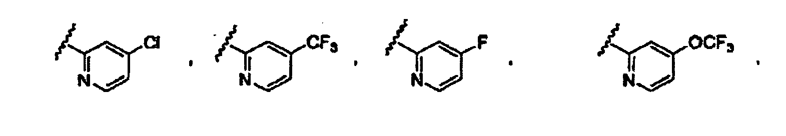

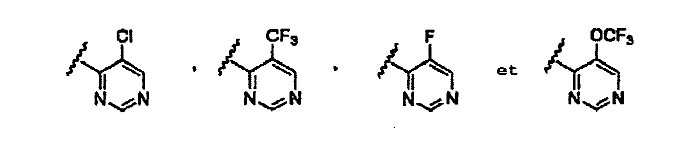

8. Composés selon une ou plusieurs des revendications 1 à 7, **caractérisés en ce que**
K représente un radical choisi dans l'ensemble constitué par

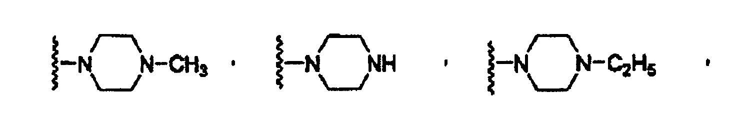

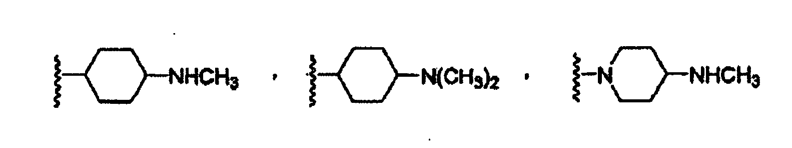

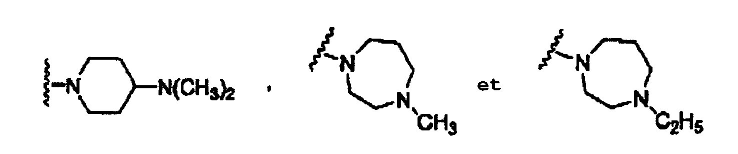

9. Composés selon une ou plusieurs des revendications 1 à 8, **caractérisés en ce que**
M représente un radical choisi dans l'ensemble constitué par

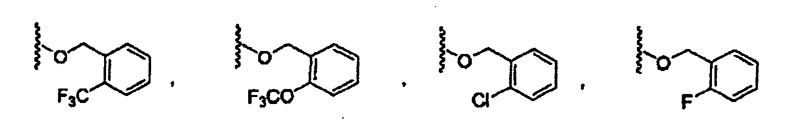

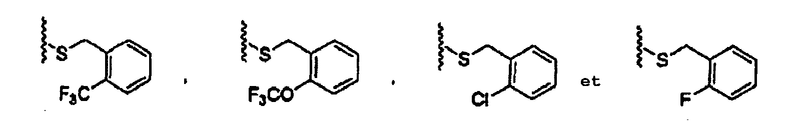

10. Composés selon une ou plusieurs des revendications 1 à 9, **caractérisés en ce que**
n est égal à 0,
R² représente un groupe -A-B-D, -E-F ou -N(H)-G, qui est dans chaque cas lié à la position 4 du radical phényle de la formule générale I,
ou représente un groupe -Q-B-D ; Q représentant un radical choisi dans l'ensemble constitué par les groupes oxazolyle, thiazolyle, isoxazolyle, isothiazolyle et imidazolyle, qui est condensé en la position 4 et la position 5 avec le radical phényle de la formule générale I et forme donc un radical éventuellement substitué, choisi dans l'ensemble constitué par les groupes benzoxazolyle, benzothiazolyle, benzisothiazolyle et benzimidazolyle ;
A représente un radical choisi dans l'ensemble constitué par -N(R³)-C(=O)-N(R⁴)-, -N(R³)-C(=S)-N(R⁴)-, -N(R⁵)-C(=O)-, -N(R⁵)-C(=S)-, 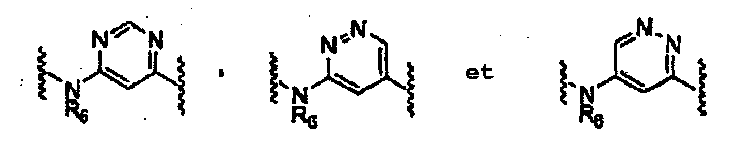
B représente un radical choisi dans l'ensemble constitué par 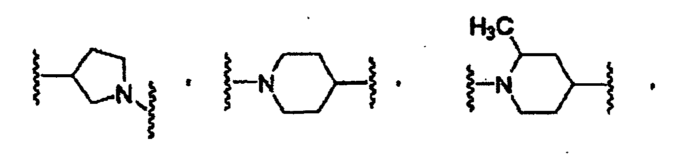 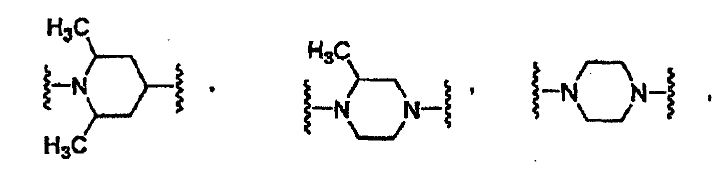 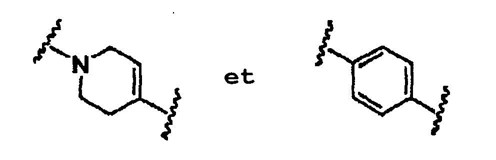
D représente un radical choisi dans l'ensemble constitué par 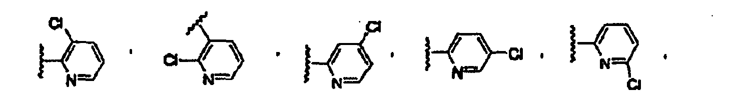 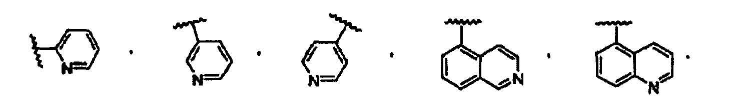 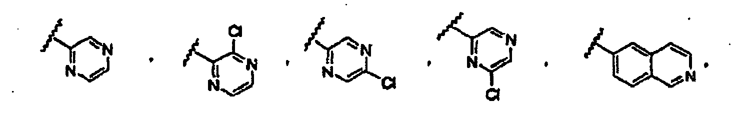 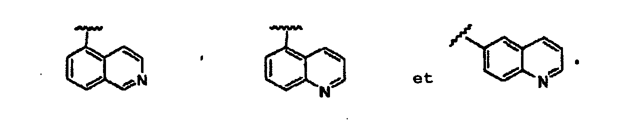
E représente un radical choisi dans l'ensemble constitué par -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -CH=CH-C(=O)-N(R¹¹)-, -CH=CH-C(=S)-N(R¹¹)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(=S)-N(R¹⁷)-, -(CH₂)-N(R¹⁸)-C(=O)-CH(CH₃)-, -(CH₂)-N(R¹⁸) -C(=S)-CH(CH₃)-, -C(=O)-N(R¹⁹)-(CH₂)-, -C(=S)-N(R¹⁹)-(CH₂₎-, -N(R²⁰)-(CH₂)-C(=O)-N(R²¹)-, -N(R²⁰)-(CH₂)-C(=S)-N(R²¹)-, -(CH₂)-(CH₂)-N(R²²)-C(=O)-, -(CH₂)-(CH₂)-N(R²²)-C(=S)-, -CH(CH₃)-N(R²³)-C(=O)-, -CH(CH₃)-N(R²³)-C(=S)-, -(CH₂)-N(R₂₄)-C(=O)-N(R²⁵)-CH(CH₃)- et -(CH₂)-N(R₂₄)-C(=S)-N(R²⁵)-CH(CH₃)- ;
F représente un radical choisi dans l'ensemble constitué par 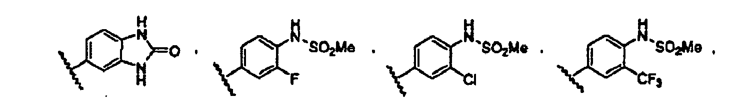 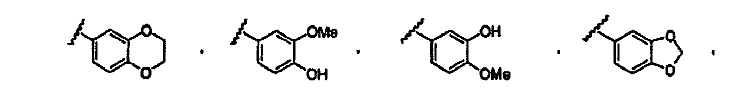 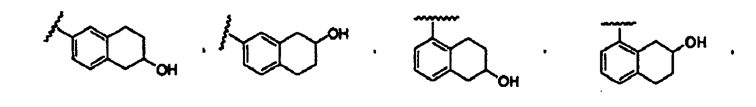 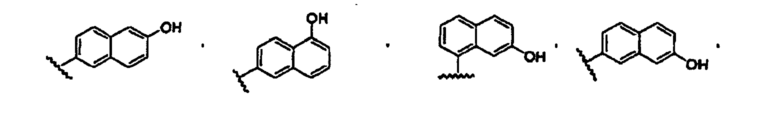 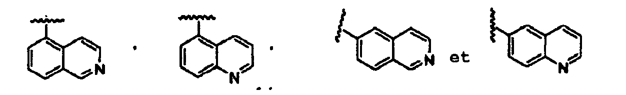
G représente un radical choisi dans l'ensemble constitué par 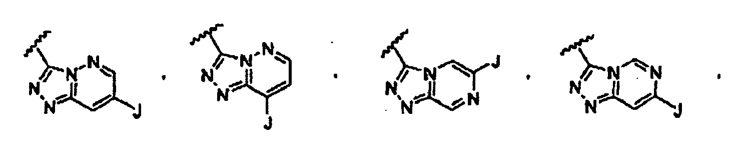 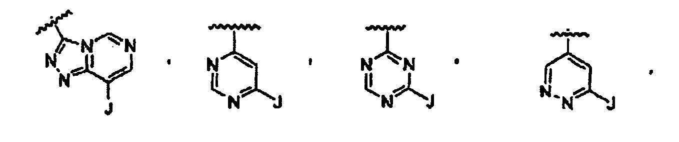 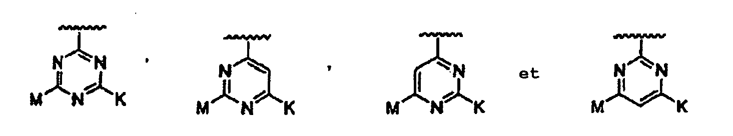
J représente un radical choisi dans l'ensemble constitué par 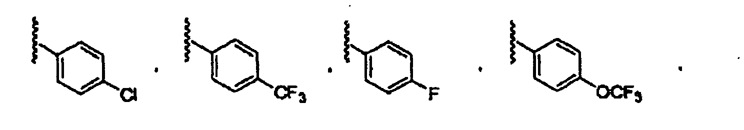 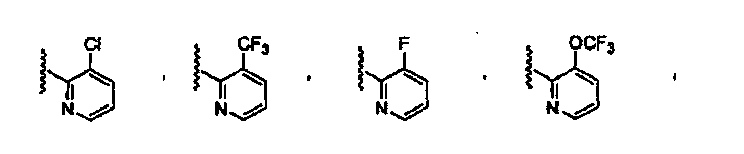 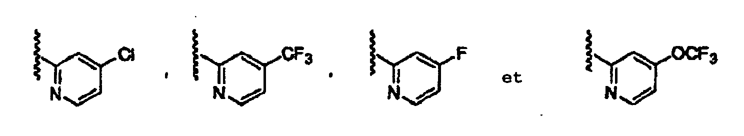
K représente un radical choisi dans l'ensemble constitué par 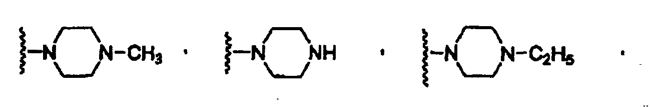 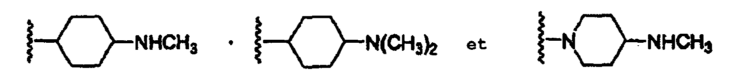
M représente un radical choisi dans l'ensemble constitué par 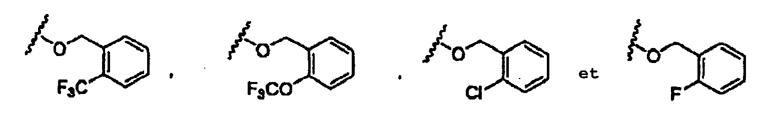 et
R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴ et R²⁵ représentent chacun un atome d'hydrogène ;
chacun éventuellement sous forme de ses stéréoisomères, en particulier énantiomères ou diastéréoisomères, purs, de leurs racémates ou sous forme d'un mélange de stéréoisomères, en particulier des énantiomères et/ou diastéréoisomères, en un rapport de mélange quelconque, ou chacun sous forme des sels correspondants, ou chacun sous forme de produits de solvatation correspondants.

11. Composés selon une ou plusieurs des revendications 1 à 10, choisis dans l'ensemble constitué par les composés suivants :
[1] N-{4-[3-(4-pentafluorosulfanyl-benzyl)-thio-uréidométhyl]-2-fluoro-phényl}méthanesulfonamide
[2] (4-pentafluorosulfanylphényl)-amide d'acide 4-(3-chloro-pyridin-2-yl)-pipérazine-1-carboxylique
[3] (4-pentafluorosulfanylphényl)-amide d'acide 4-(3-chloro-pyridin-2-yl)-pipérazine-1-thiocarboxylique
[4] 3-(4-pentafluorosulfanyl-phényl)-N-(2,3-dihydro-benzo[1,4]dioxin-6-yl)-acrylamide
[5] N-(4-pentafluorosulfanyl-benzyl)-2-(3-fluoro-4-méthanesulfonylamino-phényl)-propionamide
[6] (2R)-N-(4-pentafluorosulfanyl-benzyl)-2-(3-fluoro-4-méthanesulfonylamino-phényl)-propionamide
[7] (2S)-N-(4-pentafluorosulfanyl-benzyl)-2-(3-fluoro-4-méthanesulfonylamino-phényl)-propionamide
[8] N-(4-pentafluorosulfanyl-phényl)-4-pyridin-2-yl-benzamide
[9] 1-(4-pentafluorosulfanyl-benzyl)-3-(7-hydroxy-naphtalén-1-yl)-urée
[10] (4-pentafluorosulfanyl-phényl)-amide d'acide 4-(3-chloro-pyrazin-2-yl)-3,6-dihydro-2H-pyridine-1-carboxylique
[11] (4-pentafluorosulfanyl-phényl)-[4-(4-méthyl-pipérazin-1-yl)-6-(2-trifluorométhyl-benzyloxy)-[1,3,5]triazin-2-yl]-amine
[12] (4-pentafluorosulfanyl-phényl)-[6-(4-chloro-phényl)-pyrimidin-4-yl]-amine
[13] 4-pentafluorosulfanyl-N-(2-oxo-2,3-dihydro-1H-benzo-imidazol-5-ylméthyl)-benzamide
[14] (4-pentafluorosulfanyl-phényl)-{6-[4-(3-chloro-pyridin-2-yl)-2-(R)-méthyl-pipérazin-1-yl]-pyrimidin-4-yl}-amine
[15] (4-pentafluorosulfanyl-phényl)-amide d'acide 3'-chloro-3,6-dihydro-2H-[1,2']bipyridinyl-4-carboxylique
[16] 2-(4-pentafluorosulfanyl-phénylamino)-N-(7-hydroxy-naphtalén-1-yl)-acétamide
[17] (4-pentafluorosulfanyl-phényl)-[7-(3-trifluoro-méthyl-pyridin-2-yl)-[1,2,4]triazolo[4,3-b]pyridazin-3-yl]-amine
[18] 1-(4-pentafluorosulfanyl-benzyl)-3-isoquinoléin-5-yl-urée
[19] 1-[2-(4-pentafluorosulfanyl-phényl)-éthyl]-3-isoquinoléin-5-yl-urée
[20] 1-(4-pentafluorosulfanyl-phényl)-3-(7-hydroxy-5,6,7,8-tétrahydronaphtalén-1-yl)-urée
[21] 1-[2-(4-pentafluorosulfanyl-phényl)-éthyl]-3-quinoléin-5-yl-urée
[22] 1-(4-pentafluorosulfanyl-phényl)-3-(1-isoquinoléin-5-yl-pyrrolidin-3-yl)-urée
[23] 5-pentafluorosulfanyl-2-[4-(3-chloro-pyridin-2-yl)-pipérazin-1-yl]-benzo-oxazole
et
[24] N-{4-[3-(4-pentafluorosulfanyl-benzyl)-uréidométhyl]-2-fluoro-phényl}-méthanesulfonamide.

12. Procédé pour la préparation de composés de formule générale I selon une ou plusieurs des revendications 1 à 11, **caractérisé en ce qu'**on fait réagir au moins un composé de formule générale II, 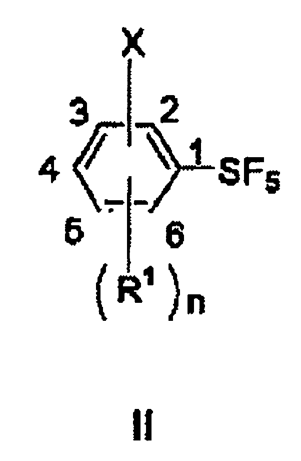 dans laquelle R¹ et n ont les significations selon une ou plusieurs des revendications 1 à 11 et X représente -N=C=O, -N=C=S, -(CH₂)-N=C=O, -(CH₂)-N=C=S, -(CH₂)-N=C=S, - (CH₂)-(CH₂)-N=C=O, - (CH₂) - (CH₂) -N=C=S, -CH(CH₃)-N=C=O ou -CH(CH₃)-N=C=S, dans un milieu réactionnel, en présence d'au moins une base, de préférence en présence d'au moins une base organique choisie dans l'ensemble constitué par la triéthylamine, la diisopropyléthylamine, la N-méthylmorpholine, la pyridine et la diméthylaminopyridine, avec au moins un composé de formule générale H-B-D ; le groupe H-B comportant au moins un groupe -N (H) -, H₂N-B-D, H₂N-F, H₂N-(CH₂)-F, H₂N-(CH₂)-(CH₂)-F ou H₂N-CH(CH₃)-F ; B, D et F ayant les significations selon une ou plusieurs des revendications 1 à 11 ;
ou au moins un composé de formule générale II, dans laquelle R¹ et n ont les significations selon une ou plusieurs des revendications 1 à 11 et X représente -NH₂, -(CH₂)-NH₂, -CH(CH₃)-NH₂ ou -(CH₂)-(CH₂)-NH₂, dans un milieu réactionnel, en présence d'au moins une base, de préférence en présence d'au moins une base organique choisie dans l'ensemble constitué par la triéthylamine, la diisopropyléthylamine, la N-méthylmorpholine, la pyridine et la diméthylaminopyridine, avec au moins un composé de formule générale F-N=C=O, F-N=C=S, F-(CH₂)-N=C=O, F-(CH₂)-N=C=S, F-(CH)₂-(CH₂)-N=C=O, F-(CH₂)-(CH₂)-N=C=S, F-CH(CH₃)-N=C=O ou F-CH(CH₃)-N=C=S ; F ayant la signification donnée précédemment ;
pour aboutir à au moins un composé de formule générale I dans lequel R¹ et n ont les significations données précédemment et R² représente -A-B-D ou -E-F, A représentant -N(R³)-C(=O)-N(R⁴)- ou -N(R³)-C(=S)-N(R⁴)- ; E représentant un radical choisi dans l'ensemble constitué par -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²) -C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH²)-, -N(R¹⁶)-C(=O)-N(R¹⁷)-, -N(R¹⁶)-C(=S)-N(R¹⁷)-, -(CH²)-N(R²⁴)-C (=O)-N(R²⁵)-CH(CH₃)- et -(CH₂)-N(R²⁴)-C(=S)-N(R²⁵)-CH(CH₃)- ; R³, R⁴, R⁷, R⁸, R⁹, R¹⁰, R¹², R¹³, R¹⁴, R¹⁵, R¹⁶, R¹⁷, R²⁴ et R²⁵ représentant chacun un atome d'hydrogène ; et B, D et F ayant les significations selon une ou plusieurs des revendications 1 à 11 ; et éventuellement on le purifie et/ou on l'isole,
et éventuellement on fait réagir au moins un composé de formule générale I, dans laquelle R¹ et n ont les significations données précédemment et R² représente -A-B-D ou -E-F, A représentant -N(R³)-C(=O)-N(R⁴)- ou -N(R³)-C(=S)-N(R⁴)- ; E représentant un radical choisi dans l'ensemble constitué par -(CH₂)-(CH₂)-N(R₇)-C(=O)-N(R₈)-, -(CH₂)-(CH₂)-N(R⁷)-C(=_{S})-_{N}(_{R}⁸)-, -(CH₂)-N(R⁹)-C(=O) -N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R₁₄)-C(=S)-N(R¹⁵)-(CH₂)-, -N(R¹⁶)-C(=O)-N(R¹⁷)-, -N(R¹⁶)-C (=S)-N(R¹⁷)-, -(CH₂)-N(R²⁴)-C(=O)-N(R²⁵)-CH(CH3)- et -(CH₂)-N(R²⁴)-C(=S)-N(R²⁵)-CH(CH₃)- ; R³, R⁴, R⁷, R⁸, R⁹, R¹⁰, R¹² R¹³, R¹⁴, R¹⁵, R¹⁶, R¹⁷, R²⁴ et R²⁵ représentant chacun un atome d'hydrogène ; et B, D et F ayant les significations selon une ou plusieurs des revendications 1 à 11 ; dans un milieu réactionnel en présence d'une base, de préférence en présence d'un hydrure métallique, de façon particulièrement préférée en présence d'hydrure de sodium et/ou d'hydrure de potassium, avec au moins un composé de formule générale LG-R³, LG-R⁴, LG-R⁷, LG-R⁸, LG-R⁹, LG-R¹⁰, LG-R¹², LG-R¹³, LG-R¹⁴, LG-R¹⁵, LG-R¹⁶, LG-R¹⁷, LG-R²⁴ et LG-R²⁵ ; R³, R⁴, R⁷, R⁸, R⁹, R¹⁰, R¹², R¹³, R¹⁴, R¹⁵, R¹⁶, R¹⁷, R²⁴ et R²⁵ ayant les significations selon une ou plusieurs des revendications 1 à 11, à l'exception de celle d'un atome d'hydrogène, et LG représentant un groupe partant, de préférence un atome d'halogène, de façon particulièrement préférée un atome de chlore, pour aboutir à au moins un composé de formule générale I dans lequel R¹ et n ont les significations données précédemment et R² représente -A-B-D ou -E-F, A représentant -N(R³)-C(=O)-N(R⁴)- ou -N(R³)-C(=S)-N(R⁴)- ; E représentant un radical choisi dans l'ensemble constitué par -(CH₂)-(CH₂)-N(R⁷)-C(=O)-N(R⁸)-, -(CH₂)-(CH₂)-N(R⁷)-C(=S)-N(R⁸)-, -(CH₂)-N(R⁹)-C(=O)-N(R¹⁰)-, -(CH₂)-N(R⁹)-C(=S)-N(R¹⁰)-, -(CH₂)-(CH₂)-N(R¹²)-C(=O)-N(R¹³)-(CH₂)-(CH₂)-, -(CH₂)-(CH₂)-N(R¹²)-C(=S)-N(R¹³)-(CH₂)-(CH₂)-, -(CH₂)-N(R¹⁴)-C(=O)-N(R¹⁵)-(CH₂)-, -(CH₂)-N(R¹⁴)-C(=S)-N(R¹⁵)-(CH₂)-, -N(R¹⁶)-C(=O)-N(R¹⁷)-, -N(R¹⁶)-C(-S)-N(R¹⁷)-, -(CH₂)-N(R²⁴)-C(=O)-N(R²⁵)-CH(CH₃)- et -(CH₂)-N(R₂₄)-C(=S)-N(R²⁵)-CH(CH₃)- ; R³, R⁴, R⁷, R⁸, R⁹, R¹⁰, R¹², R¹³, R¹⁴, R¹⁵, R¹⁶, R¹⁷, R²⁴ et R²⁵ ayant chacun le signification selon une ou plusieurs des revendications 1 à 11, à l'exception de celle d'un atome d'hydrogène ; et B, D et F ayant les significations selon une ou plusieurs des revendications 1 à 11 ; et éventuellement on le purifie et/ou on l'isole,

13. Procédé pour la préparation de composés de formule générale I selon une ou plusieurs des revendications 1 à 11, **caractérisé en ce qu'**on fait réagir au moins un composé de formule générale II, 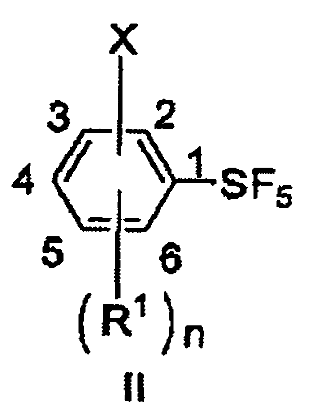 dans laquelle R¹ et n ont les significations selon une ou plusieurs des revendications 1 à 11 et X représente -NH₂, dans un milieu réactionnel, avec au moins un composé de formule LG-G, dans laquelle G a la signification selon une ou plusieurs des revendications 1 à 11 et LG représente un groupe partant, de préférence un atome d'halogène, de façon particulièrement préférée un atome de fluor ou un atome de chlore, pour aboutir à au moins un composé de formule générale I dans lequel R¹ et n ont les significations données précédemment et R² représente -N(H)-G, G ayant la signification donnée précédemment, et éventuellement on le purifie et/ou on l'isole.

14. Procédé pour la préparation de composés de formule générale I selon une ou plusieurs des revendications 1 à 11, **caractérisé en ce qu'**on fait réagir au moins un composé de formule générale III, 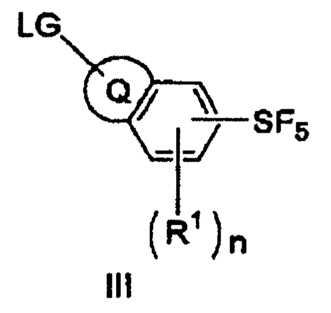 dans laquelle R¹, n et Q ont les significations selon une ou plusieurs des revendications 1 à 11 et LG représente un groupe partant, de préférence un atome d'halogène, de façon particulièrement préférée un atome de fluor ou un atome de chlore, dans un milieu réactionnel, avec au moins un composé de formule générale H-B-D ; le groupe H-B comportant au moins un groupe -N(H) et B et D ayant les significations selon une ou plusieurs des revendications 1 à 11, pour aboutir à au moins un composé de formule générale I dans lequel R¹ et n ont les significations données précédemment et R² représente -Q-B-D, Q, B et D ayant les significations données précédemment, et éventuellement on le purifie et/ou on l'isole.

15. Procédé pour la préparation de composés de formule générale I selon une ou plusieurs des revendications 1 à 11, **caractérisé en ce qu'**on fait réagir au moins un composé de formule générale II, 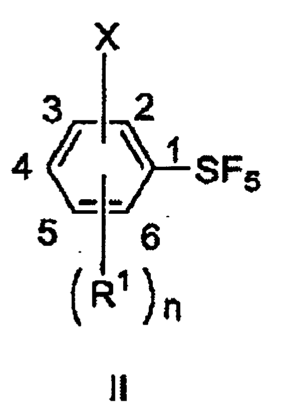 dans laquelle R¹ et n ont les significations selon une ou plusieurs des revendications 1 à 11 et X représente -C(=O)-LG, -(CH₂)-C(=O)-LG, -(CH₂)-(CH₂)-C(=O)-LG, -CH(CH₃)-C(=O)-LG, -CH=CH-C(=O)-LG et -N(R²⁰)-CH₂-C(=O)-LG, LG représentant un groupe partant, de préférence un atome d'halogène, de façon particulièrement préférée un atome de chlore, et R²⁰ ayant les significations selon une ou plusieurs des revendications 1 à 11, dans un milieu réactionnel, en présence d'au moins une base, de préférence en présence d'au moins une base organique choisie dans l'ensemble constitué par la triéthylamine, la diisopropyléthylamine, la N-méthylmorpholine, la pyridine et la diméthylaminopyridine, avec au moins un composé de formule générale H-B-D ; le groupe H-B comportant au moins un groupe -N(H)-, ou de formule H₂N-B-D, H₂N-F, H₂N-(CH₂)-F, H₂N- (CH₂)- (CH₂) -F ou H₂N-CH(CH₃)-F ; B, D et F ayant les significations selon une ou plusieurs des revendications 1 à 11 ;
ou au moins un composé de formule générale II, dans laquelle R¹ et n ont les significations selon une ou plusieurs des revendications 1 à 11 et X représente -NH₂, -(CH₂)-NH₂, -CH(CH₃)-NH₂, -(CH₂)-(CH₂)-NH₂ ou -CH=CH-NH₂, dans un milieu réactionnel, en présence d'au moins une base, de préférence en présence d'au moins une base organique choisie dans l'ensemble constitué par la triéthylamine, la diisopropyléthylamine, la N-méthylmorpholine, la pyridine et la diméthylaminopyridine, avec au moins un composé de formule générale F-C(=O)-LG, F-(CH₂)-C(=O)-LG, F-(CH₂)(CH₂)-C(=O)-LG, F-CH(CH₃)-C(=O)-LG, F-CH=CH-C(=O)-LG et F-N(R²⁰)-CH₂-C(=O)-LG, F, R²⁰ et LG ayant les significations données précédemment ;
pour aboutir à au moins un composé de formule générale I dans lequel R¹ et n ont les significations données précédemment et R² représente -A-B-D ou -E-F, A représentant -N(R⁵)-C(=O)- ; E représentant un radical choisi dans l'ensemble constitué par -CH=CH-C(=O)-N(R¹¹), -(CH₂)-N(R¹⁸)-C(=O)-CH(CH₃)-, -C(=O)-N(R¹⁹)-(CH₂)-, -N(R²⁰)-(CH₂)-C(=O)-N(R²¹)-, -(CH₂)-(CH₂)-N(R²²)-C(=O)- et -CH(CH₃)-N(R²³)-C(=O) ; R⁵, R¹¹, R¹⁸, R¹⁹, R²⁰, R²¹, R²² et R²³ représentant chacun un atome d'hydrogène ; et B, D et F ayant les significations selon une ou plusieurs des revendications 1 à 11 ; et éventuellement on le purifie et/ou on l'isole ;
et éventuellement on fait réagir un composé de formule générale I dans lequel R¹ et n ont les significations données précédemment et R² représente -A-B-D ou -E-F, A représentant -N(R⁵)-C(=O)- ; E représentant un radical choisi dans l'ensemble constitué par -CH=CH-C(=O)-N(R¹¹), -(CH₂)-N(R¹⁸)-C(=O)-CH(CH₃)-, -C(=O)-N(R¹⁹)-(CH₂)-, -N(R²⁰)-(CH₂)-C(=O)-N(R²¹)-, -(CH₂)-(CH₂)-N(R²²)-C(=O)- et -CH(CH₃)-N(R²³)-C(=O)- ; R⁵, R¹¹, R¹⁸, R¹⁹, R²⁰, R²¹, R²² et R²³ représentant chacun un atome d'hydrogène ; et B, D et F ayant les significations selon une ou plusieurs des revendications 1 à 11 ; dans un milieu réactionnel en présence d'une base, de préférence en présence d'un hydrure métallique, de façon particulièrement préférée en présence d'hydrure de sodium et/ou d'hydrure de potassium, avec au moins un composé de formule générale LG-R⁵, LG-R¹¹, LG-R¹⁸, LG-R¹⁹, LG-R²⁰, LG-R²¹, LG-R²² et LG-R²³, R⁵, R¹¹, R¹⁸, R¹⁹, R²⁰, R²¹, R²² et R²³ ayant les significations selon une ou plusieurs des revendications 1 à 11, à l'exception de celle d'un atome d'hydrogène, pour aboutir à au moins un composé de formule générale I dans lequel R¹ et n ont les significations données précédemment et R² représente -A-B-D ou -E-F, A représentant -N(R⁵)-C(=O)- ; E représentant un radical choisi dans l'ensemble constitué par -CH=CH-C(=O)-N(R¹¹), -(CH₂)-N(R¹⁸)-C(=O)-CH(CH₃)-, -C(=O)-N(R¹⁹)-(CH₂)-, -N(R²⁰)-(CH₂)-C(=O) -N (R²¹)-, -(CH₂)-(CH₂)-N(R²²) -C(=O)- et -CH(CH₃)-N(R²³)-C(=O)- ; R⁵, R¹¹, R¹⁸, R¹⁹, R²⁰, R²¹, R²² et R²³ ayant les significations selon une ou plusieurs des revendications 1 à 11 ; et B, D et F ayant les significations selon une ou plusieurs des revendications 1 à 11 ; et éventuellement on le purifie et/ou on l'isole ;
et éventuellement on fait réagir un composé de formule générale I dans lequel R¹ et n ont les significations données précédemment et R² représente -A-B-D ou -E-F, A représentant -N(R⁵)-C(=O)- ; E représentant un radical choisi dans l'ensemble constitué par -CH=CH-C(=O)-N(R¹¹), -(CH₂)-N(R¹⁸) -C(=O)-CH(CH₃)-, -C(=O)-N(R¹⁹)-(CH₂)-, -N(R²⁰)-(CH₂)-C(=O)-N(R²¹)-, -(CH₂)-(CH₂)-N(R²²) -C(=O)- et -CH(CH₃)-N(R²³)-C(=O)- ; R⁵, R¹¹, R¹⁸, R¹⁹, R²⁰, R²¹, R²² et R²³ ayant les significations selon une ou plusieurs des revendications 1 à 11 ; et B, D et F ayant les significations selon une ou plusieurs des revendications 1 à 11 ; dans un milieu réactionnel, de préférence dans un milieu réactionnel choisi dans l'ensemble constitué par le diméthylformamide, le xylène, le mésitylène, le toluène, l'acétonitrile, le dichlorométhane, l'oxyde d'éthyle, le tétrahydrofuranne et le diméthylsulfoxyde, avec au moins un composé de formule générale IV, 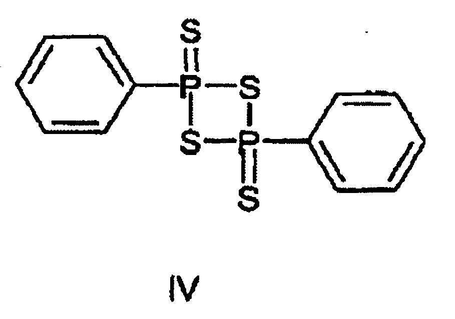 dans laquelle les radicaux phényle portent chacun 1 ou 2 substituants, choisis dans l'ensemble constitué par les groupes méthoxy, phénoxy, Cl, méthyle et Br, de préférence portent chacun un radical phénoxy ou méthoxy, de façon particulièrement préférée portent chacun un radical méthoxy en position para, ou avec du pentasulfure de phosphore,
pour aboutir à un composé de formule générale I, dans lequel R¹ et n ont les significations données précédemment et R² représente -A-B-D ou -E-F, A représentant -N(R⁵)-C(=S)- ; E représentant un radical choisi dans l'ensemble constitué par -CH=CH-C(=S)-N(R¹¹), -(CH₂)-N(R¹⁸) -C(=S)-CH(CH₃)-, -C(=S)-N(R¹⁹)-(CH₂)-, -N(R²⁰)-(CH₂)-C(=S)-N(R²¹)-, -(CH₂)-(CH₂)-N(R²²)-C(=S)- et -CH(CH₃)-N(R²³)-C(=S)- ; R⁵, R¹¹, R¹⁸, R¹⁹, R²⁰, R²¹, R²² et R²³ ayant les significations selon une ou plusieurs des revendications 1 à 11 ; et B, D et F ayant les significations selon une ou plusieurs des revendications 1 à 11 ; et éventuellement on le purifie et/ou on l'isole.

16. Procédé pour la préparation de composés de formule générale I selon une ou plusieurs des revendications 1 à 11, **caractérisé en ce qu'**on fait réagir au moins un composé de formule générale II, 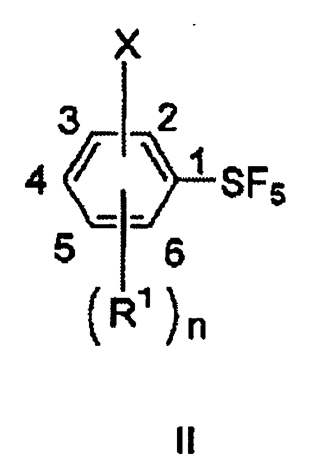 dans laquelle R¹ et n ont les significations selon une ou plusieurs des revendications 1 à 11 et X représente -NH₂ ou -NHR⁶ ; R⁶ ayant la signification selon une ou plusieurs des revendications 1 à 11 ; dans un milieu réactionnel, avec au moins un composé de formule générale A-B-D, dans laquelle B et D ont les significations selon une ou plusieurs des revendications 1 à 11 et A représente un radical choisi dans l'ensemble constitué par 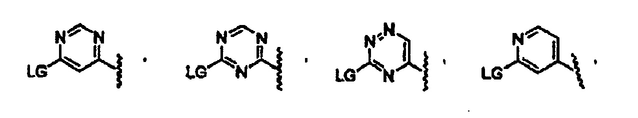 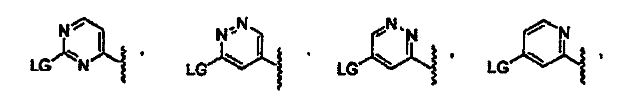 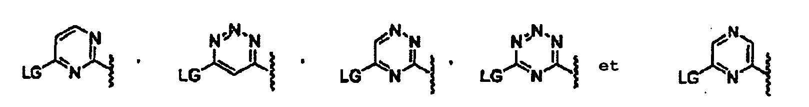 LG représentant un groupe partant, de préférence un atome d'halogène, de façon particulièrement préférée un atome de fluor ou de chlore, dans un milieu réactionnel, de préférence dans un milieu réactionnel choisi dans l'ensemble constitué par le diméthylformamide, le xylène, le mésitylène, le toluène, l'acétonitrile, le dichlorométhane, l'oxyde d'éthyle, le tétrahydrofuranne et le diméthylsulfoxyde, pour aboutir à un composé de formule I dans lequel R¹, R⁶ et n ont les significations données précédemment et R² représente -A-B-D, B et D ayant les significations données précédemment et A ayant la signification selon une ou plusieurs des revendications 1 à 11, à l'exception de celles de -N(R³)-C(=O)-N(R⁴)-, -N(R³)-C(=S)-N(R⁴)-, -N(R⁵)-C(=O)- et -N(R⁵)-C(=S)-, et éventuellement on le purifie et/ou on l'isole.

17. Médicament contenant au moins un composé selon une ou plusieurs des revendications 1 à 11 et éventuellement un ou plusieurs adjuvants physiologiquement acceptables.

18. Médicament selon la revendication 17, destiné à la prophylaxie et/ou au traitement de la douleur, de préférence la douleur choisie dans l'ensemble consistant en la douleur aiguë, la douleur chronique, la douleur névropathique et la douleur viscérale.

19. Médicament selon la revendication 17, destiné à la prophylaxie et/ou au traitement d'une ou plusieurs maladies choisies dans le groupe constitué par la migraine ; les dépressions ; l'incontinence urinaire ; l'incontinence due au stress ; la vessie hyperactive (*overactive bladder,* OAB) ; la toux ; l'ostéoporose ; des maladies neurodégénératives, de préférence choisies dans l'ensemble constitué par la maladie de Parkinson, la chorée de Huntington, la maladie d'Alzheimer et la sclérose en plaques ; les troubles de la prise d'aliments, de préférence choisis dans l'ensemble constitué par la boulimie, l'anorexie, l'obésité et la cachexie ; les états anxieux ; les dysfonctionnements cognitifs, de préférence les troubles de la mémoire ; les états de déficit cognitif (*attention déficit syndrom, ADS*) ; l'épilepsie ; la diarrhée et le prurit ; ou à la prophylaxie et/ou au traitement du mésusage d'alcool et/ou de drogues et/ou de médicaments et de la dépendance à l'alcool et/ou aux drogues et/ou à la nicotine et/ou de la pharmacodépendance, de préférence à la prophylaxie et/ou à la réduction de manifestations de sevrage dans la dépendance à l'alcool et/ou aux drogues et/ou à la nicotine et/ou dans la pharmacodépendance ; à la prophylaxie et/ou à la réduction d'un développement d'une tolérance à des médicaments, en particulier à des médicaments à base d'opioïdes ; à la régulation de la prise de nourriture ; à la modulation de l'activité motrice ; à la régulation du système cardiovasculaire ; à l'anesthésie locale ; à l'augmentation de la vigilance ; à l'augmentation de la libido ; à la diurèse et/ou à l'antinatriurèse.

20. Utilisation d'au moins un composé selon une ou plusieurs des revendications 1 à 11, pour la fabrication d'un médicament destiné à la prophylaxie et/ou au traitement de la douleur, de préférence la douleur choisie dans l'ensemble consistant en la douleur aiguë, la douleur chronique, la douleur névropathique et la douleur viscérale.

21. Utilisation d'au moins un composé selon une ou plusieurs des revendications 1 à 11, pour la fabrication d'un médicament destiné à la prophylaxie et/ou au traitement d'une ou plusieurs maladies choisies dans le groupe constitué par la migraine ; les dépressions ; l'incontinence urinaire ; l'incontinence due au stress ; la vessie hyperactive (*overactive bladder,* OAB) ; la toux ; l'ostéoporose ; des maladies neurodégénératives, de préférence choisies dans l'ensemble constitué par la maladie de Parkinson, la chorée de Huntington, la maladie d'Alzheimer et la sclérose en plaques ; les troubles de la prise d'aliments, de préférence choisis dans l'ensemble constitué par la boulimie, l'anorexie, l'obésité et la cachexie ; les états anxieux ; les dysfonctionnements cognitifs, de préférence les troubles de la mémoire ; les états de déficit cognitif (*attention deficit syndrom, ADS*) *;* l'épilepsie ; la diarrhée et le prurit ; à la prophylaxie et/ou au traitement du mésusage d'alcool et/ou de drogues et/ou de médicaments et de la dépendance à l'alcool et/ou aux drogues et/ou à la nicotine et/ou de la pharmacodépendance, de préférence à la prophylaxie et/ou à la réduction de manifestations de sevrage dans la dépendance à l'alcool et/ou aux drogues et/ou à la nicotine et/ou dans la pharmacodépendance ; à la prophylaxie et/ou à la réduction d'un développement d'une tolérance à des médicaments, en particulier à des médicaments à base d'opioïdes ; à la régulation de la prise de nourriture ; à la modulation de l'activité motrice ; à la régulation du système cardiovasculaire ; à l'anesthésie locale ; à l'augmentation de la vigilance ; à l'augmentation de la libido ; à la diurèse et/ou à l'antinatriurèse.
